(19) Europäisches Patentamt
European Patent Office
Office européen des brevets

(11) **EP 3 839 509 A1**

(12) **EUROPEAN PATENT APPLICATION**

(43) Date of publication:
**23.06.2021 Bulletin 2021/25**

(51) Int Cl.:
***G01N 33/543*** (2006.01)        ***C12N 15/10*** (2006.01)
***C12Q 1/6806*** (2018.01)

(21) Application number: **19216596.7**

(22) Date of filing: **16.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Blink AG**
**07745 Jena (DE)**

(72) Inventors:
• **LEMUTH, Oliver**
**07743 Jena (DE)**
• **KANITZ, Lea**
**07743 Jena (DE)**
• **ELLINGER, Thomas**
**07745 Jena (DE)**

• **KLINGER, Susanne**
**07743 Jena (DE)**
• **LONCAREVIC, Ivan**
**07743 Jena (DE)**
• **STEINMETZER, Katrin**
**07743 Jena (DE)**
• **HUBOLD, Stephan**
**07745 Jena (DE)**
• **ERMANTRAUT, Eugen**
**07749 Jena (DE)**

(74) Representative: **Engelhard, Markus**
**Boehmert & Boehmert**
**Anwaltspartnerschaft mbB**
**Pettenkoferstrasse 22**
**80336 München (DE)**

(54) **A LIBRARY OF PREFABRICATED MICROPARTICLES AND PRECURSORS THEREOF**

(57)     The present invention relates to prefabricated microparticles for performing a specific detection of one or several analytes in a sample, and to precursors of such microparticles, herein also sometimes referred to as "precursor-microparticles". In particular, the present invention relates to libraries of such prefabricated microparticles and libraries of such precursor-microparticles. Furthermore, the present invention relates to kits for making such libraries and to kits of using such libraries for detecting an analyte of interest in a sample. Moreover, the present invention relates to methods of detecting and/or quantitating an analyte of interest in an aqueous sample, preferably using such kits.

Figure 2

SEQUENCE LISTING

<110> Blink AG

<120> A library of prefabricated microparticles and precursors thereof

<130> B33016EP

<160> 8

<170> BiSSAP 1.3.2

<210> 1
<211> 24
<212> DNA
<213> Artificial Sequence

<223> "desthiobiotin-labeled rpoB sense primer "

<220>
<223> desthiobiotin-labeled rpoB sense primer

<400> 1
atcaacatcc ggccggtggt cgcc                                                    24

<210> 2
<211> 21
<212> DNA
<213> Artificial Sequence

<223> "desthiobiotin-labeled rpoB antisense primer "

<220>
<223> desthiobiotin-labeled rpoB antisense primer

<400> 2
tcacgtgaca gaccgccggg c                                                       21

<210> 3
<211> 22
<212> DNA
<213> Artificial Sequence

<223> "desthiobiotin-labeled IS6110 sense primer "

<220>
<223> desthiobiotin-labeled IS6110 sense primer

<400> 3
cgccgcttcg gaccaccagc ac                                                      22

<210> 4
<211> 25
<212> DNA
<213> Artificial Sequence

<223> "desthiobiotin-labeled IS6110 antisense primer "

<220>
<223> desthiobiotin-labeled IS6110 antisense primer

<400> 4
gtgacaaagg ccacgtaggc gaacc                                                    25

<210> 5
<211> 25
<212> DNA
<213> Artificial Sequence

<223> "desthiobiotin-labeled IS1081 sense primer "

<220>
<223> desthiobiotin-labeled IS1081 sense primer

<400> 5
gcgcggcaag atcatcaatg tggag                                                    25

<210> 6
<211> 21
<212> DNA
<213> Artificial Sequence

<223> "desthiobiotin-labeled IS1081 antisense primer "

<220>
<223> desthiobiotin-labeled IS1081 antisense primer

<400> 6
gccaccgcgg ggagtttgtc g                                                        21

<210> 7
<211> 25
<212> DNA
<213> Artificial Sequence

<223> "desthiobiotin-labeled internal control (Bac. globigii) sense primer"

<220>
<223> desthiobiotin-labeled internal control (Bac. globigii) sense
      primer

<400> 7
gcgcggcaag atcatcaatg tggag                                                    25

<210> 8
<211> 21
<212> DNA
<213> Artificial Sequence

<223> "desthiobiotin-labeled internal control (Bac. globigii) antisense
primer "

<220>
<223> desthiobiotin-labeled internal control (Bac. globigii) antisense
      primer

<400> 8
gccaccgcgg ggagtttgtc g                                                        21

**Description**

BACKGROUND OF THE INVENTION

The present invention relates to prefabricated microparticles for performing a specific detection of one or several analytes in a sample, and to precursors of such microparticles, herein also sometimes referred to as "precursor-microparticles". In particular, the present invention relates to libraries of such prefabricated microparticles and libraries of such precursor-microparticles. Furthermore, the present invention relates to kits for making such libraries and to kits of using such libraries for detecting an analyte of interest in a sample. Moreover, the present invention relates to methods of detecting and/or quantitating an analyte of interest in an aqueous sample, preferably using such kits.

Sensitive detection of multiple analytes in a sample is desirable. Various methods for the simultaneous detection of analytes in solution have been developed. For example, for the sensitive detection of nucleic acids, methods have been used which, by means of template-specific amplification, permit the detection of a nucleic acid sequence in a sample. For this purpose, generally well-described methods are available and used, such as for example the polymerase chain reaction (PCR), the recombinase polymerase reaction (RPA), transcription-mediated amplification (TMA), loop-mediated amplification reaction (LAMP) and others. However, cross-reactivity of the employed reagents has limited the achievable levels of multiplexing to low number of targets to be detected in the same assay.

For the sensitive detection of protein antigens, template-dependent amplification is not feasible. However, methods of nucleic acid amplification have been combined with classical immunoassay formats. For example in Immuno-PCR (Adler, Wacker et al. 2003), a binding antibody immobilized to a solid phase and a detection antibody with an attached nucleic acid sequence label are used. After a sandwich consisting of the first antibody, the antigen and the second antibody has been formed, the nucleic acid sequence attached to the second antibody is amplified by means of an amplification reaction. In another assay format (Todd, Freese et al. 2007), a sandwich is formed between an antibody bound to a solid phase, the antigen and a second antibody provided with a fluorophore. The fluorophore is cleaved selectively and quantitatively and counted molecule by molecule with a molecular flow counter. The number of detected fluorophores corresponds directly to the number of bound analyte.

A method known as SiMOA (Rissin, Kan et al. 2010) ("single-molecule array") is also based on the digital determination of single binding events. Antigens are first captured to the outside of solid particles by means of a first antibody. A second antibody labeled with an enzyme is bound to the antigen. The solid particles are contacted with a solution containing a substrate which is converted into a fluorescent product by the enzyme. The particles are placed in small cavities of an array such that only one particle is contained in a cavity and a small amount of the solution. The cavities which function as reaction spaces are then covered with a non-aqueous solution thus isolating the individual cavities. By counting the individual cavities with a detectable signal and by quantifying the signal in each cavity the ultra-sensitive determination of antigen concentration is accomplished.

In order to facilitate improved handling of the employed micro-carriers, magnetic particles have been used (Tekin and Gijs 2013).

(Leng, Zhang et al. 2010) describe hydrogel particles with covalently attached primer nucleotides, using a modified agarose polymer for primer binding. The primer-modified agarose was then mixed with the amplification reagents and the target containing solution, and a microfluidic droplet generator is used to form hydrogel microparticles. The agarose emulsion formed in oil is applied for amplification/detection.

There is a need in the art for parallel detection of multiple analytes in a sample meeting the following requirements:

a) no cross-interference of the analyte-specific reactions,
b) high sensitivity and specificity for each analyte, and
c) simple set-up and combination of the analyte-specific reagents with minimal equipment.

The present invention is aimed at providing and meeting these requirements.

SUMMARY OF THE INVENTION

The invention is now described by reference to various aspects and embodiments:
In a first aspect, the present invention relates to:

1. A library of prefabricated precursor-microparticles for making a library of prefabricated microparticles, said prefabricated microparticles being configured for performing specific detection of one or several analytes of interest in a sample, such specific detection occurring within such microparticles by a suitable chemical or biochemical reaction, each of said prefabricated precursor-microparticles in said library comprising:

- a porous matrix having a void volume for receiving an aqueous sample and for providing a reaction space for the specific detection of an analyte;
- a reagent binding component allowing the attachment, preferably the reversible attachment, of an analyte-specific reagent to the precursor-microparticle; said reagent binding component being one of:

(i) a polymer or polymer mixture that forms said porous matrix or is said porous matrix;
(ii) a reagent binding molecule attached to said porous matrix;
(iii) at least one ionisable group, or a plurality of ionisable groups, immobilized on said porous matrix, said ionisable group(s) being capable of changing its (their) charge(s) according to ambient conditions surrounding said precursor-microparticle;
(iv) at least one charged group, or a plurality of charged groups immobilized on said porous matrix;
(v) a combination of any of (i) - (iv);

- a label component attached to, contained within or otherwise associated with said precursor-microparticle for identifying said analyte-specific reagent, when attached to the precursor-microparticle.

2. The library according to embodiment 1, wherein, in said library, there are at least two separate subsets of prefabricated precursor-microparticles, preferably three or more separate subsets of prefabricated precursor-microparticles, with each subset having its distinct label component attached to, contained within or otherwise associated with said precursor-microparticles within said subset, such that said at least two or more separate subsets of prefabricated precursor-microparticles differ by the respective label component attached to, contained within or otherwise associated with each subset.

3. The library according to any of embodiments 1 - 2, wherein said label component is a mixture of at least two different dyes, preferably at least two fluorescent dyes, wherein said at least two different dyes, preferably said at least two fluorescent dyes, are present on each subset of said precursor-microparticles at a respective defined ratio and/or in defined amounts of said at least two different dyes, such that said different subsets of precursor-microparticles differ from each other in terms of the respective ratio and/or amounts of said at least two different dyes, thus allowing a distinction between said different subsets of precursor-microparticles by the respective ratios and/or amounts of said at least two different dyes attached to or contained within the respective subset of precursor-microparticles.

4. The library according to any of embodiments 1 - 3, wherein said porous matrix is a porous polymeric matrix.

5. In a further aspect, the present invention also relates to a library of prefabricated microparticles for performing specific detection of one or several analytes of interest in a sample, such specific detection occurring within such microparticles by a suitable chemical or biochemical reaction, each of said prefabricated microparticles comprising a prefabricated precursor-microparticle according to any of embodiments 1 - 4 and further comprising an analyte-specific reagent attached, preferably reversibly attached, to said precursor-microparticle.

6. The library of prefabricated microparticles according to embodiment 5, wherein said analyte-specific reagent that is attached to each of said precursor-microparticles, is attached, preferably reversibly attached, through said reagent binding component by

a) direct binding of said analyte-specific reagent to said polymer or polymer mixture that forms said porous polymeric matrix or is part of said porous polymeric matrix (i);
b) said analyte-specific reagent being conjugated to a binding entity which, in turn, binds to said reagent binding molecule (ii);
c) direct binding of said analyte-specific reagent to said ionisable group(s) (iii) under conditions in which said ionisable group(s) has(have) a suitable net charge;
d) direct binding of said analyte-specific reagent to said charged group(s) (iv) on said polymer, wherein said analyte-specific reagent has at least one ionisable group, or a plurality of ionisable groups, said ionisable group(s) being capable of changing its(their) charge(s) according to ambient conditions surrounding said analyte-specific reagent; or
e) a combination of any of (a) - (d).

7. The library of prefabricated precursor-microparticles according to any of embodiments 1 - 4 or the library of prefabricated microparticles according to any of embodiments 5 - 6, wherein

- said polymer (i) is a hydrogel-forming agent selected from the group comprising ia) synthetic polymers, such as poly(methyl)(meth)acrylate, polyamide; ib) silicone-based polymers, e.g. polydimethylsiloxanes; ic) naturally occurring polymers selected from polysaccharides, e.g. agarose, chitin, chitosan, dextran, alginate, carrageenan, cellulose, fucoidan, laminaran; gums selected from xanthan gum, arabic gum, ghatti gum, guar gum, locust bean gum, tragacanth gum, karaya gum, and inulin; polypeptides, e.g. collagens, gelatins; poly-amino acids, such as poly-lysine; polynucleotides; and said polymer mixture is a combination of any of the foregoing;
- said reagent binding molecule (ii) is selected from avidin, in particular tetrameric avidin; streptavidin; monomeric avidin; avidin having a nitrated tyrosine in its biotin binding site; other proteins, derived from or related to, avidin and retaining binding functionality of avidin; biotin; desthiobiotin; iminobiotin; biotin having a cleavable spacer arm; selenobiotin; oxybiotin; homobiotin; norbiotin; iminobiotin; diaminobiotin; biotin sulfoxide; biotin sulfone; epibiotin; 5-hydroxybiotin; 2-thiobiotin; azabiotin; carbobiotin; methylated derivatives of biotin; ketone biotin; other molecules, derived from or related to, biotin and retaining binding functionality of biotin;
- said at least one ionisable group (iii) or said ionisable group of embodiment 6 d) is selected from

- N-2-acetamido-2-aminoethanesulfonic acid (ACES);
- N-2-acetamido-2-iminodiacetic acid (ADA);
- amino methyl propanediol (AMP);
- 3-1,1-dimethyl-2-hydroxyethylamino-2-hydroxy propanesulfonic acid (AMPSO);
- N,N-bis2-hydroxyethyl-2-aminoethanesulfonic acid (BES);
- N,N-bis-2-hydroxyethylglycine (BICINE);
- bis-2-hydroxyethyliminotrishydroxymethylmethane (Bis-Tris);
- 1,3-bistrishydroxymethylmethylaminopropane (Bis-Tris Propane);
- 4-cyclohexylamino-i-butane sulfonic acid (CABS);
- 3-cyclohexylamino-1-propane sulfonic acid (CAPS);
- 3-cyclohexylamino-2-hydroxy-1-propane sulfonic acid (CAPSO);
- 2-N-cyclohexylaminoethanesulfonic acid (CHES);
- 3-N,N-bis-2-hydroxyethylamino-2-hydroxypropanesulfonic acid (DIPSO);
- N-2-hydroxyethylpiperazine-N-3-propanesulfonic acid (EPPS);
- N-2-hydroxyethylpiparazine-N-4-butanesulfonic acid (HEPBS);
- N-2-hydroxyethylpiperazine-N-2-ethanesulfonic acid (HEPES);
- N-2-hydroxyethylpiperazine-N-2-propanesulfonic acid (HEPPSO);
- 2-N-morpholinoethanesulfonic acid (MES);
- 4-N-morpholinobutanesulfonic acid (MOBS);
- 3-N-morpholinopropanesulfonic acid (MOPS);
- 3-N-morpholino-2-hydroxypropanesulfonic acid (MOPSO);
- piperazine-N-N-bis-2-ethanesulfonic acid (PIPES);
- piperazine-N-N-bis-2-hydroxypropanesulfonic acid (POPSO);
- N-trishydroxymethyl-methyl-4-aminobutanesulfonic acid (TABS);
- N-trishydroxymethyl-methyl-3-aminopropanesulfonic acid (TAPS);
- 3-N-trishydroxymethyl-methylamino-2-hydroxypropanesulfonic acid (TAPSO);
- N-trishydroxymethyl-methyl-2-aminoethanesulfonic acid (TES);
- N-trishydroxymethylmethylglycine (TRICINE);
- trishydroxymethylaminomethane (Tris);
- polyhydroxylated amines;
- imidazole, and derivatives thereof (i.e. imidazoles), especially derivatives containing hydroxyl groups;
- triethanolamine dimers and polymers; and di/tri/oligo/poly amino acids, such as for example Ala-Ala; Gly-Gly; Ser-Ser; Gly-Gly-Gly; or Ser-Gly. Oligo-His, Poly-His, Oligo-Lys, Poly-Lys.

8. The library of prefabricated precursor-microparticles according to any of embodiments 1 - 4, 7 or the library of prefabricated microparticles according to any of embodiments 5 - 7,
wherein said porous polymeric matrix, or said polymer or polymer mixture that forms or is part of said porous polymeric matrix, is composed of a polymer that is not crosslinked, wherein, preferably, said polymer or polymer mixture that forms or is part of said porous polymeric matrix, is composed of agarose or a combination of agarose and gelatin, wherein, more preferably, in said combination of agarose and gelatin, said agarose is present in a range of from 0.1 %(w/v) to 4%(w/v), and said gelatin is present in a range of from 0.1%(w/v) to 20%(w/v), preferably from 0.5%(w/v) to 20%(w/v).

9. The library of prefabricated microparticles according to any of embodiments 5 - 8, wherein said analyte-specific reagent is selected from nucleic acids, including aptamers, Spiegelmers; antibodies or antibody fragments; non-antibody proteins capable of specifically binding an analyte or analyte complex, such as receptors, receptor fragments, and affinity proteins; wherein preferably said analyte-specific reagent is selected from nucleic acids, in particular nucleic acid oligomers and nucleic acid primers.

10. The library of prefabricated microparticles according to any of embodiments 5 - 9, wherein, for each of said microparticles, said analyte-specific reagent is reversibly attached to said microparticle through said reagent binding component by b) said analyte-specific reagent being conjugated to a binding entity which, in turn, binds to said reagent binding molecule (ii), wherein

- said binding entity is independently selected from biotin, desthiobiotin, iminobiotin, biotin having a cleavable spacer arm, selenobiotin, oxybiotin, homobiotin, norbiotin, iminobiotin, diaminobiotin, biotin sulfoxide, biotin sulfone, epibiotin, 5-hydroxybiotin, 2-thiobiotin, azabiotin, carbobiotin, methylated derivatives of biotin, ketone biotin, other molecules, derived from or related to, biotin and retaining binding functionality of biotin; and said reagent binding molecule is independently selected from avidin and streptavidin; or vice versa; or
- said binding entity is biotin, and said reagent binding molecule is selected from monomeric avidin, avidin having a nitrated tyrosine in its biotin binding site, and other proteins, derived from or related to, avidin and retaining binding functionality of avidin; or vice versa.

11. The library of prefabricated microparticles according to any of embodiments 5 - 10, wherein said library is for performing a specific detection of a single analyte of interest in several samples, wherein, in said library, there are at least two separate subsets of prefabricated microparticles, preferably three or more separate subsets of prefabricated microparticles,
with each subset

- having its distinct label component attached to, contained within or otherwise associated with said microparticles of said subset; and

all of said at least two, three or more separate subsets having

- the same analyte-specific reagent attached to the porous matrix of said microparticles of said subsets, said analyte-specific reagent being specific for one analyte of interest;

such that said at least two or more separate subsets of prefabricated microparticles are identical in terms of the analyte-specific reagent attached, but differ by

- the respective label component attached to, contained within or otherwise associated with said microparticles of each subset;

with each subset being unambiguously defined and identifiable by said respective label component.

12. The library of prefabricated microparticles according to any of embodiments 5 - 10, wherein said library is for performing a specific detection of multiple analytes of interest in a single sample, wherein, in said library, there are at least two separate subsets of prefabricated microparticles, preferably three or more separate subsets of prefabricated microparticles,
with each subset

- having its distinct label component attached to, contained within or otherwise associated with said microparticles of said subset; and
- having a different analyte-specific reagent attached to the porous matrix of said microparticles of said subset; each analyte-specific reagent being specific for one analyte of interest;

such that said at least two or more separate subsets of prefabricated microparticles differ by

- the respective label component attached to, contained within or otherwise associated with said microparticles of each subset; and
- the respective analyte-specific reagent attached to each subset;

with each subset being unambiguously defined and identifiable by said respective label component and said respective analyte-specific reagent.

13. The library of prefabricated microparticles according to any of embodiments 5 - 10, wherein said library is for performing a specific detection of multiple analytes of interest in several samples, wherein, in said library, there are a plurality of different separate subsets of prefabricated microparticles,
with each subset

- having its distinct label component attached to, contained within or otherwise associated with said microparticles of said subset; and

wherein, in said plurality of separate subsets of prefabricated microparticles, there are different classes of separate subsets of prefabricated microparticles, with each of said classes comprising several subsets of microparticles and each class having a different analyte-specific reagent attached to the porous matrix of said microparticles, and all subsets of microparticles within one class having the same analyte-specific reagent attached; each analyte-specific reagent being specific for one analyte of interest;
such that, in said library, said plurality of different separate subsets of prefabricated microparticles differ by

- the respective label component attached to, contained within or otherwise associated with said microparticles of each subset; and

each separate subset of microparticles forms part of one class of subsets of microparticles;
with each subset being unambiguously defined and identifiable by said respective label component and said respective analyte-specific reagent; and
such that said different classes of subsets of microparticles differ by the respective analyte-specific reagent attached to the porous matrix of said microparticles; and each of said different classes comprises several subsets of microparticles, all of which subsets within one class having the same analyte-specific reagent attached.

14. A kit for making a library of prefabricated microparticles according to any of embodiments 5 - 13, said kit comprising:

- at least two containers, namely a first and a second container, and, optionally, further containers, each containing:

  • a subset of prefabricated precursor-microparticles as defined in any of embodiments 1 - 4, 7 and 8, with each subset having its distinct label component attached to, contained within or otherwise associated with said precursor-microparticles within said subset, such that said two subsets of prefabricated precursor-microparticles differ by the respective label component attached to, contained within or otherwise associated with each subset;

- a further container containing:

  • a conditioning solution to enable the attachment, preferably the reversible attachment, of an analyte-specific reagent to said subset of said precursor-microparticles through said reagent binding component by

    a) direct binding of said analyte-specific reagent to said polymer or polymer mixture that forms said porous polymeric matrix or is part of said porous polymeric matrix (i);
    b) said analyte-specific reagent being conjugated to a binding entity which, in turn, binds to said reagent binding molecule (ii);
    c) direct binding of said analyte-specific reagent to said ionisable group(s) (iii) under conditions in which said ionisable group(s) has(have) a suitable net charge;
    d) direct binding of said analyte-specific reagent to said charged group(s) (iv) on said polymer, wherein said analyte-specific reagent has at least one ionisable group, or a plurality of ionisable groups, said ionisable group(s) being capable of changing its(their) charge(s) according to ambient conditions surrounding said analyte-specific reagent; or
    e) a combination of any of (a) - (d);

    said reagent binding component being as defined in embodiment 1, said reagent binding molecule being as defined in any of embodiments 1, 6 and 7, and said binding entity being as defined in any of embodiments 6 - 7; said polymer or polymer mixture being as defined in any of embodiments 1, 6 - 8; and said ionisable groups being as defined in any of embodiments 1, 6, and 7 , and said analyte-specific reagent being as defined in any of embodiments 5 - 6, 9, and 10.

15. The kit according to embodiment 14, further comprising:

- a further container containing a washing buffer for removing free, i.e. unattached, analyte-specific reagent(s) from microparticles, without removing analyte-specific reagent(s) attached to microparticles.

16. The kit according to any of embodiments 14 and 15, further comprising:

- at least one mixing container for mixing components together, preferably several mixing containers, more preferably as many mixing containers as there are containers containing subsets of precursor-microparticles each.

17. In a further aspect the present invention also relates to a method of making a library of prefabricated microparticles according to any of embodiments 5 - 13, said method comprising:

- Providing, in any order:

    • a library of prefabricated precursor-microparticles as defined in any of embodiments 1 - 4, 7 and 8;
    • at least one analyte-specific reagent, as defined in any of embodiments 5 - 6, 9, and 10;

- mixing said library of prefabricated precursor-microparticles, or selected subsets thereof, and the at least one analyte-specific reagent under conditions allowing the attachment, preferably the reversible attachment, of said at least one analyte-specific reagent to some or all of said prefabricated precursor-microparticles, thus generating a library of prefabricated microparticles according to any of embodiments 5 - 13;
- optionally, washing said prefabricated microparticles to remove any unattached analyte-specific reagent therefrom.

18. The method according to embodiment 17, wherein said method is performed by using a kit as defined in any of embodiments 14 - 16, wherein said library is provided in the form of said at least two containers of said kit, with each container containing

    • a subset of prefabricated precursor-microparticles as defined in any of embodiments 1 - 4, 7 and 8, with each subset having its distinct label component attached to, contained within or otherwise associated with said precursor-microparticles within said subset, such that said at least two subsets of prefabricated precursor-microparticles differ by the respective label component attached to, contained within or otherwise associated with each subset;

wherein said conditions allowing the attachment, preferably the reversible attachment, of said at least one analyte-specific reagent to some or all of said prefabricated precursor-microparticles, are generated by mixing said library of prefabricated precursor-microparticles, or selected subsets thereof, and the at least one analyte-specific reagent in the presence of a conditioning solution, as defined in embodiment 14, wherein, preferably, said conditioning solution is a buffer.

19. A kit for detecting an analyte in a sample, said kit comprising:

    a)

- a container containing a generic detection composition comprising reagents for performing a chemical or biochemical detection reaction of an analyte, wherein said chemical or biochemical detection reaction of an analyte is a nucleic acid amplification, said generic detection composition comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid, OR
- a container containing a first detection composition comprising reagents for performing a chemical or biochemical detection reaction of an analyte, and yet a further container containing a second detection composition comprising a detection reagent, wherein said chemical or biochemical detection reaction of an analyte is an immunochemistry detection reaction, said first detection composition comprises reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry detection reaction; and said second detection composition comprises, as a detection reagent, a suitable substrate for said reporter enzyme which substrate upon having been reacted by said reporter enzyme, becomes detectable, preferably optically detectable, more preferably detectable by fluorescence, OR

- a container containing a first detection composition comprising reagents for performing a chemical or biochemical detection reaction of an analyte, and yet a further container containing a second detection composition comprising a detection reagent, wherein the chemical or biochemical reaction is an immunochemistry detection reaction, said first detection composition comprises reagents for performing an immunochemistry detection reaction, such as a buffer, and a secondary antibody or secondary antibody fragment coupled to a suitable oligonucleotide tag and being specific for the same analyte as a primary antibody, used as analyte-specific reagent (ASR) in said immunochemistry detection reaction; and said second detection composition comprises, as detection reagent(s), a buffer, mono-nucleoside-triphosphates, an amplification enzyme, such as a suitable nucleic acid polymerase, e. g. Taq polymerase, and a nucleic acid dye for detection of an amplification product, such as an amplified nucleic acid, and primers suitable for amplifying the oligonucleotide tag attached to the secondary antibody;

and
b)

- a container containing a non-aqueous phase, such as an oil, e.g. fluorocarbon oil, optionally supplemented with an emulsifier;

and
c)

- a mixing container for mixing components.

20. The kit according to embodiment 19, further comprising:
d)

- a container for performing a detection reaction;

21. The kit according to any of embodiments 19 - 20, further comprising:
e)

- the kit according to any of embodiments 14 - 16, or
- a library of prefabricated microparticles according to any of embodiments 5 - 13.

22. In a further aspect the present invention also relates to a method of detecting and/or quantitating an analyte of interest in an aqueous sample, said method comprising the steps:

- providing, in any order:

• an aqueous sample known or suspected to contain an analyte of interest;
• a generic detection composition comprising reagents for performing a chemical or biochemical detection reaction of the analyte;
• a library of prefabricated microparticles according to any of embodiments 5 - 13, wherein said analyte-specific reagent(s) attached to said microparticles, is (are) chosen such that it is (they are) capable of specifically binding to or reacting with said analyte of interest;

- optionally, mixing said aqueous sample and said generic detection composition;
- incubating said aqueous sample with said library of prefabricated microparticles, thereby allowing said library of microparticles to absorb aqueous sample in the void volumes of said microparticles and, optionally, to bind said analyte of interest, if present in said sample;

- optionally, washing said microparticles;
- adding said generic detection composition to the microparticles, if said aqueous sample has not been previously mixed with said generic detection composition;

  - transferring said library of prefabricated microparticles into a non-aqueous phase and removing any aqueous phase surrounding the individual prefabricated microparticle(s), thereby creating a plurality of insulated reaction spaces for detecting said analyte, which reaction spaces comprise an aqueous phase and are confined to said void volume(s) of said microparticles;
  - optionally, releasing the analyte-specific reagent(s) attached to said microparticles by applying an external trigger;
  - performing a detection reaction of said analyte of interest;
  - detecting and/or quantitating said analyte of interest.

23. The method according to embodiment 22, wherein said analyte of interest is a nucleic acid, and

- said analyte-specific reagent is a nucleic acid or a pair of nucleic acids, sufficiently complementary to said analyte of interest to be able under hybridizing conditions to hybridize to said analyte of interest, wherein, preferably, said analyte-specific reagent is a suitable primer or primer pair for amplification of said analyte of interest;
- said generic detection composition comprises reagents for performing an amplification reaction of said nucleic acid analyte of interest, except for primers, wherein, in particular, said generic detection composition comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid;
- said step of transferring is a step wherein said prefabricated microparticles are transferred into a non-aqueous phase and suspended and, optionally, repeatedly washed with said non-aqueous phase, said step more optionally involving also a filtration or mechanical agitation to ensure the formation of a monodisperse suspension, of microparticles with no aqueous phase or no substantial aqueous phase remaining outside of any of the microparticles;
- said optional step of releasing the analyte-specific reagent(s) attached to said microparticles by applying an external trigger is a step of temporarily increasing the temperature, changing the pH, or changing the salt conditions, preferably of increasing the temperature, more preferably from ambient temperature to a temperature >90°C.
- said step of performing a detection reaction is a step of performing an amplification reaction of said analyte if present in said aqueous sample; and
- said step of detecting and/or quantitating said analyte of interest is a step of detecting and/or quantitating said amplified analyte.

24. In a further aspect the present invention also relates to a method of detecting and/or quantitating an analyte of interest in an aqueous sample, said method comprising the steps:

- providing, in any order:

  • an aqueous sample known or suspected to contain an analyte of interest;
  • a first detection composition comprising necessary reagents for performing a chemical or biochemical detection reaction of the analyte;
  • a second detection composition comprising a detection reagent;
  • a library of prefabricated microparticles according to any of embodiments 5 - 13, wherein said analyte-specific reagent(s) attached to said microparticles, is (are) chosen such that it is (they are) capable of specifically binding to or reacting with said analyte of interest;

- optionally, mixing said aqueous sample and said first detection composition;
- incubating said aqueous sample with said library of prefabricated microparticles and, if said aqueous sample has not already been mixed with said first detection composition, also with said first detection composition, thereby allowing said library of microparticles to absorb aqueous sample and said first detection composition in the void volumes of said microparticles and, optionally, to bind said analyte of interest, if present in said sample;
- optionally, washing said library of prefabricated microparticles to remove any non-absorbed or non-reacted first detection composition;

9

- incubating said library of prefabricated microparticles including absorbed aqueous sample with said second detection composition, thereby allowing said library of microparticles to absorb said second detection composition;
- optionally, further washing said library of prefabricated microparticles to remove any non-absorbed or non-reacted second detection composition;
- transferring said library of prefabricated microparticles into a non-aqueous phase and removing any aqueous phase surrounding the individual prefabricated microparticle(s), thereby creating a plurality of insulated reaction spaces for detecting said analyte which reaction spaces comprise an aqueous phase and are confined to said void volume(s) of said microparticles; wherein, preferably, said step of transferring into a non-aqueous phase is performed immediately after said library of prefabricated microparticles have been incubated with said second detection composition;
- optionally, releasing the analyte-specific reagent(s) attached to said microparticles by applying an external trigger;
- detecting and/or quantitating said analyte of interest by detecting and/or quantitating said detection reagent.

25. The method according to embodiment 24, wherein said analyte of interest is a protein or other non-nucleic acid molecule, and

- said analyte-specific reagent is a primary antibody, antibody fragment, or a non-antibody protein capable of specifically binding said protein analyte or other non-nucleic acid analyte;
- said first detection composition comprises necessary reagents for performing an immunochemistry detection reaction, such as a buffer, and a secondary antibody or secondary antibody fragment coupled to a suitable reporter enzyme and being specific for said analyte;
- said second detection composition comprises, as a detection reagent, a suitable substrate for said suitable reporter enzyme which substrate upon having been reacted by said reporter enzyme, becomes detectable, preferably optically detectable, more preferably detectable by fluorescence;
- said step of incubating said aqueous sample with said library of prefabricated microparticles and with said first detection composition is a step of performing an immunochemistry reaction involving the binding of said analyte, if present, in said aqueous sample, to said primary antibody, primary antibody fragment, or non-antibody protein, thus forming a complex of analyte and said primary antibody, primary antibody fragment, or non-antibody protein; said immunochemistry reaction further involving a binding of said secondary antibody to said complex, thus forming a sandwich between primary antibody, antibody fragment or non-antibody protein, analyte, and secondary antibody;
- said first optional step of washing said library is a step of removing any non-bound secondary antibody from said library;
- said step of incubating said library of prefabricated microparticles including absorbed aqueous sample with said second detection composition is a step of allowing said substrate to be reacted by said reporter enzyme;
- said further optional step of washing said library is a step of removing any non-reacted substrate from said library;
- said step of transferring is a step wherein said prefabricated microparticles are transferred into a non-aqueous phase and suspended and, optionally, repeatedly washed with said non-aqueous phase, more optionally involving also a filtration or mechanical agitation to ensure the formation of a monodisperse suspension of microparticles with no aqueous phase or no substantial aqueous phase remaining outside of any of the microparticles;
- said optional step of releasing the analyte-specific reagent(s) attached to said microparticles by applying an external trigger is a step of temporarily increasing the temperature, changing the pH, or changing the salt conditions, preferably a step of increasing the temperature, more preferably from ambient temperature to a temperature >90°C;
- said step of detecting and/or quantitating said analyte of interest is a step of detecting and/or quantitating said reacted substrate.

26. In a further aspect the present invention also relates to a method of detecting and/or quantitating an analyte of interest in an aqueous sample, said method comprising the steps:

- providing, in any order:

  • an aqueous sample known or suspected to contain an analyte of interest;
  • a first detection composition comprising necessary reagents for performing a chemical or biochemical detection reaction of the analyte;

- a second detection composition comprising a detection reagent;
- a library of prefabricated microparticles according to any of embodiments 5 - 13, wherein said analyte-specific reagent(s) attached to said microparticles, is(are) chosen such that it is (they are) capable of specifically binding to or reacting with said analyte of interest;

- optionally, mixing said aqueous sample and said first detection composition;
- incubating said aqueous sample with said library of prefabricated microparticles and, if said aqueous sample has not already been mixed with said first detection composition, also with said first detection composition, thereby allowing said library of microparticles to absorb aqueous sample and said first detection composition in the void volumes of said microparticles and, optionally, to bind said analyte of interest, if present in said sample;
- optionally, washing said library of prefabricated microparticles to remove any non-absorbed or non-reacted first detection composition;
- incubating said library of prefabricated microparticles including absorbed aqueous sample with said second detection composition, thereby allowing said library of microparticles to absorb said second detection composition;
- optionally, further washing said library of prefabricated microparticles to remove any non-absorbed or non-reacted second detection composition;
- transferring said library of prefabricated microparticles into a non-aqueous phase and removing any aqueous phase surrounding the individual prefabricated microparticle(s), thereby creating a plurality of insulated reaction spaces for detecting said analyte which reaction spaces comprise an aqueous phase and are confined to said void volume(s) of said microparticles;
- optionally, releasing the analyte-specific reagent(s) attached to said microparticles by applying an external trigger;
- performing a detection reaction of said analyte of interest; and
- detecting and/or quantitating said analyte of interest.

27. The method according to embodiment 26, wherein said analyte of interest is a protein or other non-nucleic acid molecule, and

- said analyte-specific reagent is a primary antibody, antibody fragment, or a non-antibody protein capable of specifically binding said protein analyte or other non-nucleic acid analyte;
- said first detection composition comprises necessary reagents for performing an immunochemistry detection reaction, such as a buffer, and a secondary antibody or secondary antibody fragment coupled to a suitable oligonucleotide tag and being specific for the same analyte as said primary antibody;
- said second detection composition comprises, as detection reagent(s), a buffer, mono-nucleoside-triphosphates, an amplification enzyme, such as a suitable nucleic acid polymerase, e. g. Taq polymerase, and a nucleic acid dye for detection of an amplification product, such as an amplified nucleic acid, and primers suitable for amplifying the oligonucleotide tag attached to the secondary antibody;
- said step of incubating said aqueous sample with said library of prefabricated microparticles and with said first detection composition is a step of performing an immunochemistry reaction involving the binding of said analyte, if present, in said aqueous sample, to said primary antibody, primary antibody fragment, or non-antibody protein, thus forming a complex of analyte and said primary antibody, primary antibody fragment, or non-antibody protein; said immunochemistry reaction further involving a binding of said secondary antibody to said complex, thus forming a sandwich between primary antibody, antibody fragment or non-antibody protein, analyte, and secondary antibody;
- said first optional step of washing said library is a step of removing any non-bound secondary antibody from said library;
- said step of incubating said library of prefabricated microparticles including absorbed aqueous sample with said second detection composition is a step of allowing said primers in said second detection composition hybridize to the oligonucleotide tag attached to the secondary antibody;
- said further optional step of washing said library is a step of removing any non-hybridized primers from said library;
- said step of transferring is a step wherein said prefabricated microparticles are transferred into a non-aqueous phase and suspended and, optionally, repeatedly washed with said non-aqueous phase, more optionally involving also a filtration or mechanical agitation to ensure the formation of a monodisperse suspension, of microparticles with no aqueous phase or no substantial aqueous phase remaining outside of any of the microparticles;
- said optional step of releasing the analyte-specific reagent(s) attached to said microparticles by applying an external trigger is a step of temporarily increasing the temperature, changing the pH, or changing the salt conditions, preferably a step of increasing the temperature, more preferably from ambient temperature to a temperature >90°C;
- said step of performing a detection reaction is a step of performing an amplification reaction of said oligonucleotide tag; and

said step of detecting and/or quantitating said analyte(s) of interest is a step of detecting and/or quantitating said amplified oligonucleotide tag(s).

28. The method according to any of embodiments 22 - 27, wherein said step of releasing the analyte-specific reagent(s) attached to said microparticles by applying an external trigger is performed by raising the temperature to which said microparticles are exposed.

29. The method according to any of embodiments 22 - 28, wherein,

- the method is a method of detecting and/or quantitating a single analyte in a plurality of aqueous samples, e.g. from different patients,
- in said method, there are provided a plurality of different aqueous samples known or suspected to contain an analyte of interest, e.g. samples from different patients,
- the library of prefabricated microparticles that is provided in said method, is a library according to embodiment 11, wherein, in such library, there are as many or at least as many separate subsets of microparticles provided, as there are different aqueous samples provided and to be tested, wherein all of said separate substeps have the same analyte-specific reagent attached to the porous matrix of said microparticles of said subsets, said analyte-specific reagent being specific for one analyte of interest;
- in said step of incubating said aqueous samples with said library of prefabricated microparticles, each of said different aqueous samples is incubated separately with a separate subset of microparticles of said library;
- in said step of incubating said library of prefabricated microparticles including absorbed aqueous sample with a second detection composition, each of said separate subsets of microparticles of said library is incubated separately with said second detection composition;
- said step of transferring said library into a non-aqueous phase is performed for each subset of microparticles separately, i.e. each separate subset of microparticles is separately transferred into a non-aqueous phase, and the aqueous phase surrounding the individual microparticles is removed for each subset separately, thereby creating, for each subset separately, a plurality of insulated reaction spaces for detecting said analyte which reaction spaces comprise an aqueous phase and are confined to said void volume(s) of said microparticles,

wherein said method further comprises, after said step of transferring said library into a non-aqueous phase, a step of mixing said plurality of insulated reaction spaces of all the separate subsets in said non-aqueous phase together, before subsequently a detection reaction of said analyte of interest is performed, and before detecting and/or quantitating said analyte of interest.

30. The method according to any of embodiments 22 - 28, wherein,

- the method is a method of detecting and/or quantitating multiple analytes in a single aqueous sample,
- in said method, there is provided a single aqueous sample known or suspected to contain multiple analytes of interest,
- the library of prefabricated microparticles that is provided in said method, is a library according to embodiment 12, wherein, in such library, there are as many or at least as many separate subsets of microparticles provided, as there are different analytes of interest to be detected, with each of said separate subsets having a different analyte-specific reagent attached to the porous matrix of said microparticles of said subset; each analyte-specific reagent being specific for one analyte of interest;
- in said step of incubating said aqueous sample with said library of prefabricated microparticles, said aqueous sample is incubated with all of said separate subsets of microparticles of said library together;
- in said step of incubating said library of prefabricated microparticles including absorbed aqueous sample with a second detection composition, all of said separate subsets of microparticles of said library are incubated together with said second detection composition;
- said step of transferring said library into a non-aqueous phase is performed for all subsets of microparticles together, i.e. all subsets of microparticles are transferred together into a non-aqueous phase, and the aqueous phase surrounding the individual microparticles is removed, thereby creating a plurality of insulated reaction spaces for detecting and/or quantitating said multiple analytes which reaction spaces comprise an aqueous phase and are confined to said void volume(s) of said microparticles.

31. The method according to any of embodiments 22 - 28, wherein,

- the method is a method of detecting and/or quantitating multiple analytes in a plurality of aqueous samples,
- in said method, there are provided a plurality of different aqueous samples known or suspected to contain multiple analytes of interest, e.g. samples from different patients,
- the library of prefabricated microparticles that is provided in said method, is a library according to embodiment 13, wherein, in such library, there are different classes of separate subsets of prefabricated microparticles, with each of said classes comprising several subsets of microparticles and each of said classes having a different analyte-specific reagent attached to the porous matrix of said microparticles, and all subsets of microparticles within one class having the same analyte-specific reagent attached; each analyte-specific reagent being specific for one analyte of interest; wherein, in said method, in said step of providing said library, there are

  • as many or at least as many different classes of separate subsets of microparticles provided, as there are different analytes to be detected,
  • as many or at least as many different subsets of microparticles provided within each class , as there are aqueous samples provided and to be tested,
  • as many or at least as many different subsets of microparticles provided within the library as there are different aqueous samples to be tested multiplied by the number of different analytes of interest to be detected,

- in said step of incubating said aqueous sample with said library of prefabricated microparticles, , exactly one aqueous sample is incubated with exactly one subset of microparticles from each class, with each aqueous sample being incubated with as many different subsets of microparticles from different classes together as there are classes of microparticles in said library,
- in said step of incubating said library of prefabricated microparticles including absorbed aqueous sample with a second detection composition, the combined subsets from different classes of microparticles with which subsets one respective aqueous sample had been previously incubated, are incubated together with said second detection composition,
- said step of transferring said library into a non-aqueous phase is performed for each aqueous sample separately, namely the combined subsets of microparticles with which one respective aqueous sample had been previously incubated, are transferred together into a non-aqueous phase, and the aqueous phase surrounding the individual microparticles is removed, thereby creating, for each aqueous sample separately, a plurality of insulated reaction spaces for detecting said multiple analytes which reaction spaces comprise an aqueous phase and are confined to said void volume(s) of said microparticles,

wherein said method further preferably comprises, after said step of transferring said library into a non-aqueous phase, a step of mixing said pluralities of insulated reaction spaces of all the separate samples in said non-aqueous phase together, before subsequently a detection reaction of said analytes of interest is performed, and before detecting and/or quantitating said analytes of interest.

32. The method according to any of embodiments 22 - 31, wherein, in said step of detecting and quantitating said analyte of interest, quantitation of said analyte is performed by a method selected from:

  a) digital nucleic acid amplification, in particular digital polymerase chain reaction (PCR);
  b) real-time quantitative nucleic acid amplification, in particular real-time polymerase chain reaction (PCR);
  c) immunochemistry detection methods, in particular digital immunochemistry detection methods, such as digital immunoassays, e.g. digital enzyme-linked immunosorbent assays (ELISAs);
  d) immunochemistry detection methods combined with nucleic acid amplification, such as immuno-polymerase chain reaction; in particular digital immune-PCR;

wherein quantitation is performed using any of methods a) or b), or a combination of a) and b), if the analyte of interest is a nucleic acid; and wherein quantitation is performed using any of methods c) or d), if the analyte is a protein, peptide or other non-nucleic acid analyte.

DETAILED DESCRIPTION OF THE INVENTION

The present inventors have devised a library of prefabricated precursor-microparticles which serves as a starting point for making a ready-to-use and/or "ready-to-fill" library of prefabricated microparticles. Such library of prefabricated microparticles is intended and configured to perform a specific detection of one or several, preferably multiple, analytes of interest in a sample, according to user needs. Such library of prefabricated microparticles is extremely versatile and can be made in a customized way by an end-user who selects the specific analyte(s) to be detected in a sample. As used herein, the "library of prefabricated precursor-microparticles" that serves as a starting point for making a "library of prefabricated microparticles" differs from such "library of prefabricated microparticles" only in that the respective prefabricated precursor-microparticles do not, yet, comprise an analyte-specific reagent (herein also sometimes abbreviated as "ASR"), or several such analyte-specific reagents ("ASRs"), attached. However, such precursor-microparticles can be easily converted into ready-to-use microparticles by attaching an analyte-specific reagent to such precursor-microparticles. Because the precursor-microparticles differ from the respective microparticles only in the attached analyte-specific reagent(s), the qualities and details of the respective prefabricated microparticles are the same, as described for the respective precursor-microparticles, from which they originate. Hence, in the library of prefabricated precursor-microparticles, each of said prefabricated precursor-microparticles in the library comprises a porous matrix, preferably a porous polymeric matrix, having a void volume for receiving an aqueous sample and for providing a reaction space for the specific detection of an analyte. The same applies also to the respective resultant microparticles. Specific detection of an analyte using such microparticles is performed by a chemical or biochemical reaction within the aforementioned reaction space provided by the porous matrix of the microparticles. Typically, such chemical or biochemical reaction is a target (analyte) amplification reaction or a signal amplification reaction. Typical examples of a target amplification reaction are nucleic acid amplification reactions, such as PCR. Typical examples of signal amplification reactions are immunochemistry reactions, such as immunoassays.
Furthermore, each of said prefabricated precursor-microparticles in the library (and each of said prefabricated microparticles in the library) comprise a reagent binding component allowing the attachment, preferably the reversible attachment, of an analyte-specific reagent to the precursor-microparticle. As outlined above, such reagent binding component is one of:

(i) a polymer or polymer mixture that forms said porous matrix or is said polymer matrix;
(ii) a reagent binding molecule attached to said porous matrix;
(iii) at least one ionisable group, or a plurality of ionisable groups, immobilized on said porous polymer matrix, said ionisable group(s) being capable of changing its(their) charge(s) according to ambient conditions surrounding said precursor-microparticle;
(iv) at least one charged group, or a plurality of charged groups immobilized on said porous matrix;
(v) a combination of any of (i) - (iv).

Moreover, each of said prefabricated precursor-microparticles in the library comprise a label component that is attached to, contained within or otherwise associated with the precursor-microparticle, and the same also applies to the respective prefabricated microparticle resulting from such prefabricated precursor-microparticle. The label component attached to, contained within or otherwise associated with the precursor-microparticle or the microparticle serves the purpose for identifying (in the sense of "uniquely labelling" or "uniquely marking") an analyte-specific reagent, when attached to the precursor-microparticle. Examples of such labels are given in (Mandecki, Ardelt et al. 2006, Wilson, Cossins et al. 2006, Birtwell and Morgan 2009) and others. The label component attached to, contained within or otherwise associated with precursor-microparticle is of particular relevance, when it comes to considerations pertaining to an entire library of prefabricated precursor-microparticles (or an entire library of prefabricated microparticles): As used herein, the term "library" is meant to refer to a plurality or collection of microparticles, wherein, in such plurality or collection or library of microparticles, there are at least two different types of such microparticles, herein also sometimes referred to as "subsets". In a preferred embodiment, there are at least two subsets of prefabricated precursor-microparticles (or microparticles), preferably three or more subsets of prefabricated precursor-microparticles (or microparticles), with each subset having its distinct label component attached to, contained within or otherwise associated with said precursor-microparticles (or microparticles) within said subset, such that said at least two or more subsets of prefabricated precursor-microparticles (or microparticles) differ by the respective label component attached to, contained within or otherwise associated with each subset. In such a library of different subsets, the respective subsets are typically and preferably stored separately from each other, e. g. in different containers. This means that such term "separate subset", as used herein, is meant to refer to a collection or subset of precursor-microparticles or microparticles that is physically separated from other subsets. Such physical separation may be achieved by a barrier surrounding such subset. In a simple embodiment, such separate subset may be located in its own container or compartment. Typically, multiple subsets may be combined to form a library targeting different analytes/targets (with different subsets targeting different analytes), which library may subsequently be used for performing an assay that leads to a distribution of analytes/targets to be detected/quantitated in a sample, across the microparticles representing the multiple subsets within the library.

In a preferred embodiment, the label component that identifies each subset of precursor-microparticles (and of the microparticles resulting therefrom) is a mixture of at least two different dyes, preferably at least two fluorescent dyes, which are present, preferably being attached, on each subset of said precursor-microparticles (and of the microparticles resulting therefrom) at a respective defined ratio and/or in defined amounts of said at least two different dyes. Consequently, in such embodiment, the different subsets of precursor-microparticles differ from each other in terms of the respective ratio and/or amounts of the at least two different dyes, thus allowing a distinction between the different subsets of precursor-microparticles by the respective ratios and/or amounts of the at least different dyes attached to the respective subset of precursor-microparticles.

In another embodiment, the label component may be contained within, the precursor-microparticles. Again, such label component may be a mixture of at least two different dyes that is contained within the precursor-microparticles, and the respective different subsets of precursor-microparticles differ from each other in terms of the respective ratio and/or amounts of said at least two different dyes, such that the different subsets of precursor-microparticles differ from each other in terms of the respective ratio and/or amounts of the at least two different dyes contained within the microparticles, thus allowing a distinction between different subsets of precursor-microparticles by the respective ratios and/or amounts of said at least two different dyes contained within the respective subset of precursor-microparticles.

The subset specific labeling (or encoding) may be achieved as previously established (Fulton, McDade et al. 1997).

In yet other embodiments, a labelling of the respective precursor-microparticles (and of the corresponding microparticles) may also be achieved via different shapes of the respective microparticles. This would be an example for an embodiment of precursor-microparticles (and of corresponding microparticles), where the label component is not attached to or contained within the precursor-microparticles, but is "otherwise associated" with the precursor-microparticles. Such an embodiment presupposes the concomitant presence of at least two different label components, i.e. in this case shapes of precursor-microparticles by which the respective precursor-microparticles thus labelled may be distinguished. In this example, different shapes can mean different external shapes, different internal structures, different internal volumes within the porous matrix etc.

Preferably, the porous matrix of said precursor-microparticles (and of said microparticles) is a porous polymeric matrix, implying that the respective precursor-microparticles (and said microparticles resulting therefrom) are made of or comprise a polymer or a polymer mixture.

As outlined above, the library of prefabricated precursor-microparticles may be converted into a library of prefabricated microparticles by attaching an analyte-specific reagent, preferably by reversibly attaching such reagent, to said precursor-microparticles through a reagent binding component. Different subsets of microparticles may have the same analyte-specific reagent attached, or they may have different analyte-specific reagents attached.

As used herein, the term "analyte-specific reagent" ("ASR") is meant to refer to a reagent that is capable of specifically targeting or recognizing an analyte of interest. Such specific targeting or such specific recognition manifests itself in the capability of such analyte-specific reagent to specifically bind to such analyte of interest or to specifically react therewith. In one embodiment, an analyte-specific reagent is selected from nucleic acids, including aptamers, spiegelmers, nucleic acid oligomers and nucleic acid primers; antibodies or antibody fragments; non-antibody proteins capable of specifically binding an analyte or analyte complex, and affinity proteins. In a preferred embodiment, an analyte-specific reagent is selected from nucleic acids, in particular nucleic acid oligomers and nucleic acid primers. Nucleic acid primers are particularly suitable for performing a nucleic acid amplification. In one embodiment, when the analyte-specific reagent is a nucleic acid primer, such analyte-specific reagent is, in fact, a pair of primers which flank the region within an analyte of interest that will subsequently be amplified (and detected). Optionally, in such an embodiment (of a pair of primers being an analyte-specific reagent), such analyte-specific reagent may additionally comprise a detectable probe provided together with the primer(s) and allowing the detection of the respective primer(s) and the resultant amplification product(s).

The term "analyte of interest", as used herein, is meant to refer to any molecular entity a detection and/or quantitation of which within a sample may be desirable. Sometimes such term "analyte" is used synonymously herein with the term "target". In one embodiment, an analyte of interest may be a nucleic acid; in another embodiment, an analyte of interest may be a protein, peptide, or other non-nucleic acid entity.

The libraries in accordance with the present invention and the respective microparticles or precursor microparticles can be dried, e.g. freeze-dried. In accordance with embodiments of the present invention, a gel forming agent may be used for forming prefabricated precursor microparticles (and the respective microparticles resulting therefrom), and such gel-forming agent is as defined further above. In one embodiment, such gel forming agent is used to form precursor-microparticles (and the respective microparticles resulting therefrom) which may subsequently be dried, preferably freeze-dried. After drying the particles may be stored as a powder. In some embodiments involving a gel-forming agent, such gel-forming agent forms a gel that is furthermore capable of undergoing a transition to a sol-state. In particular such transition may occur upon application of an external trigger, such as a change of temperature, pH or salt conditions. The present inventors have surprisingly found that by providing entire libraries of prefabricated microparticles or precursors thereof in accordance with the present invention, it is possible to provide miniaturized and versatile tools that can be used in a point-of care environment and act as defined reaction spaces that may be used in a very versatile manner for detection reactions, for example for performing a digital detection of an analyte in a sample. Because the microparticles serve as or provide confined reaction spaces, they are herein also sometimes referred to as "nanoreactors". The prefabricated microparticles (or "nanoreactors"), in accordance with embodiments of the present invention, may be tailor-made by choosing appropriate analyte-specific reagents to be attached. With respect to different subsets of microparticles within a library, there may be the same or different analyte-specific reagents attached.

As used herein, the terminology "same analyte-specific reagent" and "different analyte-specific reagent" is meant to refer to the fact that when two analyte-specific reagents are the same, this means that they target and/or recognize the same analyte of interest, whereas, if they are different, they target and/or recognize different analytes of interest. Accordingly, in a library of prefabricated microparticles, wherein different subsets of microparticles differ from each other by the respective analyte-specific reagent attached or contained within, this means that these different subsets will target and/or recognize different analytes of interest. If, on the other hand, different subsets have the same analyte-specific reagent attached or contained within, this means that they recognize and/or target the same analyte of interest.

The term "microparticle", as used herein, is meant to refer to a particle the average dimensions of which are in the micrometer range. In one embodiment, the microparticles in accordance with the present invention have an average size or average dimension or average diameter of approximately 1 $\mu$m - 200 $\mu$m, preferably 5 $\mu$m - 150 $\mu$m, more preferably 10 $\mu$m - 100 $\mu$m. In one embodiment, the microparticles in accordance with the present invention are spherical or oval or ellipsoidal, preferably spherical, and the above-mentioned dimensions refer to the average diameter of such spherical, oval or ellipsoidal microparticle. In one embodiment, the microparticles have the shape of a (spherical) droplet. In another embodiment, a microparticle in accordance with the present invention is a spherical body or a quasi-spherical body, i. e. having the shape of a sphere (or nearly approaching it), such sphere having an average diameter of the aforementioned dimensions. Typically, microparticles in accordance with the present invention are porous and have a porous polymeric matrix having a void volume for receiving an aqueous sample and for providing a reaction space for the specific detection of an analyte.

As used herein, a precursor-microparticle is converted into a microparticle by attaching an analyte-specific reagent thereto. Thus, a library of prefabricated precursor-microparticles serves the purpose for making a library of prefabricated microparticles. The respective and resultant microparticles serve the purpose for performing a specific detection and/or quantitation of one or several analytes of interest in a sample, depending on how many different analyte-specific reagents are attached to the microparticles (as for example defined by a user). If there is only one analyte-specific reagent (or only one analyte-specific "reagent set" comprising multiple components necessary to detect a single analyte, e.g. amplification primer(s) for a target nucleic acid and, optionally, a detection probe) attached, such library can only be used to detect a single analyte of interest, albeit still in a plurality of samples that may be distinguished by virtue of the respective label component attached; if different analyte-specific reagents are attached to different microparticles within such library of prefabricated microparticles, such library can be used to detect different analytes of interest, in one or several samples.

In a preferred embodiment, the analyte-specific reagent(s) is (are) reversibly attached to the respective precursor-microparticle. Preferably, such reversible attachment occurs by virtue of the precursor-microparticle(s) having a reagent binding component that allows the reversible attachment of an analyte-specific reagent to the precursor-microparticle. In a preferred embodiment, such reagent binding component is a reagent binding molecule that is attached to the porous polymer matrix of the precursor-microparticle. Such reagent binding molecule is designed and intended to bind to a binding entity which, in turn, is conjugated to the analyte-specific reagent. In this embodiment, there is thus an interaction between a reagent binding molecule attached to the porous polymer matrix of the precursor-microparticle(s), on the one hand, and a binding entity to which the analyte-specific reagent is conjugated, on the other hand. As an example, the reagent binding molecule attached to the porous polymer matrix may be streptavidin or a derivative thereof, and the binding entity to which the analyte-specific reagent is conjugated may be biotin or a biotin derivative, such as desthiobiotin, or vice versa (i.e. reagent binding molecule being biotin or a biotin derivative, such as desthiobiotin, and the binding entity being streptavidin or a derivative thereof). If, for example, the analyte-specific reagent is a nucleic acid primer (or a pair of nucleic acid primers, and, optionally, a detection probe), such primer(s) may be easily conjugated to desthiobiotin or are even commercially provided in such desthiobiotinylated form. On the other side, the porous polymer matrix may have reagent binding molecules attached which may be streptavidin. It is extremely simple and easy for an end-user, under ambient conditions, to select a pair of desthiobiotin-primers, specific for an analyte of interest, and reversibly attach this to the precursor-microparticles having streptavidin attached to their porous polymer matrix. A binding event between a biotin derivative and streptavidin under ambient conditions is easy to achieve by simply exposing the precursor-microparticles to a solution of such biotin-derivative-labeled primers, under suitable conditions. A "conditioning solution" which generates conditions that facilitate binding may be used. Such conditioning solution may for example be a buffer, providing an appropriate pH and salt environment. On the other hand, if subsequently, for example during a nucleic acid amplification, the temperature is raised, the analyte-specific reagents can easily become detached again from the microparticles, because the binding between the reagent binding molecule attached to the porous polymer matrix of the precursor-microparticle(s) (e.g. streptavidin), on the one hand, and the binding entity on the analyte-specific reagent (e.g. desthiobiotin on the primer(s)) is not possible anymore under elevated temperature conditions. After such detachment, the analyte-specific reagent(s) are thus fully available for any amplification reaction occurring.

The term "protein, derived from or related to, avidin", as used herein, is meant to refer to any protein that is similar in terms of its structure or sequence to avidin and has or retains binding functionality of avidin.

The term "molecule, derived from or related to, biotin", as used herein, is meant to refer to any molecule that is similar in terms of its structure to biotin and has or retains binding functionality of biotin.

In one embodiment of the respective library, each of the prefabricated precursor microparticles or microparticles in the library comprise a porous polymeric matrix having a void volume for receiving an aqueous sample and for providing a reaction space for the specific detection of an analyte. Such porous polymeric matrix is formed by a polymer or polymer mixture and more preferably is formed by or is a hydrogel-forming agent or a mixture of such hydrogel-forming agents. In a preferred embodiment, the polymer or polymer mixture forming the porous polymeric matrix has the capability of switching between a gel-state and a sol-state. In one embodiment, the polymer or polymer mixture that forms or is part of the porous polymeric matrix is not a cross-linked polymer. In a particularly preferred embodiment, the polymer is agarose and more preferably a combination of agarose and gelatin. If a combination of agarose and gelatin is used, it is preferred that the agarose is present in a range of from 0.1% (w/v) to 4% (w/v), and the gelatin is present in a range of from 0.1% (w/v) to 20% (w/v), preferably 0.5% (w/v) to 20% (w/v). In one particular embodiment, the concentration of agarose in said porous polymeric matrix is 0.5% (w/v), and the concentration of gelatin is in the range of from 1% (w/v) to 2% (w/v).

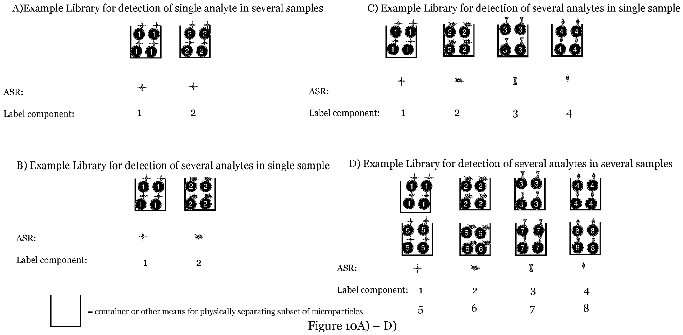

The technology devised by the present inventors is highly versatile in that it allows to detect multiple analytes in a sample, by using a library of prefabricated microparticles that have as many different analyte-specific reagents attached as there are analytes to be detected in a sample. Hence, according to one embodiment, in a library of prefabricated microparticles, there are at least two subsets of prefabricated microparticles, preferably three or more subsets of prefabricated microparticles, wherein each subset has its distinct label component attached to, contained within or otherwise associated with the microparticles of said subset; and each subset has a distinct analyte-specific reagent attached to the microparticles of the subset; such that said at least two or more subsets of prefabricated microparticles differ by the respective label component attached or contained within, and by the respective analyte-specific reagent attached to each subset; with each subset thus being unambiguously defined and identifiable by the respective label component and by the respective analyte-specific reagent. In such library, there are as many or at least as many separate subsets of microparticles provided, as there are different analytes of interest to be detected, with each of said separate subsets having a different analyte-specific reagent attached to the porous matrix of said microparticles of said subset; each analyte-specific reagent being specific for one analyte of interest. Such a library is described, inter alia, in embodiment 12 and is claimed in claim 10, its use is described, inter alia, in embodiment 30 and is claimed in claim 23, and examples thereof are shown and further described with reference to Figures 10B), 10C), and 10E).

In case there is only one analyte of interest to be detected, but, possibly, in different samples, the present invention, in one embodiment also provides for a library of prefabricated microparticles, wherein, in said library, there are at least two subsets of prefabricated microparticles, preferably three or more subsets of prefabricated microparticles, wherein each subset has its distinct label component attached to, contained within or otherwise associated with said microparticles of said subset; and all of said at least two, three or more subsets have the same analyte-specific reagent attached to said microparticles of said subsets; such that said at least two or more subsets of prefabricated microparticles are identical in terms of the analyte-specific reagent attached, but differ by the respective label component attached to, contained within or otherwise associated with said microparticles of each subset. This particular library is particularly useful for the detection of a single analyte in a multiplicity of samples, namely, preferably, in as many samples as there are subsets within the library. To put it differently, in such library, there are as many or at least as many separate subsets of microparticles provided, as there are different aqueous samples provided and to be tested, wherein all of said separate subsets have the same analyte-specific reagent attached to the porous matrix of said microparticles of said subsets, said analyte-specific reagent being specific for one analyte of interest. Such a library is described, inter alia, in embodiment 11 and is claimed in claim 9, its use is described, inter alia, in embodiment 29 and is claimed in claim 22, and examples thereof are shown and further described with reference to Figure 10A).

The technology devised by the present inventors moreover also allows to detect multiple analytes in several samples, by using a library of prefabricated microparticles that have as many different analyte-specific reagents attached as there are analytes to be detected in a sample, and in said library, there are altogether a plurality of different separate subsets of microparticles the total number of which equals the number of samples to be tested, multiplied by the number of analytes to be detected and/or quantitated. Hence, according to one such embodiment, in such a library of prefabricated microparticles, there are different separate subsets of microparticles, with each subset having its distinct label component attached to, contained within or otherwise associated therewith. Moreover in such a library, there are different classes of separate subsets of prefabricated microparticles, with each of said classes comprising several subsets of microparticles that, within such class, have the same analyte-specific reagent attached thereto; whereas subsets of microparticles that belong to different classes have a different analyte-specific reagent attached to the porous matrix of said microparticles. In other words, all subsets of microparticles within one class have the same analyte-specific reagent attached (and are therefore intended to be used to detect and quantitate the same analyte, with however, different subsets within one class being intended to be used with different samples. If such a library is used in a method for detecting and/or quantitating multiple analytes in a plurality of samples, in such library, there are

- as many, or at least as many, different classes of separate subsets of microparticles provided, as there are different analytes to be detected,
- as many, or at least as many, different subsets of microparticles provided within each class , as there are aqueous samples provided and to be tested,
- as many, or at least as many, different subsets of microparticles provided within the library in total as there are different aqueous samples to be tested multiplied by the number of different analytes of interest to be detected,

Such a library is described, inter alia, in embodiment 13 and is claimed in claim 11, its use is described, inter alia, in embodiment 31 and is claimed in claim 24, and examples thereof are shown and further described with reference to Figures 10D), 11A) and 11B).

The versatility of the present invention is also reflected by the fact that the libraries according to the present invention can be manufactured extremely easily by an appropriate kit that can be used by an end-user who decides which analytes are of interest and should therefore be detected and how many samples must be investigated.

Accordingly, in a further aspect, the present invention relates to a kit for making a library of prefabricated microparticles, as defined above, wherein the kit comprises:

- at least two containers, namely a first and a second container, and optionally further containers, each container containing:

  - a subset of prefabricated precursor-microparticles as defined further above, with each subset having its distinct label component attached to, contained within or otherwise associated with said precursor-microparticles within said subset, such that said two subsets of prefabricated precursor microparticles contained in said at least two containers, differ by the respective label component attached to, contained within or otherwise associated with;

- a further container containing:

  - a conditioning solution to enable the reversible attachment of an analyte-specific reagent to said subset of said precursor-microparticles through said reagent binding component of said precursor-microparticles, as defined further above, by

    a) direct binding of said analyte-specific reagent to said polymer mixture that forms said porous polymeric matrix or is part of said porous polymeric matrix (i);
    b) said analyte-specific reagent being conjugated to a binding entity which, in turn, binds to said reagent binding molecule (ii), as defined further above;
    c) direct binding of said analyte-specific reagent to said ionizable group(s) (iii), as defined above, under conditions in which said ionizable group(s) has (have) a suitable net charge;
    d) direct binding of said analyte-specific reagent to said charged group(s) (iv), as defined above, on said polymer, wherein said analyte-specific reagent has at least one ionizable group, or a plurality of ionizable groups, said ionizable group(s) being capable of changing its (their) charge(s) according to ambient conditions surrounding said analyte-specific reagent; or
    e) a combination of any of a)-d); wherein the reagent binding component, the reagent binding molecule, the binding entity, the polymer or polymer mixture, the ionizable groups, and the analyte-specific reagent being as defined further above.

Using such an embodiment, an end-user is capable of producing its own library of prefabricated microparticles that is specific for a selected number and type of analytes of interest and can be used with a selected number of samples. Attachment of the respective and desired analyte-specific reagents, e. g. primers or antibodies, occurs simply by exposing said prefabricated precursor-microparticles to said analyte-specific reagent(s) under appropriate conditions, preferably established or generated by the aforementioned conditioning solution, such as a buffer.

If in one embodiment, a multiplicity of analytes of interest are to be detected in a single sample, the corresponding library of prefabricated microparticles will be produced using a kit according to the present invention, in which there are different subsets of microparticles, each subset being specific for a specific analyte of interest, and each subset being different in terms of the respective label component attached to, contained within or otherwise associated with each subset. Such a library is described, inter alia, in embodiment 12 and is claimed in claim 10, its use is described, inter alia, in embodiment 30 and is claimed in claim 23, and examples thereof are shown and further described with reference to Figures 10B), 10C), and 10E).

If, on the other hand, the detection of a single analyte is desired and intended, but in a plurality of samples, for example from different patients, a corresponding library will be produced in which there are different subsets of prefabricated microparticles, with all the microparticles having the same analyte-specific reagent attached, but still being different in terms of the respective label component attached to, contained within or otherwise associated with each subset. Such a library is described, inter alia, in embodiment 11 and is claimed in claim 9, its use is described, inter alia, in embodiment 29 and is claimed in claim 22, and examples thereof are shown and further described with reference to Figure 10A).

As a third possibility, a library may also be provided which is useful for detecting more than one analyte of interest, for example two analytes of interest, **and** in a plurality of samples. In such a library, each subset of microparticles will be different by the respective label component attached to, contained within or otherwise associated with each subset, but there will be pairs, or triples or quadruples, or n-tuples of subsets that have the same analyte-specific reagent attached (if two, three, four or n samples are to be tested for a particular analyte of interest. Moreover, in such a library, there will be pairs, or triples, quadruples, or n-tuples of subsets of microparticles with each subset within such pair, ... n-tuple having a different analyte-specific reagent attached (if two, three, four or n different analytes of interest are to be detected). Such a library is described, inter alia, in embodiment 13 and is claimed in claim 11, its use is described, inter alia, in embodiment 31 and is claimed in claim 24, and examples thereof are shown and further described with reference to Figures 10D), 11A) and 11B).

In one embodiment, the kit for making a library of prefabricated microparticles may further comprise a further container that contains a washing buffer or washing solution for removing free, i. e. unattached, analyte-specific reagent(s) from microparticles, without removing analyte-specific reagent(s) attached to said microparticles. In a further embodiment, the kit for making the prefabricated microparticles further comprises at least one mixing container for mixing components together, preferably several mixing containers, more preferably as many mixing containers as there are containers containing subsets of precursor-microparticles.

In one embodiment, the mixing of components together may occur in the same container(s) that contains (contain) the respective subset of prefabricated precursor-microparticles. However, in other embodiments, it may be prudent to separately provide for one or several of such specifically designated mixing containers. This may be particularly useful, when subsets of microparticles are pooled after they have been separately provided with different analyte-specific reagents.

In a further aspect, the present invention also relates to a method of making a library of prefabricated microparticles, wherein, in such method, a library of prefabricated precursor-microparticles and at least one analyte-specific reagent, both as defined above are provided in any order. In such method, said library of prefabricated precursor-microparticles, or selected subsets thereof, and the at least one analyte-specific reagent are mixed under conditions allowing the attachment, preferably the reversible attachment, of said at least one analyte-specific reagent to some or all of said prefabricated precursor-microparticles, thus generating a library of prefabricated microparticles according to the present invention.

Optionally, the method further comprises washing said prefabricated microparticles to remove any unattached analyte-specific reagent therefrom.

In one embodiment, such method may be performed by using a kit for making a library as defined above, wherein said library is provided in the form of said at least two containers of said kit, with each container containing a subset of prefabricated precursor-microparticles as defined further above, with each subset having its distinct label component attached to, contained within or otherwise associated with said precursor-microparticles within said subset, such that said at least two subsets of prefabricated precursor-microparticles differ by the respective label component attached to, contained within or otherwise associated with each subset;
wherein said conditions allowing the attachment, preferably the reversible attachment, of said at least one analyte-specific reagent to some or all of said prefabricated precursor-microparticles, are generated by mixing said library of prefabricated precursor-microparticles, or selected subsets thereof, and the at least one analyte-specific reagent in the presence of a conditioning solution, as defined above, wherein, preferably, said conditioning solution is a buffer.

In a preferred embodiment of the method of making a library of prefabricated microparticles, as defined further above, the method comprises:

- providing, in any order:

- a library of prefabricated precursor-microparticles, each subset being provided in a separate container, as defined further above, OR several subsets of prefabricated precursor-microparticles are provided together in a separate container ;
- at least one analyte-specific reagent, as defined further above;
- mixing the subsets of said library of prefabricated precursor-microparticles separately with one or more analyte-specific reagents, or selected combined subsets of said library of prefabricated precursor-microparticles, and the at least one analyte-specific reagent under conditions allowing the attachment, preferably the reversible attachment, of said at least one analyte-specific reagent to some or all of said prefabricated precursor-microparticles, thus generating a library of prefabricated microparticles as defined further above;
- optionally, washing said prefabricated microparticles to remove any unattached analyte-specific reagent therefrom.
- (optionally) combining the individual or mixed subsets into one library of microparticles with different subsets with analyte specific reagents selectively attached to defined subsets of the library.

In a further aspect, a kit for making a library of prefabricated microparticles, as defined above, herein also sometimes referred to as a "manufacturing kit", may serve as a platform for providing a kit for detecting an analyte. The latter kit is herein also sometimes referred to as a "detection kit", as opposed to the "manufacturing kit", that is the kit for making a library of prefabricated microparticles.

In a further aspect, the present invention also relates to a kit for detecting an analyte in a sample ("detection kit"), said detection kit comprising:

a)

- a container containing a generic detection composition for nucleic acid amplification, said generic detection composition comprising reagents for performing a chemical or biochemical detection reaction of an analyte, wherein said chemical or biochemical detection reaction of an analyte is a nucleic acid amplification, said generic detection composition comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid,
  OR
- a container containing a first detection composition comprising reagents for performing a chemical or biochemical detection reaction of an analyte, wherein said chemical or biochemical detection reaction of an analyte is an immunochemistry detection reaction, and yet a further container containing a second detection composition comprising a detection reagent, said first detection composition comprises reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry detection reaction; and said second detection composition comprises, as a detection reagent, a suitable substrate for said reporter enzyme which substrate upon having been reacted by said reporter enzyme, becomes detectable, preferably optically detectable, more preferably detectable by fluorescence,
  OR
- a container containing a first detection composition comprising reagents for performing a chemical or biochemical detection reaction of an analyte, wherein the chemical or biochemical reaction is an immunochemistry detection reaction, and yet a further container containing a second detection composition comprising a detection reagent, said first detection composition comprises reagents for performing an immunochemistry detection reaction, such as a buffer, and a secondary antibody or secondary antibody fragment coupled to a suitable oligonucleotide tag and being specific for the same analyte as a primary antibody, used as analyte-specific reagent (ASR) in said immunochemistry detection reaction; and said second detection composition comprises, as detection reagent(s), a buffer, mono-nucleoside-triphosphates, an amplification enzyme, such as a suitable nucleic acid polymerase, e. g. Taq polymerase, and a nucleic acid dye for detection of an amplification product, such as an amplified nucleic acid, and primers suitable for amplifying the oligonucleotide tag attached to the secondary antibody;
  AND

b)

- a container containing a non-aqueous phase, such as an oil, e.g. fluorocarbon oil, optionally supplemented with an emulsifier;

and

c)

- a mixing container for mixing components.

In one embodiment, the detection kit may comprise d) a further container for performing a detection reaction.

According to the invention, the detection kit is to be used in conjunction with a library of microparticles, as defined herein and further above.

Accordingly, in some embodiments, the detection kit may also comprise a library of prefabricated microparticles, as defined further above. In such a library of microparticles in accordance with the present invention, the microparticles comprise an analyte-specific reagent, also as defined above. It is these microparticles comprising analyte-specific reagents which act as defined reaction spaces for performing the detection through a chemical or biochemical reaction. The respective analyte-specific reagent specifically targets or recognizes the analyte of interest.

In other embodiments, the library of prefabricated microparticles as defined above, is provided separate from the kit and does not form part thereof.

In the detection kit according to embodiments of the present invention, component a) effectively is the determining component for the type of detection to be used. Hence, there are various possibilities for component a): Depending on what detection is intended to be performed and, thus, depending on the analyte to be detected, such component a) may be configured either for performing a detection in the context of a nucleic acid amplification, or for performing a detection in the context of an immunochemistry detection, or for performing a detection in the context of an immune-amplification reaction, such as e.g. an immuno-PCR.

Accordingly, such component a) may be either a container which contains a generic detection composition to be used in the context of a nucleic acid amplification. Such generic detection composition to be used in the context of a nucleic acid amplification comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid.

Or, in the context of an immunochemistry detection, component a) maybe a combination of two separate containers wherein the first container of such combination contains a first detection composition which comprises reagents for performing an immunochemistry detection, such as a buffer, and a secondary antibody or secondary antibody fragment, coupled to a suitable reporter enzyme and being specific for the same analyte as a primary antibody, antibody fragment, or non-antibody protein, that is used as analyte-specific reagent in the immunochemistry reaction. In this context, the term "specific for the/an analyte", when used in the context of a secondary antibody, is meant to mean that the secondary antibody will be able to specifically recognize or target the same analyte as the primary antibody, antibody fragment, or non-antibody protein, that is used as analyte-specific reagent, irrespective of whether such analyte is free or already bound to the primary antibody, fragment ... etc. that is used as analyte-specific reagent in the immunochemistry detection reaction. The primary antibody, antibody fragment, or non-antibody protein, that is used as analyte-specific reagent, is part of the microparticles as defined above, and the library of these microparticles, as defined above, may form part of the detection kit, or may be provided separate from such kit.

The third option for component a) is an option for a detection in the context of an immune-amplification reaction, such as e.g. an immuno-PCR. In this embodiment, component a) is a combination of two separate containers wherein the first container comprises a first detection composition which comprises reagents for performing an immunochemistry detection reaction, such as a buffer, and a secondary antibody or secondary antibody fragment coupled to a suitable oligonucleotide tag and being specific for the same analyte as the primary antibody, antibody fragment or non-antibody protein used as analyte-specific reagent; and the second container comprises a second detection composition, and such second detection composition comprises, as detection reagent(s), a buffer, mono-nucleoside-triphosphates, an amplification enzyme, such as a suitable nucleic acid polymerase, e. g. Taq polymerase, and a nucleic acid dye for detection of an amplification product, such as an amplified nucleic acid, and primers suitable for amplifying the oligonucleotide tag attached to the secondary antibody.

The libraries according to the present invention are extremely easy to make and extremely versatile to use in a wide variety of analytical or diagnostic applications:

For example, as already outlined further above, a library according to one embodiment of the present invention may be used in a method for detecting a single analyte in a plurality of different samples. The respective subsets are typically provided in separate containers allowing for interrogating the subsets with the respective samples. In such an embodiment, the number of separate subsets of microparticles present in said library at least equals (or sometimes is greater than) the number of samples in said plurality of different samples. If the number of separate subsets of microparticles present in said library is greater than the number of samples in said plurality of different samples, it is not necessary to use the entire library (including all the subsets). Such a library is described in embodiment 11.

As another example, and as also already outlined further above, a library according to one embodiment of the present invention may be used in a method for detecting multiple analytes in a single sample. The subsets of the library may be provided combined in a single container, or may be combined into a single container, after initially having been provided in separate containers, and are interrogated simultaneously with the sample. In such an embodiment, the number of separate subsets of microparticles present in said library at least equals (or sometimes is greater than) the number of analytes to be detected in said single sample. If the number of separate subsets of microparticles present in said library is greater than the number of analytes to be detected in said single sample, it is not necessary to use the entire library (including all the subsets). Such a library is described in embodiment 12.

As another example, a library according to one embodiment of the present invention may be used in a method for detecting multiple analytes in a plurality of different samples. In this case, the library comprises sub-libraries of different subsets, with each subset representing a different class of analyte-specific reagents, and each sub-library being used to be interrogated with a different sample. Such a library is described in embodiment 13.

As used herein, the terms "sublibrary" and "class" are meant to designate the following:
The term "class", as used herein, is meant to refer to the entirety of all subsets of microparticles within the library that have the same analyte-specific reagent (ASR). All of the subsets within one class are specific for one analyte of interest. Within one class, the different subsets differ from each other by their respective label component. Within one class, the different subsets are kept in separate containers, because each of said different subsets is used for interrogating a different sample.

The term "sublibrary", as used herein in the context of a method for detecting multiple analytes in a plurality of different samples, is meant to refer to the entirety of all subsets of microparticles within the library that are used to interrogate one particular sample at a time. Because there will be a plurality of different samples, the library therefore comprises different sublibraries of separate subsets of prefabricated microparticles, with each of said sublibraries being used to interrogate a different sample. Within each sublibrary, each subset of prefabricated microparticles within one sublibrary has a different analyte-specific reagent attached; each analyte-specific reagent being specific for one analyte of interest.
Effectively, therefore each sublibrary contains one subset of microparticles from each class, such that within each sublibrary, each subset of microparticles has a different analyte-specific reagent attached.

The term "interrogate a sample", as used herein in the context of a subset that is being used to "interrogate" a sample, is meant to refer to a scenario wherein such subset is exposed to or incubated with a particular sample, allowing the subset of microparticles to take up sample into the void volume of the microparticles.

The terms "incubate", "interrogate", and "expose to", when used in the context of a sample that is incubated with or interrogated with a library of microparticles, or in the context of a library of microparticles which is exposed to a sample are all meant to designate a scenario in which sample and library are brought in contact with each other for a time sufficient to allow the microparticles to take up sample into their void volumes.

The term "sample" or "aqueous sample", as used herein, is meant to refer to any suitable fluid, isolated from an environment, e. g. body fluid, or components thereof to be analyzed for the presence of one or several analytes and/or its (their) respective quantity (quantities). For example, the sample may be an aqueous solution to be analyzed, for example obtained from a particular environment, such as patient, in particular from a patient tissue or patient fluid. Such sample may be a body fluid or a component thereof. Examples are blood, blood plasma, serum, saliva, urine, tears, sweat, lymph, semen, and cerebrospinal fluid. The term may also refer to samples that have undergone some processing steps such as nucleic acid extraction, cell depletion, analyte enrichment etc. and that represent a liquid containing the analyte and to be subjected to the reagents and steps outlined in this invention. In one embodiment, the term "sample" or "aqueous sample", as used herein, refers to a liquid sample obtained from the body of an organism which may or may not have been further processed, e.g. to make analyte(s) of interest accessible or amenable to further analysis. Preferably such "sample" or "aqueous sample" is selected from blood, plasma, serum, urine, sweat, tears, sputum, lymph, semen, ascites, amniotic fluid, bile, breast milk, synovial fluid, peritoneal fluid, pericardial fluid, cerebrospinal fluid, chyle, and urine, wherein, more preferably, said sample is plasma. Preferably such aqueous sample is further processed, e.g. it may be lysed or digested or otherwise treated, before it is subjected to the method according to the present invention. As an example, if the aqueous sample is plasma, such plasma may have been further lysed and/or digested to get rid of components that may otherwise interfere with the subsequent detection reaction. For example if the analyte of interest is a particular nucleic acid, the aqueous sample, e.g. the plasma, may be first digested with a protease and other suitable enzymes to remove any unwanted proteins or peptides, as well as lipids or other unwanted components, before it is subjected to the method according to the present invention. An "aqueous sample", as used herein, may therefore also refer to an extract obtained from any of the aforementioned bodily fluids; it may for example be a nucleic acid extract that has been obtained from any of the aforementioned bodily fluids by way of a suitable extraction, e.g. using cell disruption (if necessary), removal of lipids, proteins and unwanted nucleic acid (if necessary), and purification of nucleic acids and/or enrichment of particular nucleic acids. In some embodiments, a nucleic acid extract may have been produced using ethanol or another suitable alcohol.

According to embodiments of the present invention, in said library that is used in a method for detecting multiple analytes in a plurality of different samples, there are a plurality of different separate subsets of prefabricated microparticles, with each subset

- having its distinct label component attached to, contained within or otherwise associated with said microparticles of said subset; and

wherein, in said plurality of separate subsets of prefabricated microparticles, there are different classes of separate subsets of prefabricated microparticles, with each of said classes comprising several subsets of microparticles and each class having a different analyte-specific reagent attached to the porous matrix of said microparticles, and all subsets of microparticles within one class having the same analyte-specific reagent attached; each analyte-specific reagent being specific for one analyte of interest; and

such that, in said library, said plurality of different separate subsets of prefabricated microparticles differ by

- the respective label component attached to, contained within or otherwise associated with said microparticles of each subset; and

each separate subset of microparticles forms part of one class of subsets of microparticles;
with each subset being unambiguously defined and identifiable by said respective label component and said respective analyte-specific reagent; and
such that said different classes of subsets of microparticles differ by the respective analyte-specific reagent attached to the porous matrix of said microparticles; and each of said different classes comprises several subsets of microparticles, all of which subsets within one class having the same analyte-specific reagent attached.

When such a library is put into use in a method for detecting multiple analytes in a plurality of different samples, it should be noted that in such library there are a plurality of separate subsets of prefabricated microparticles, wherein in said plurality of separate subsets of prefabricated microparticles, there are different sublibraries of separate subsets of prefabricated microparticles, with each of said sublibraries being used to interrogate a different sample. Each of said sublibraries comprises several subsets of prefabricated microparticles and each subset within a sublibrary has a different analyte-specific reagent attached.

Such a library is described in embodiment 13.

One particularly useful feature of microparticles in accordance with some embodiments of the present invention is that they may also facilitate the enrichment of analyte(s) from samples by binding the analyte(s) to

(i) an analyte-specific reagent bound to the microparticle;
(ii) the polymer or polymer mixture that forms said porous polymer matrix or is said polymer matrix;
(iii) one or more ionisable group, or a plurality of ionisable groups, immobilized on said porous polymer matrix, said ionisable group(s) being capable of changing its(their) charge(s) according to ambient conditions surrounding said precursor-microparticle or microparticle;
(iv) one or more charged group, or a plurality of charged groups immobilized on said porous polymer matrix;
(v) a combination of any of (i) - (iv).

and thus can be used as a means for enrichment, clean up and detection/quantitation of analyte/target in a sample.

Typical examples of analyte-specific reagents bound to the microparticle that may actually be used to facilitate enrichment of an analyte from a sample (i.e. option (i) above), are antibodies or antibody fragments; non-antibody proteins capable of specifically binding an analyte or analyte complex, such as receptors, receptor fragments, and affinity proteins.

In a further aspect, the present invention relates to a method of detecting and/or quantitating an analyte of interest in an aqueous sample, said method comprising the steps:

- providing, in any order:

  • an aqueous sample known or suspected to contain an analyte of interest;
  • a generic detection composition comprising reagents for performing a chemical or biochemical detection reaction of the analyte;
  • a library of prefabricated microparticles according to any of the above defined embodiments of the present invention, wherein said analyte-specific reagent(s) attached to said microparticles, is(are) chosen such that it is (they are) capable of specifically binding to or reacting with said analyte of interest;

- optionally, mixing said aqueous sample and said generic detection composition;
- incubating said aqueous sample with said library of prefabricated microparticles, thereby allowing said library of microparticles to absorb aqueous sample in the void volumes of said microparticles and, optionally, to bind said analyte of interest, if present in said sample;
- optionally, washing said microparticles;
- adding said generic detection composition to the microparticles, if said aqueous sample has not been previously mixed with said generic detection composition;

  - transferring said library of prefabricated microparticles into a non-aqueous phase and removing any aqueous phase surrounding the individual prefabricated microparticle(s), thereby creating a plurality of insulated reaction spaces for detecting said analyte, which reaction spaces comprise an aqueous phase and are confined to said void volume(s) of said microparticles;
  - optionally, releasing the analyte-specific reagent(s) attached to said microparticles by applying an external trigger;
  - performing a detection reaction of said analyte of interest;
  - detecting and/or quantitating said analyte of interest.

Such a method is particularly useful, when the analyte(s) of interest is(are) a nucleic acid(nucleic acids), and the detection reaction is a nucleic acid amplification reaction.

In a further aspect, the present invention relates to a method of detecting and/or quantitating an analyte of interest in an aqueous sample, said method comprising the steps:

- providing, in any order:

  - an aqueous sample known or suspected to contain an analyte of interest;
  - a first detection composition comprising necessary reagents for performing a chemical or biochemical detection reaction of the analyte;
  - a second detection composition comprising a detection reagent;
  - a library of prefabricated microparticles according to any of the above defined embodiments of the present invention, wherein said analyte-specific reagent(s) attached to said microparticles, is(are) chosen such that it is (they are) capable of specifically binding to or reacting with said analyte of interest;

- optionally, mixing said aqueous sample and said first detection composition;
- incubating said aqueous sample with said library of prefabricated microparticles and, if said aqueous sample has not already been mixed with said first detection composition, also with said first detection composition, thereby allowing said library of microparticles to absorb aqueous sample and said first detection composition in the void volumes of said microparticles and, optionally, to bind said analyte of interest, if present in said sample;
- optionally, washing said library of prefabricated microparticles to remove any non-absorbed or non-reacted first detection composition;
- incubating said library of prefabricated microparticles including absorbed aqueous sample with said second detection composition, thereby allowing said library of microparticles to absorb said second detection composition;
- optionally, further washing said library of prefabricated microparticles to remove any non-absorbed or non-reacted second detection composition;
- transferring said library of prefabricated microparticles into a non-aqueous phase and removing any aqueous phase surrounding the individual prefabricated microparticle(s), thereby creating a plurality of insulated reaction spaces for detecting said analyte which reaction spaces comprise an aqueous phase and are confined to said void volume(s) of said microparticles; wherein, preferably, said step of transferring into a non-aqueous phase is performed immediately after said library of prefabricated microparticles have been incubated with said second detection composition;
- optionally, releasing the analyte-specific reagent(s) attached to said microparticles by applying an external trigger;

detecting and/or quantitating said analyte of interest by detecting and/or quantitating said detection reagent.

Such a method is particularly useful, when the analyte(s) of interest is(are) a protein(proteins) or other non-nucleic acid(s), and the detection reaction is an immunochemistry detection.

In yet a further aspect, the present invention also relates to a method of detecting and/or quantitating an analyte of interest in an aqueous sample, said method comprising the steps:

- providing, in any order:

- an aqueous sample known or suspected to contain an analyte of interest;
- a first detection composition comprising necessary reagents for performing a chemical or biochemical detection reaction of the analyte;
- a second detection composition comprising a detection reagent;
- a library of prefabricated microparticles according to any of the above defined embodiments of the present invention, wherein said analyte-specific reagent(s) attached to said microparticles, is(are) chosen such that it is (they are) capable of specifically binding to or reacting with said analyte of interest;

- optionally, mixing said aqueous sample and said first detection composition;
- incubating said aqueous sample with said library of prefabricated microparticles and, if said aqueous sample has not already been mixed with said first detection composition, also with said first detection composition, thereby allowing said library of microparticles to absorb aqueous sample and said first detection composition in the void volumes of said microparticles and, optionally, to bind said analyte of interest, if present in said sample;
- optionally, washing said library of prefabricated microparticles to remove any non-absorbed or non-reacted first detection composition;
- incubating said library of prefabricated microparticles including absorbed aqueous sample with said second detection composition, thereby allowing said library of microparticles to absorb said second detection composition;
- optionally, further washing said library of prefabricated microparticles to remove any non-absorbed or non-reacted second detection composition;
- transferring said library of prefabricated microparticles into a non-aqueous phase and removing any aqueous phase surrounding the individual prefabricated microparticle(s), thereby creating a plurality of insulated reaction spaces for detecting said analyte which reaction spaces comprise an aqueous phase and are confined to said void volume(s) of said microparticles;
- optionally, releasing the analyte-specific reagent(s) attached to said microparticles by applying an external trigger;
- performing a detection reaction of said analyte of interest; and
- detecting and/or quantitating said analyte of interest.

Such a method is particularly useful, when the analyte(s) of interest is(are) a protein(proteins) or other non-nucleic acid(s), and a detection of the analyte(s) occurs by an immunochemistry detection coupled with an amplification reaction. Typical examples of such a reaction are an immune-PCR-reaction.

The libraries of microparticles according to the present invention can be tailor-made according to the needs of a user and may be used in a wide variety of different methods:

Accordingly, in one embodiment,

- the method is a method of detecting and/or quantitating a single analyte in a plurality of aqueous samples, e.g. from different patients,
- in said method, there are provided a plurality of different aqueous samples known or suspected to contain an analyte of interest, e.g. samples from different patients,
- the library of prefabricated microparticles that is provided in said method, is a library according to embodiment 11, wherein, in such library, there are as many or at least as many separate subsets of microparticles provided, as there are different aqueous samples provided and to be tested, wherein all of said separate subsets have the same analyte-specific reagent attached to the porous matrix of said microparticles of said subsets, said analyte-specific reagent being specific for one analyte of interest;
- in said step of incubating said aqueous samples with said library of prefabricated microparticles, each of said different aqueous samples is incubated separately with a separate subset of microparticles of said library;
- in said step of incubating said library of prefabricated microparticles including absorbed aqueous sample with a second detection composition, each of said separate subsets of microparticles of said library is incubated separately with said second detection composition;
- said step of transferring said library into a non-aqueous phase is performed for each subset of microparticles separately, i.e. each separate subset of microparticles is separately transferred into a non-aqueous phase, and the aqueous phase surrounding the individual microparticles is removed for each subset separately, thereby creating, for each subset separately, a plurality of insulated reaction spaces for detecting said analyte which reaction spaces comprise an aqueous phase and are confined to said void volume(s) of said microparticles,

wherein said method further comprises, after said step of transferring said library into a non-aqueous phase, a step of mixing said plurality of insulated reaction spaces of all the separate subsets in said non-aqueous phase together, before subsequently a detection reaction of said analyte of interest is performed, and before detecting and/or quantitating said analyte of interest.

In another embodiment,

- the method is a method of detecting and/or quantitating multiple analytes in a single aqueous sample,
- in said method, there is provided a single aqueous sample known or suspected to contain multiple analytes of interest,
- the library of prefabricated microparticles that is provided in said method, is a library according to embodiment 12, wherein, in such library, there are as many or at least as many separate subsets of microparticles provided, as there are different analytes of interest to be detected, with each of said separate subsets having a different analyte-specific reagent attached to the porous matrix of said microparticles of said subset; each analyte-specific reagent being specific for one analyte of interest;
- in said step of incubating said aqueous sample with said library of prefabricated microparticles, said aqueous sample is incubated with all of said separate subsets of microparticles of said library together;
- in said step of incubating said library of prefabricated microparticles including absorbed aqueous sample with a second detection composition, all of said separate subsets of microparticles of said library are incubated together with said second detection composition;
- said step of transferring said library into a non-aqueous phase is performed for all subsets of microparticles together, i.e. all subsets of microparticles are transferred together into a non-aqueous phase, and the aqueous phase surrounding the individual microparticles is removed, thereby creating a plurality of insulated reaction spaces for detecting and/or quantitating said multiple analytes which reaction spaces comprise an aqueous phase and are confined to said void volume(s) of said microparticles.

In yet another embodiment,

- the method is a method of detecting and/or quantitating multiple analytes in a plurality of aqueous samples,
- in said method, there are provided a plurality of different aqueous samples known or suspected to contain multiple analytes of interest, e.g. samples from different patients,
- the library of prefabricated microparticles that is provided in said method, is a library according to embodiment 13, wherein, in such library, there are different classes of separate subsets of prefabricated microparticles, with each of said classes comprising several subsets of microparticles and each of said classes having a different analyte-specific reagent attached to the porous matrix of said microparticles, and all subsets of microparticles within one class having the same analyte-specific reagent attached; each analyte-specific reagent being specific for one analyte of interest; wherein, in said method, in said step of providing said library, there are

    • as many or at least as many different classes of separate subsets of microparticles provided, as there are different analytes to be detected,
    • as many or at least as many different subsets of microparticles provided within each class , as there are aqueous samples provided and to be tested,
    • as many or at least as many different subsets of microparticles provided within the library as there are different aqueous samples to be tested multiplied by the number of different analytes of interest to be detected,

- in said step of incubating said aqueous sample with said library of prefabricated microparticles, exactly one aqueous sample is incubated with exactly one subset of microparticles from each class, with each aqueous sample being incubated with as many different subsets of microparticles from different classes together as there are classes of microparticles in said library,
- in said step of incubating said library of prefabricated microparticles including absorbed aqueous sample with a second detection composition, the combined subsets from different classes of microparticles with which subsets one respective aqueous sample had been previously incubated, are incubated together with said second detection composition,
- said step of transferring said library into a non-aqueous phase is performed for each aqueous sample separately, namely the combined subsets of microparticles with which one respective aqueous sample had been previously incubated, are transferred together into a non-aqueous phase, and the aqueous phase surrounding the individual microparticles is removed, thereby creating, for each aqueous sample separately, a plurality of insulated reaction spaces for detecting said multiple analytes which reaction spaces comprise an aqueous phase and are confined to said void volume(s) of said microparticles,

wherein said method further preferably comprises, after said step of transferring said library into a non-aqueous phase, a step of mixing said pluralities of insulated reaction spaces of all the separate samples in said non-aqueous phase together, before subsequently a detection reaction of said analytes of interest is performed, and before detecting and/or quantitating said analytes of interest.

As regards the quantitation in the above mentioned methods, in some embodiments, in said step of detecting and quantitating said analyte of interest, quantitation of said analyte is performed by a method selected from:

> a) digital nucleic acid amplification, in particular digital polymerase chain reaction (PCR);
> b) real-time quantitative nucleic acid amplification, in particular real-time polymerase chain reaction (PCR);
> c) immunochemistry detection methods, in particular digital immunochemistry detection methods, such as digital immunoassays, e.g. digital enzyme-linked immunosorbent assays (ELISAs);
> d) immunochemistry detection methods combined with nucleic acid amplification, such as immuno-polymerase chain reaction; in particular digital immune-PCR;

wherein quantitation is performed using any of methods a) or b), or a combination of a) and b), if the analyte of interest is a nucleic acid; and wherein quantitation is performed using any of methods c) or d), if the analyte is a protein, peptide or other non-nucleic acid analyte

In particular with respect to the quantitation of analyte(s), the versatility of the present invention is also reflected by the fact that embodiments of libraries of microparticles according to the invention allow for parallel (multiplexed) ultra-sensitive detection of multiple analytes in a sample. In particular this works with libraries in accordance with any of embodiment 12 - 13. More specifically, this works in particular with a library according to embodiment 12 (which is a library for detecting and/or quantitating multiple analytes in a sample). Whereas each microparticle represents a discrete signal amplification space or a target amplification space allowing single molecule detection similar to established digital detection techniques, the microparticles can be used to detect and even quantify multiple different analytes (i.e. multiple analyte species) (>1) simultaneously in a sample, whereby the achievable theoretical limit of detection for each different analyte can be determined by the following formulae:

$$N = \log_{\left(\frac{B-1}{B}\right)}(1 - P) \quad \text{OR} \quad P(B,N) = 1 - \left(1 - \frac{1}{B}\right)^{N}$$

With

B=Number of different types of microparticles, each type (or "subset") being specific for the detection of one individual analyte

N=Number of Analyte molecules of one specific analyte species in a sample

P= Probability, that specific analyte species is actually present in the respective microparticle type specific for such analyte, if B (=number of) different types of microparticles are used and if the number of different analyte species present in a sample is ≤ B.

The formulae result from the binomial distribution of analyte molecules across all microparticles interrogated with the sample. The limit of detection is represented by N, whereby P is set at 95%. For the sake of clarity the formulae do not take into consideration the distribution of analyte in the sample of the body fluid to be analyzed for the presence and quantity of analyte.

In addition, embodiments of libraries of microparticles according to the present invention enable an elegant approach for analyte quantification by bridging quantitative digital and quantitative real time analysis approaches. While this statement is true for any kind of signal or target amplification assay being employed on the microparticles, PCR amplification may serve as an example to illustrate the approach to quantitation of targets with embodiments of the method according to the present invention. Quantitation of target molecules is preferably conducted by digital PCR (dPCR) because this method allows more sensitive and precise quantitation than real-time quantitative PCR (qPCR). A disadvantage of digital PCR is the inherent limitatbn of the measuring range that depends on the number of microparticles specific for one sample/analyte. To overcome this limitation, the invention supplements the analysis of digital PCR with real-time quantitative PCR for target concentrations above the digital PCR measuring range. The implementation of this approach requires acquisition of fluorescence images at the end of PCR and during all cycles of the real-time PCR. All acquired images are analysed by an image analysis algorithm in order to quantify fluorescence level of each microparticle in the reactor chamber. A segmentation algorithm separates bright disk-shaped microparticle objects from dark background. Based on this segmentation information, finally circles are fitted to microparticle object contours allowing estimation of mean microparticle fluorescence and microparticle volume. The analysis of real-time PCR images includes tracking of microparticle positions in consecutive images to allow monitoring fluorescence during the course of the reaction in each respective microparticle.

The selection of the applied quantitation approach is based on the number/proportion of microparticles remaining negative after amplification reaction, i.e. showing no amplification. This information is taken from digital PCR data. If a predefined lower limit for number/proportion of negative microparticles is exceeded (i.e. if the number/proportion of negative microparticles is higher than such predefined lower limit), an end point Poisson analysis can be applied. Otherwise, i.e. if the number/proportion of negative microparticles is lower than such predefined lower limit, real-time analysis is conducted using real-time quantitative PCR data acquired during all cycles. A reasonable lower limit for the proportion of negative microparticles allowing still robust quantification with Poisson is 0.5%. Corresponding mean number of targets per microparticle is 5.3. To consider possible artefacts on fluorescence images an additional requirement can be a lower limit for the total number of negative microparticles of e.g. 50.

The end point Poisson analysis is performed by determining the proportion of negative microparticles and applying a Poisson correction to account for the fact that positive microparticles can contain more than one target molecule. The threshold, distinguishing positive and negative microparticles, is directly estimated from fluorescence signal intensities of microparticles known to be negative. Possible microparticle volume variations are factored into the quantitation by performing microparticle volume specific Poisson corrections. Possible variations of the total microparticle volume between measurements are also corrected by the algorithm.

If target concentration exceeds the measuring range of digital PCR, real-time analysis fits a nonlinear function to the fluorescence signal course of each single microparticle. The fitted nonlinear model combines a sigmoid and a linear function where the sigmoid component reveals amplification kinetics and the linear component represents baseline of the signal. The cycle threshold value (Ct) is calculated from the intersection of tangent in definition value of sigmoid function with maximum second derivation and baseline. Respective target numbers per microparticle are calculated using a calibration data set.

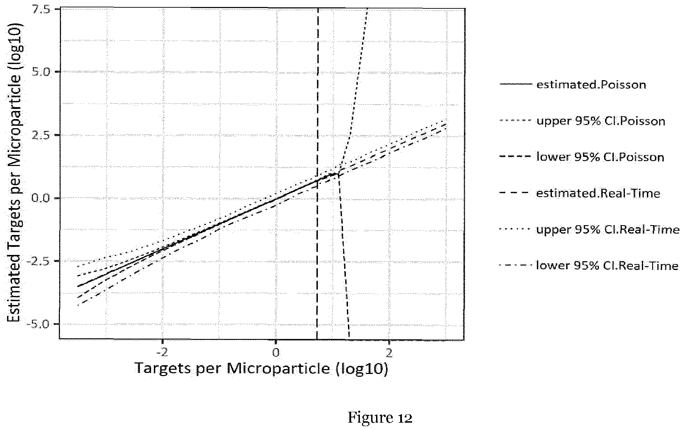

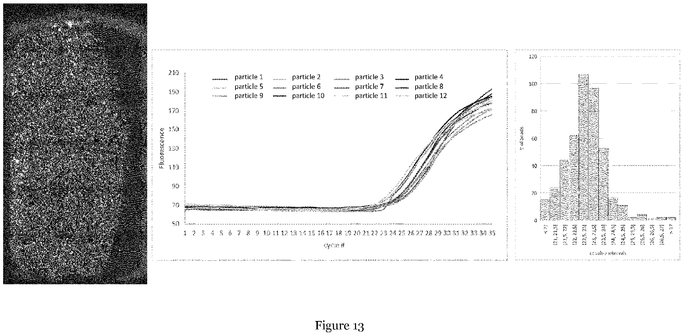

This principle of quantification is also illustrated in Figures 12 and 13 as well as in Example 3. Furthermore, reference is made to the figures wherein

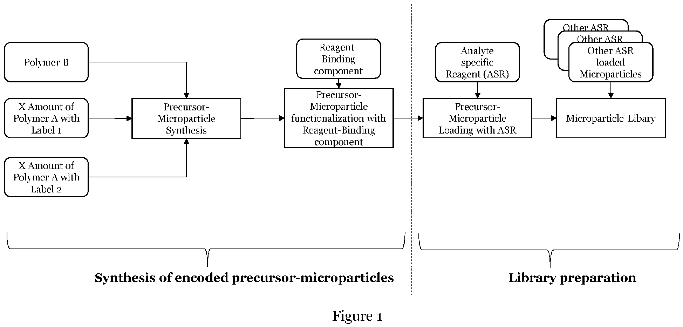

**Figure 1** shows an embodiment of a process for generating a library of prefabricated precursor-microparticles (left part of the figure) and for subsequently generating a library of prefabricated microparticles (right part of the figure). In the left part of the figure, prefabricated precursor-microparticles are produced each of which has a respective label component attached to, contained within or otherwise associated with said precursor-microparticle; and this process on the left part of the figure is repeated for different prefabricated precursor-microparticles using different label components, thus producing different subsets of prefabricated precursor-microparticles. The result thereof is a library of prefabricated precursor-microparticles wherein, in said library, there are at least two separate subsets of prefabricated microparticles, or possibly, also three or more separate subsets of prefabricated microparticles, with each subset having its distinct label component attached to, contained within or otherwise associated with said precursor microparticles within said subset such that said at least two or more separate subsets of prefabricated precursor-microparticles differ by the respective label component attached thereto or contained within each subset. On the right part of the figure, each of the subsets of prefabricated precursor-microparticles is loaded with an analyte-specific reagent (ASR). If the library of microparticles is to be used to detect different analytes, the respective analyte-specific reagent that is attached to each subset of microparticles is different. If the library is to be used for the detection of the same analyte, but with a plurality of samples, the respective analyte-specific reagent that is attached to the different subsets of microparticles is the same. The resultant library of prefabricated microparticles is very versatile and can thus be prepared and used, according to different needs. It may be used for the detection of multiple analytes in a single sample, or it may be used for the detection of a single analyte in a plurality of samples. In the embodiment shown in figure 1, binding of the analyte-specific reagent (ASR) occurs through a reagent binding component, which may be one of the possibilities (i)-(v) listed further above. As an example, in its simplest form, the reagent binding component may be the polymer or the polymer mixture that forms the porous polymeric matrix or is the polymeric matrix of the microparticles. In another embodiment, it may be a specific reagent binding molecule that is attached to the porous polymeric matrix. In yet another embodiment, it may be an ionisable group or a plurality of ionisable groups, or it may be a charged group or a plurality of charged groups that are immobilized on the porous polymeric matrix, or it may be the combination of any of the foregoing. In a preferred embodiment, the reagent-binding component is a reagent binding molecule attached to the porous polymeric matrix which, in turn interacts with a binding entity to which the analyte-specific reagent is conjugated. Examples of this embodiment are shown further below, wherein, as an example, the reagent binding molecule that is attached to the porous polymeric matrix, is a streptavidin-molecule or a streptavidin-related molecule. Its counterpart on the analyte-specific reagent, i.e. the binding entity which binds to the reagent binding molecule is desthiobiotin or a similar molecule, allowing for a reversible attachment to streptavidin or avidin. Reversibility is achieved in that the bond(s) between the binding entity (on the analyte-specific reagent (ASR) and the reagent binding molecule (on the porous polymer matrix) may be released through application of an external trigger, e.g. a change in temperature to which the microparticles are exposed. It is clear to a person skilled in the art, that many different variations of such an embodiment are possible and envisageable.

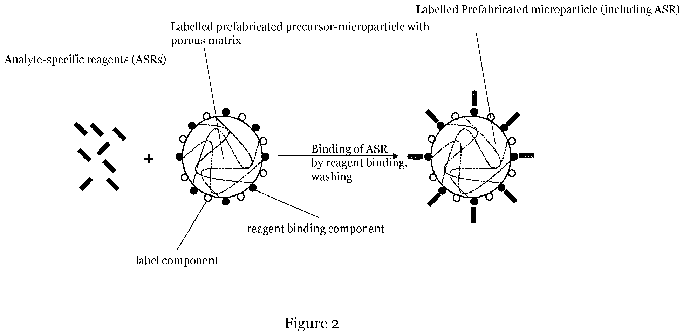

**Figure 2** shows an embodiment of a scheme outlining the relationship between a precursor-microparticle and a prefabricated microparticle resulting therefrom upon binding of an analyte-specific reagent. A prefabricated precursor-microparticle having a porous polymeric matrix is provided. The porous polymeric matrix has a void volume for receiving an aqueous sample and for providing a reaction space for the specific detection of an analyte. The prefabricated precursor-microparticle further comprises a reagent binding component that allows the attachment, preferably the reversible attachment, of an analyte-specific reagent to the precursor-microparticle. Furthermore, the precursor-microparticle has a label component attached which label component allows to identify the precursor-microparticle (and subsequently, the resultant prefabricated precursor-microparticle as well as the analyte-specific reagent that becomes attached to the precursor-microparticle). Also shown are analyte-specific reagents (ASR) which become attached to the prefabricated precursor-microparticle. It should be noted that, as an example, such analyte-specific reagent may be a pair of nucleic acid primers and, optionally, a probe, which are specific for a particular (nucleic acid) analyte and allow the amplification and detection of such analyte, if present in a sample to which the respective microparticle is subsequently exposed. Although in the figure, all ASRs are shown alike, it is envisaged that such pair of primers and, optionally, probe qualifies as one analyte-specific reagent. Other examples for an analyte-specific reagent may be a (primary) antibody that is specific for a particular analyte.

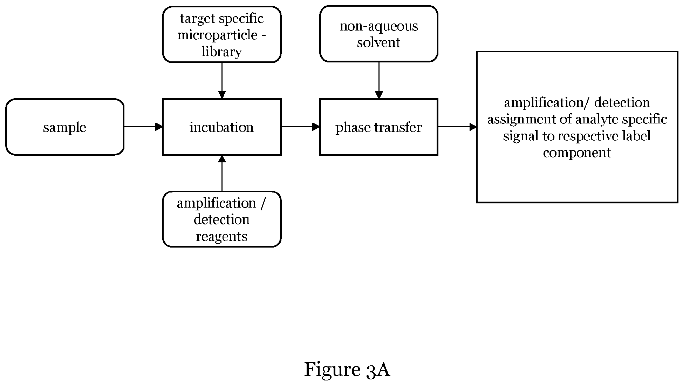

**Figure 3A** shows an embodiment of an exemplary scheme for a method of detecting an analyte of interest in an aqueous sample, wherein a library of microparticles, in accordance with embodiments of the present invention is provided and is exposed to such aqueous sample. The library is exposed to (or "incubated with") the sample and to a detection composition comprising amplification/detection reagents. As a result of such exposure, the library of microparticles is allowed to absorb the aqueous sample and the detection composition in the void volumes of the microparticles and, optionally, to bind or enrich the analyte(s) of interest, if present in the sample. Depending on the type and number of different analyte-specific reagents attached to the microparticles within the library, one or several different analytes may be detected. Once the library has been exposed to the sample and the detection composition, the library is transferred into a non-aqueous phase, and aqueous phase surrounding the individual prefabricated microparticles is removed, for example by exerting mechanical force(s) on the microparticles. Each microparticle however, still has an aqueous phase inside in its respective void volume. As a result of such transfer into a non-aqueous phase and of removal of any surrounding aqueous phase, a plurality of insulated reaction spaces is created which reaction spaces act as "reactors" allowing to detect the analyte(s), and the reaction spaces comprise an aqueous phase and are confined to the void volume(s) of the respective microparticles. Depending on the precise nature of the respective microparticles, it may be preferable to have such microparticles undergo a phase transition, such as a gel-sol transition (i.e. from a solid or quasi-solid **gel** state to a liquid **sol**uble state) which effectively will trigger the release of the respective analyte-specific reagent(s) attached to the microparticles. This may occur by applying an external trigger, such as a temperature change, pH change, the addition of specific chemical agents etc. In a preferred embodiment, such external trigger is a rise in temperature. A gel-sol transition will also typically result in a transformation of a suspension of microparticles (solid in liquid) to a proper emulsion of microdroplets (formlerly solid microparticles) in a liquid phase (liquid in liquid). The respective microparticles, once transferred to the non-aqueous phase, and optionally, having the analyte-specific reagent(s) released, undergo a detection reaction of the analyte(s) of interest. Because each of the microparticles is insulated within a non- aqueous phase, there is no crosstalk between different microparticles/reactions spaces provided by such microparticles. Depending on the type of analyte of interest and the analyte-specific reagent(s), such detection reaction may be an amplification reaction (in case that the analyte(s) of interest is (are) nucleic acid(s)) or it may be an immunochemistry reaction (for example if the analyte(s) of interest is (are) protein). Subsequently, the analyte of interest may be detected in the individual microparticle. Alternatively, the detection reaction may also be an immuno-amplification reaction where, in a first step, a primary antibody is used to bind an analyte of interest, and a sandwich is formed using a secondary antibody which, however, is attached to an oligonucleotide tag, which in a second step may be amplified using suitable primers. Because each microparticle has a specific label component, it is possible to assign the presence of a particular analyte and the signal associated (or generated) therewith to the label component of the respective microparticle in which the signal has been detected.

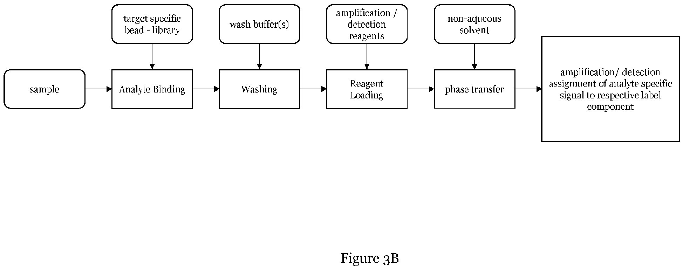

**Figure 3B** shows an embodiment of an exemplary scheme for a method of detecting and/or quantitating an analyte of interest in an aqueous sample, wherein a library of microparticles, in accordance with embodiments of the present invention is provided and is exposed to such aqueous sample under conditions favoring the binding of the analytes to the microparticles. The microparticles with the bound analytes may be washed with a suitable buffer in order to remove any unwanted materials. Subsequently the library is exposed to a detection composition comprising amplification/detection reagents. After having been exposed to a detection composition comprising the necessary amplification/detection reagents, the library is transferred into a non-aqueous phase, thus effectively generating an insulated reaction space within each microparticle, and therefore, effectively, generating a plurality of insulated reaction spaces. Each microparticle contains an aqueous phase including sample and the necessary amplification/detection reagents and is isolated from other microparticles by the surrounding non-aqueous phase. Subsequently, an amplification/detection reaction is performed, and the analyte (analytes) of interest may be detected on a per-microparticle-basis. Because each microparticle has its own label component, the respective analyte-specific signal that is generated if the analyte is present in the respective microparticle, such signal and the corresponding presence of the analyte may be assigned to the respective label component of the individual microparticle and thus to the individual microparticle.

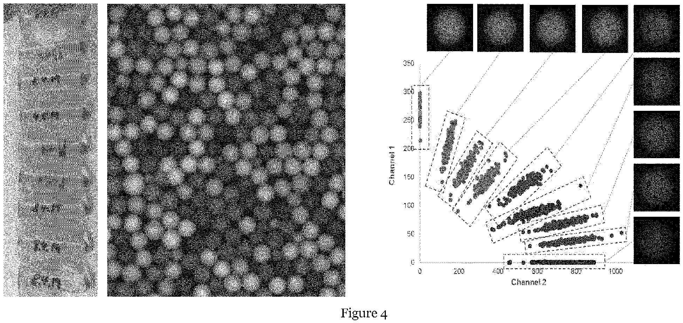

**Figure 4** shows an embodiment of a synthesis of encoded precursor-microparticles, as described in detail in example 1. Using differently labeled gelatin and agarose, agarose/gelatin microparticles with different label components are generated. Shown on the left are the respective differently labelled types of gelatin. In the middle photograph, there is shown an image of multiple thus generated differently labelled microparticles, and on the right is shown a scatter plot of fluorescence signals obtained for individual microparticles in two separate fluorescence channels and false color images of the different microprecursor-microparticles as they would be detected by means of their respective fluorescence. 9 different types of precursor-microparticles can be clearly distinguished as separate particle populations in accordance with their fluorescence.

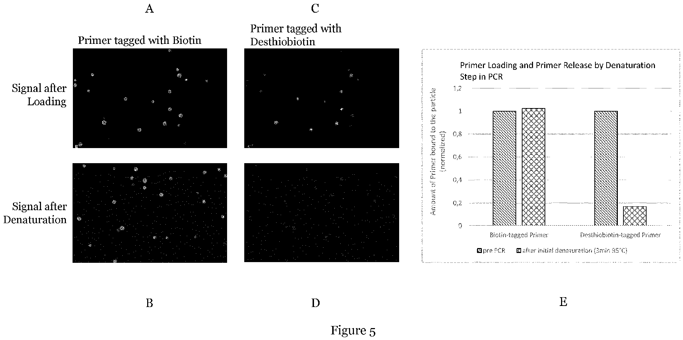

**Figure 5** shows that by selecting an appropriate binding entity for primer oligonucleotides (which oligonucleotides act as analyte-specific reagents) these can be reversibly attached to the Streptavidin-coated microparticles prepared in accordance with embodiments of the present invention. In this example, the reagent binding molecule on the microparticles is streptavidin, and the binding entity on the analyte-specific reagent is biotin or desthiobiotin. Desthiobiotin allows for a reversible attachment, whereas biotin does not. In order to demonstrate the reversible nature of the attachment in the case of desthiobiotin, crosslinked microparticles have been incubated ("loading" stage) with biotin tagged fluorescence labelled oligonucleotides (panel A) or with desthiobiotin tagged fluorescence labelled oligonucleotides (panel C). The incubation ("loading") was carried out at room temperature. The microparticles have been washed and a fluorescence image taken. In both images (left and center upper images, panels A and C) microparticles are clearly visible. Thereafter the temperature has been increased to 95°C ("denaturation" stage) and the microparticles are washed again. Whereas the biotin labelled oligonucleotide remains bound to the microparticle (as can be seen by the remaining fluorescence in panel B), the desthiobiotin labelled oligonucleotide (and the fluorescence associated with it) clearly has been removed by the treatment (no fluorescence in panel D). The bar graph on the right (panel E) is a quantitative representation of the signal obtained from the microparticles before and after heating.

Thus it has been shown that by using such reversibly binding entities (e.g. desthiobiotin) the primer oligonucleotide can be released from the microparticle matrix. This is a preferable feature for highly efficient amplification reactions in the droplet space created by the microparticles in a non-aqueous environment. Whilst some degree of amplification may be possible even with attached primers, for optimum amplification reaction conditions, primer oligonucleotides should ideally not be bound to any matrix and, thus, will be deliberately released prior to any amplification reaction.

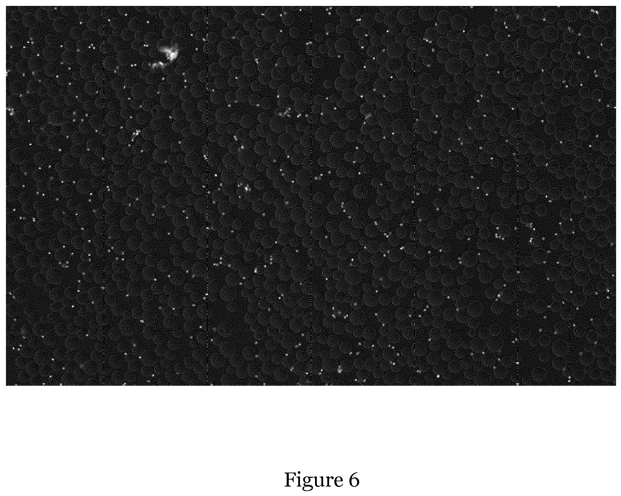

**Figure 6** shows a microscope-image with microparticles prepared in accordance with embodiments of the present invention. The microparticles have been coated with anti-CD45 antibodies and incubated with whole blood that was stained with the fluorescent dye Acridine Orange. After carefully washing the sample with PBS buffer, the microparticles have been imaged. Microparticles with variable diameters between 35-50 $\mu$m prepared in accordance with embodiments of the present invention can be distinguished from the background with some of the microparticles carrying a single cell attached to the microparticle. Because CD45 is a surface antigen characteristic for leucocytes, the cells bound are likely to be leukocytes. This embodiment clearly shows that such microparticles can be also used to selectively bind cell populations and to single out cells on individual particles that subsequently can be processed according to the invention according to the processes outlined in figure 3A and figure 3B.

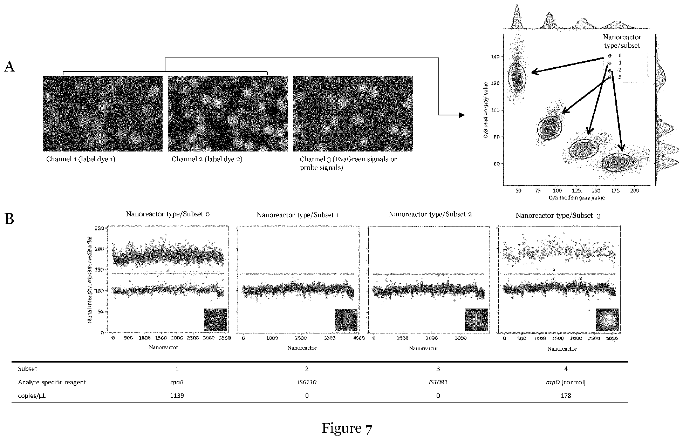

**Figure 7** shows an embodiment of a method in which several analytes are detected within a single sample ("analyte multiplexing") using a library of four differently labelled microparticles ("nanoreactors") in accordance with the present invention. This figure 7 exemplifies the experiment performed in example 3. Four different types of microparticles (in the figure and elsewhere herein also sometimes synonymously referred to as "nanoreactor") (made specific for the four different molecular targets rpoB, IS6110, IS1081 and atpD by attachment of target specific primers and probes (as analyte-specific reagents) to the respective microparticle), are used which can be distinguished in accordance with their respective label components. In figure 7A three greyscale images are shown representing three fluorescence channels that have been used for microimaging the color labeled agarose-gelatin-hybrid microparticles.

Four different labels can be assigned according to the fluorescence signals obtained in channel 1 and channel 2 for the detected microparticles as exemplified by the scatter plot. A further channel, channel 3 is used to monitor nucleic acid amplification signals (e. g. PCR-signals). Each label component corresponds to a specific target (analyte), when present. The 1D plots in Figure 7B for each type (or "subset") of microparticle show positive and negative signals which are translated into a specification of detected copy number per volume of sample. Figure 7B shows the signal intensity for the different nanoreactor (or microparticle) types (i. e. also for the different analytes); after amplification it can be seen that in the sample which has been tested here, microparticle type ("subset") 0 and microparticle type ("subset") 3, which are specific for rpoB and atpD, respectively, show a positive signal, indicating the presence of such analyte in the original sample. Also the graphs indicate the presence of positive and negative microparticles. By counting the positive and negative microparticles and applying Poisson analysis, the number of targets in the sample can be determined with great precision. Thus, this also enables a quantification with great precision. In contrast thereto, microparticle type ("subset") 1 and microparticle type ("subset") 2, specific for analytes IS6110 and IS1081, respectively, provide no positive signal, indicating the absence of such analyte from the original sample.

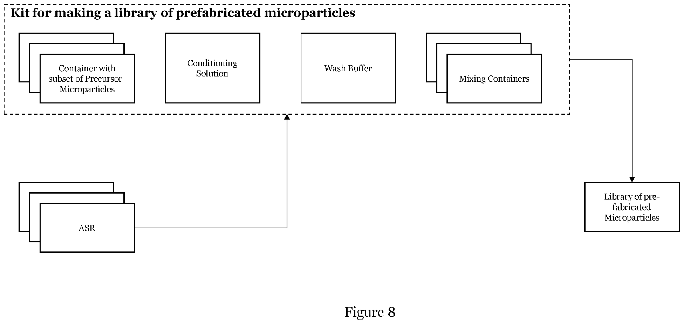

**Figure 8** shows an embodiment of a kit for making a library of prefabricated microparticles ("manufacturing kit"). Such embodiment of such kit comprises at least two containers or more, each containing a subset of prefabricated precursor-microparticles, as defined further above. Each subset of prefabricated precursor-microparticles has its distinct label component attached to, contained within or otherwise associated with said precursor-microparticles, such that the different subsets of prefabricated precursor-microparticles differ by the respective label component attached to, contained within or otherwise associated therewith. The kit also comprises a further container containing a conditioning solution (e.g. a buffer, such as PBS buffer, or water) which conditioning solution enables the attachment, preferably the reversible attachment of an analyte-specific reagent to the subset(s) of said precursor-microparticles. In a preferred embodiment, such kit further comprises a further container which contains a washing buffer for removing free, i. e. non-attached, analyte-specific reagent(s) from microparticles, without, however, removing analyte-specific reagent(s) that is(are) attached to microparticles. Furthermore, in a preferred embodiment, such kit also comprises one or more mixing container(s) for mixing components together. Also shown are different analyte specific reagents which are selected and provided by a user of such kit, enabling such user to generate a library of micro prefabricated microparticles according to the user's need. Attachment of the respective analyte-specific reagent(s) is facilitated by using the conditioning solution contained within the kit.

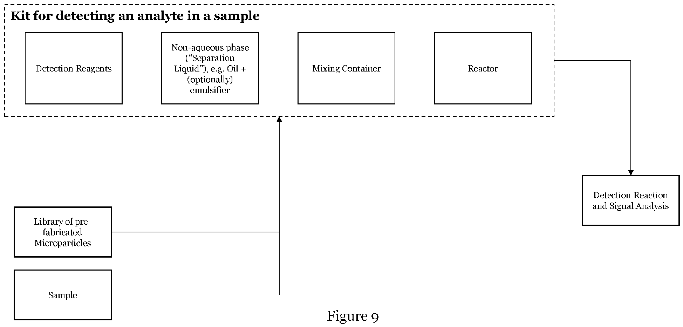

**Figure 9** shows an embodiment of a kit for detecting an analyte in a sample in accordance with embodiments of the present invention ("detection kit"). In this exemplary figure, the kit comprises a container containing a generic detection composition (which is a composition comprising reagents necessary for performing a detection reaction, without, however, any analyte-specific reagent(s) (ASRs)), a container containing a non-aqueous phase, such as an oil, optionally with a suitable emulsifier. Furthermore, such kit may optionally contain one or several mixing containers. Furthermore, also optionally, such kit may comprise a further container which functions as a reactor. In this embodiment, the library of prefabricated microparticles may be provided separately, as well as the sample, and such library is exposed to the sample and to the detection reagents in the mixing container of the kit. Thereafter, a phase-transfer is performed by using the "reactor"-container in which then also the subsequent detection reaction is performed.

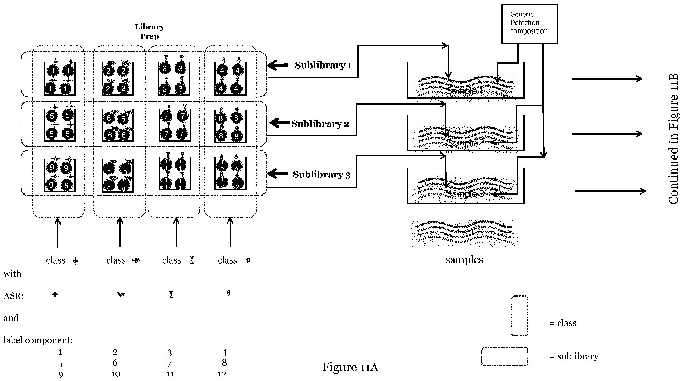

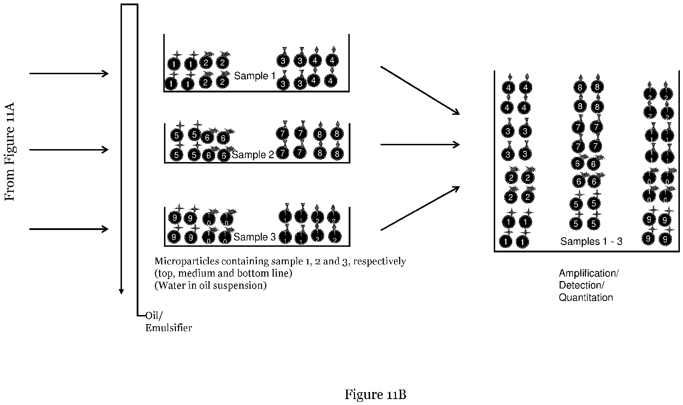

**Figures 10A-D** show different example libraries in accordance with embodiments of the present invention. **Figure 10A** shows an example library for detection of a single analyte in several samples. The library shown in figure 10A is the simplest library for detecting a single analyte in two different samples. As can be seen, in such library, there are two subsets of microparticles which have the same analyte-specific reagent attached but which differ from each other by the respective label component ("1" and "2"). Each subset within such a library is used for the detection of the same analyte, but in different samples. **Figure 10B** shows a simple example library for the detection of several analytes in a single sample. In this case, the analytes that may be detected are two different analytes, because in the library there are two different subsets, each of which subsets has a different analyte specific reagent attached. Moreover, the subsets differ by their respective label component ("1" and "2"). The two different subsets may initially be stored in containers but may ultimately be brought into single container when they are exposed to the sample. **Figure 10C** is another example library for the detection of several analytes in a single sample. In this exemplary library, there are provided four different subsets of microparticles, each of which subsets has its own distinct analyte-specific reagent attached. Moreover, the respective subsets differ from each other by their label component ("1", "2", "3" and "4"). Initially, the different subsets may be stored in separate containers but may ultimately be brought together, when they are being exposed to the sample, in which sample there are suspected to be four different analytes present. **Figure 10D** is an example library for the detection of several analytes in several samples. As can be seen, there are eight different subsets of microparticles. The respective subsets of microparticles each have their distinct label component ("1" to "8"), but there are four pairs of subsets of microparticles each pair of which has a different analyte specific reagent attached, and the two subset within each pair have the same analyte-specific reagent attached. Sometimes in this application, the subsets of microparticles which have the same analyte-specific reagent attached, but which nevertheless differ by their respective label component, are herein also sometimes referred to as being part of one "class". The example library that is shown in figure 10D, may be used to detect the presence of four analytes in two samples. Accordingly, each sample is probed or interrogated with or exposed to four subsets of microparticles, each subset of which has a different analyte-specific reagent attached. Such subsets of different microparticles that are being used for interrogating the same single sample (out of a plurality of several samples) are herein also sometimes referred to as a "sublibrary" of subsets of microparticles. The concepts of "class" and "sub-library" are further outlined and explained in figure 11.

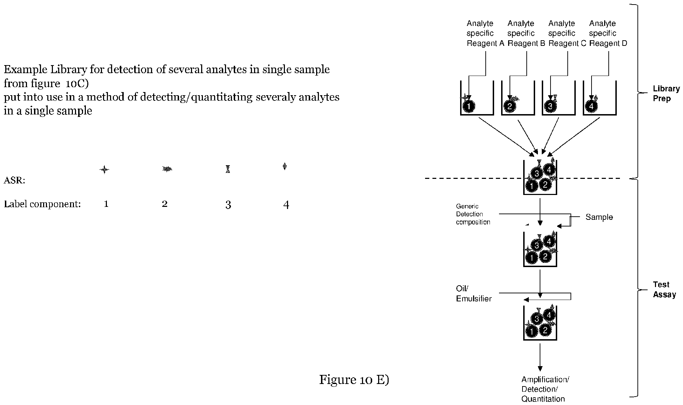

**Figure 10E** shows an exemplary schematic diagram of a method for detecting several analytes within a single sample, in which the generation of a library is shown which allows the detection of four different analytes, using four different analyte-specific reagents (ASRs) in such library. The respective microparticles are differently labeled by using different label components ("1" to "4") and can thus be distinguished. The resulting library is the exemplary library of figure 10C. The single sample is exposed to the library as well as to the necessary detection composition, where upon the microparticles are transferred into a non-aqueous phase. Thereafter, the detection reaction is performed and the resultant signal(s) is(are) detected and analyzed.

**Figure 11A** and **Figure 11B** show an exemplary library for the detection of several analytes in several samples. In this particular case shown here, there are three different samples that are being tested for the presence of four different analytes. As can be seen in figure 11A there are four different subsets of microparticles per sample to be tested. At the same time, however, there are also three subsets of microparticles which have the same analyte-specific reagent attached, forming thus a triple (or N-tupel) of subsets of microparticles having the same analyte-specific reagent attached. In such N-tupel (having the same analyte-specific reagent attached), there are as many different subsets, as there are samples to be tested (sometimes "at least as many" different subsets, if not all subsets of the N-tupel are finally used in the experiment). Effectively, therefore N designates the number of samples to be tested. Such N-tupel is herein also sometimes referred to as "class" which refers to the entirety of all subsets of microparticles within a library that have the same analyte-specific reagent attached. All of the subsets within one class are specific for one analyte of interest. In contrast thereto, the term "sub-library" is meant to refer to the entirety of all subsets of microparticles within a library that are used to interrogate one particular sample at a time. Within each sub-library, each subset of prefabricated microparticles has a different analyte-specific reagent attached; each analyte-specific reagent being specific for one analyte of interest. In figure 11A, sub-libraries 1, 2 and 3 contain four subsets of microparticles each, and these four subsets of microparticles within each sub-library may initially be stored in separate containers. When the respective sub-library, however, is brought into contact with the respective sample, corresponding subsets of microparticles within each sublibrary may be combined. Thereafter, the microparticles that have been exposed to their respective sample may optionally be washed, and will then be exposed to a suitable detection composition comprising the necessary reagents for performing an amplification/detection reaction. Thereafter, a phase-transfer is performed, and the respective microparticles are brought into a non-aqueous phase, thus effectively generating a plurality of insulated reaction spaces for each sample, as a suspension. Once the respective microparticles have been transferred into a non-aqueous phase, they may be pooled in a single container, and a suitable amplification/detection reaction may be performed.

**Figure 12** illustrates the combination of digital and real-time quantitative PCR for target quantitation in microparticles. The quantitation approach presented here takes advantage of the digital PCR if target concentration does not exceed an upper limit of measuring range. Digital PCR is considerably more precise and more resistant to PCR inhibitors. Confidence intervals ("CI") of digital PCR caused by statistical effects are shown. They are determined by Poisson distribution of sample collection at the lower end of the measuring range and binomial distribution of targets over microparticles at the upper end of measuring range. Poisson distribution of sample collection also contributes to imprecision of real-time PCR. However, real-time PCR is additionally subjected to the variable nature of the PCR process to its full extent. Quantitative fluorescence readout during amplification reaction is much more likely to be influenced by variations in reaction efficiency than binary fluorescence readout upon completion of PCR. Typical reproducibility for commercial test assays based on real-time quantitative PCR ranges from approximately 0.30 log cp/mL (copies per ml) at very low target concentrations to 0.10 log cp/mL at high target concentrations. An advantage of the proposed quantitation approach is that real-time quantitative PCR is, preferably, only applied for higher target concentrations, where the method enables more precise results than for lower concentrations. Overall, the approach allows utilization of the large dynamic range of quantitative PCR (qPCR) and the exquisite sensitivity and quantitation precision of digital PCR at the lower end of the measurement range. In one embodiment, once a method of detection in accordance with the present invention has been performed, a quantitation can be achieved using the following exemplary guideline: For the number of negative (dark) microparticles of >0.5% of all microparticles available for the test assay, Poisson analysis (digital) is to be applied. For any value below that, real-time-analysis is to be applied. This corresponds to an approximate average concentration (Lambda) of 5.3 Targets per microparticle. Possible artefacts on fluorescence images can be considered by introducing a minimal requirement for the total number of negative microparticles of e.g. 50 per analysis.

More generally speaking, once a method of detection in accordance with the present invention has been performed, a quantitation can be achieved using the following exemplary guideline: If the number of negative microparticles (i.e. microparticles in which no signal can be detected), exceeds a percentage in the range of from 0.1 - 1.0%, preferably 0.5 - 1.0%, more preferably 0.5 - 0.8%, then Poisson analysis is to be applied. If the number of negative microparticles (i.e. microparticles in which no signal can be detected) is below a percentage in the range of from 0.1 - 1.0%, preferably below a percentage in the range of from 0.5 - 1.0%, more preferably below a percentage in the range of from 0.5 - 0.8%, then quantitative real time analysis is to be applied, e.g. involving determination of cycle threshold values (e.g. using a comparative $C_T$ method also referred to as 2-$\Delta$ $\Delta CT$ method (see for example Schmittgen et al., 2008, Nature Protocols, 3, pp. 1101-1108)
Further details are explained in Example 3.

**Figure 13** shows real time fluorescence data obtained from an image series collected on microparticles in oil during PCR amplification. An image of a detection chamber with the endpoint fluorescence signal in one fluorescence channel specific for amplification is shown on the left. The graph in the center shows fluorescence intensity for 12 representative individual microparticles selected from the fluorescence image on the left. A distribution of the calculated ct-values for all microparticles detected in the fluorescence image is shown in the histogram on the right.

Furthermore, reference is made to the following examples, which are given to illustrate, not to limit the present invention.

## Examples

## Example 1

### Synthesis of encoded precursor microparticles

_Labeling of gelatin with fluorescent dues for identification of analyte-specific reagents_

The acetone insoluble fraction of gelatin from bovine skin type A or porcine skin type B is labelled with a distinct mixture of two fluorescent dyes taking advantage of NHS coupling chemistry. Cy®3 Mono NHS Ester and Cy®5 Mono NHS Ester (GE Healthcare) were dissolved in DMSO to make a final solution of 1% (w/v) in potassium phosphate buffer (pH 8.0, steril filtered). An 8-fold molar excess of the respective dye over free gelatin amino groups is utilized to label 25mL of 0.25% (w/v) of either gelatine type. The label solutions are incubated at 4°C overnight using the Multi-Rotator PTR-60 (Grant-bio) in the vertical mode. Purification of the fluorescently labelled gelatin is accomplished by repeated ammonium sulfate precipitation using a saturated $(NH_4)_2SO_4$ solution. Alternatively, ultracentrifugation, solvent extraction with isopropanol, acetone or methanol, gel filtration using sepharose columns or dialysis can be performed. In either case, purification is repeated until effluent appears clear and shows no fluorescence. Purified fluorescently labelled gelatin samples are finally vacuum-dried.

_Preparation of gelatin/agarose hybrid solution_

A hybrid hydrogel solution consisting of four components is prepared for fabricating nano-reactors.

_Component 1_: Acetone-insoluble gelatin from bovine skin type A G1890 (Sigma) or porcine skin type B G9391 (Sigma)

_Component 2_: Low-gelling 2-Hydroxyethyl agarose (A4018, Sigma)

_Component 3_: Cy3-labeled gelatin (type A or type B)

Component 4: Cy5-labeled gelatin (type A or type B)

To generate a homogeneous 4% (w/v) solution of component 1, 40mg of component 1 is dissolved in 1mL nuclease-free water (Carl Roth) and incubated at 50°C under gentle agitation (750rpm). Likewise, 20mg of component 2 is dissolved and melted in 1mL nuclease-free water and incubated at 80°C under gentle agitation to prepare a homogeneous 2% (w/v) agarose solution. To prepare a 4% (w/v) solutions of each labelled gelatin, the dried pellets of component 3 and 4 are taken up in a respective volume of nuclease-free water and incubated at 55°C until the gelatin is molten. All four components are mixed and filled up with nuclease-free water to generate a hybrid hydrogel solution with final concentrations of 1% (w/v) total for gelatin and 0.5% (w/v) for agarose A4018, respectively. Various volumes of component 3 and component 4 are mixed yielding n distinctly coloured microsphere sets. In this embodiment resuspended component 3 and component 4 are mixed in ratios 1:0, 3:1, 1:3, 0:1 at a maximum of 3% (v/v) of the total gelatine fraction to accomplish four individual label components (label components 1 - 4, respectively) for identifying analyte specific reagents and allowing a 4-plex reaction assay. All solutions are kept at 55°C until further use.

*Generation ofnon-crosslinked gelatin/agarose microparticles*

Monodisperse color coded agarose-gelatin hybrid microparticles are fabricated using either parts of the QX100/QX200 Droplet Digital (ddPCR™) system (BioRad) or a modified μEncapsulator system (Dolomite microfluidics). In the case of the BioRad system, a DG8 Cartridge is kept on a Thermomixer to keep solutions at 55°C. After loading 50μL of the gelatin/agarose hybrid solution, 100μL of the emulsion reagent HFE-7500 containing 2-5% Picosurf 2 (Sphere Fluidics) is applied into the bottom wells of the cartridge. Vacuum is applied in the collection well by pulling gently on a syringe connected to the well. Alternatively, the QX200™/QX100™ Droplet Generator can be used for droplet generation. Approximately 80,000 hydrogel droplets of 100μm in diameter are produced per well.

In the case of the Dolomite microfluidics system, monodisperse hybrid microparticles can be fabricated in a one-step process of suspension formation using a simple flow-focus device. In detail, a standard droplet junction chip (100μm) of fluorophilic nature is used with a 4-way linear connector and a chip interface H to interface the fluidic connection between tubing and chip. Two Mitos P-Pumps deliver the hydrogel solution and the carrier oil. The system is modified by the integration of a heating rig which is placed on top of a hot plate and allows for maintaining the gelatin/agarose hybrid solution in liquid state and heating up the driving fluid ensuring consistent temperature when oil and gelatin/agarose hybrid solution get in contact at the chip junction. HFE-7500/Picosurf 2 and the hybrid hydrogel solution are both pre- filtered with a 0.22μm filter before placing them into the P-Pump (Mitos) and the hydrogel reservoir within the heating rig of the droplet system, respectively. Temperature of the heating rig is set to 55°C. The fluid lines are primed at 2000mbar for 1 min. A flow rate of 15-17μl/min is adjusted for stable droplet formation. Parameters are monitored with the Dolomite Flow Control Advanced Software.

In both cases, the color coded agarose-gelatin hybrid microparticles are collected on ice in either 2mL microcentrifuge tubes or 15mL falcon tubes to initiate solidification of the hybrid hydrogel. To prevent loss of aqueous phase of the microparticles at the oil-air boundary, 500μL of the emulsion oil containing the microparticles is overlaid with 500μL of nuclease-free water. Subsequently, microparticles are stored at 4°C for at least 1h (preferentially overnight) to form stable hybrid scaffolds.

*Recovery of hybrid microparticles from continuous phase*

Solidified hybrid microparticles accumulate on top of the emulsion oil. The emulsion oil is removed carefully with a pipette taking care not to remove the particles. Afterwards, 500μL iH,iH,2H,2H-perfluorooctanol (PFO; Sigma) is added to the tube to break the suspension. To transfer the hybrid hydrogel particles into the oil phase, the tube is vortexed for 5s and centrifuged at 2,500 x g for 5s. The hybrid hydrogel microparticles are transferred to a fresh 1.5 mL microcentrifuge tube. Optional: This procedure can be repeated to remove residual fluorocarbon oil and surfactant. Following the PFO wash, recovered microparticles are washed once with 1mL nuclease-free water. Microparticle quality and sizes are visually examined using a microscope. Exemplary nine microparticle preparations are shown in figure 4 (on the left as coloured sediments of individual microparticle preps in microtubes, in the center image a mixed set of nine different microparticles is shown).

*Functionalisation of hybrid precursor microparticles*

To be able to equip hybrid precursor microparticles with analyte-specific components, a flexible binding chemistry is established. Streptavidin is covalently attached to the amine-containing fraction of the hybrid matrix of the microparticles using the following 3-step protocol. First, sulfhydryl groups are added to Streptavidin using the amine-reactive portion of SPDP reagent (NHS) ester and conducting a subsequent reduction step. In a second step, a fraction of the amino groups of precursor microparticles is maleimide-activated using a Sulfo-SMCC crosslinking reagent. Finally, activated streptavidin and the maleimide-activated hybrid microparticles are conjugated resulting in nanoreactor precursors with a porous polymeric matrix and a reagent binding component.

*Protocol 1:* SPDP crosslinking

A vial of 3-(2-Pyridyldithio)propionic acid N-hydroxysuccinimide ester (N-Succinimidyl 3-(2-pyridyldithio)propionate; SPDP; P3415, Sigma) crosslinker is allowed to equilibrate to ambient temperatures before opening to prevent condensation. For the SPDP modification of Streptavidin, a 2-fold molar excess of SPDP over streptavidin is used. SPDP is dissolved in $50\mu L$ DMF to give a 0,23 molar SPDP solution. Also, 300mg of 15.8 U/mg Streptavidin (SA10; Prozyme) is dissolved in 10mL 100mM potassium phosphate buffer containing 20mM NaCl (pH 7.5) to make a 30mg/mL solution. The solution is centrifuged at 3000rpm and the supernatant is kept on ice for further experiments. The full volume ($50\mu L$) of the SPDP solution is added to 30mL of the streptavidin solution and the reaction is allowed to proceed overnight at 4°C. The reaction is quenched by adding Tris to a final concentration of 100 mM and further incubated at room temperature for 30min. In a subsequent step, unreacted SPDP is removed from the streptavidin solution by centrifugation at 8000rpm for 15min using Vivacon 500 Ultrafiltration columns (100 kDa MWCO) (Sartorius Stedim Biotech). The flow-through is discarded and washing with 100mM potassium phosphate buffer containing 20mM NaCl repeated 5 times. The activated streptavidin is reduced by incubation with 2mM DTT at ambient temperatures for 30min. Thus, the pyridine-2-thione groups are removed from the modified streptavidin.

*Protocol 2:* Maleimid-activation of gelatin fraction (coupling of Sulfo-SMCC)

A fresh vial of 4-(N-Maleimidomethyl)cyclohexane-1-carboxylic acid 3-sulfo-N-hydroxysuccinimide ester sodium salt (Sulfo-SMCC; M6035, Sigma) is allowed to fully equilibrate to ambient temperature before opening. Amine-containing hybrid microparticles are washed three times in non-amine containing conjugation buffer (potassium phosphate, 20mM NaCl pH 7.2). Immediately before use, a 10mg/mL of Sulfo-SMCC stock solution is prepared for conjugation. Sufficient Sulfo-SMCC stock solution is added to the microparticle solution to obtain 10 molar excess of crosslinking reagent over available amino groups (in the case of gelatine typeB-10% of amino acids of gelatine are expected to carry free amino groups. A 50% (v/v) hydrogel microparticle slurry is incubated with 10mM Sulfo-SMCC and immediately put on ice. The reaction is allowed to proceed overnight at 4°C at 100rpm using the Multi-Rotator PTR-60 (Grant-bio) in the vertical mode. The reaction is quenched by adding Tris to a final concentration of 100 mM followed by an incubation at room temperature for 30min. Maleimide activated particles are purified by repeated washing in conjugation buffer and centrifugation at 1000rcf.

*Protocol 3:* Conjugation of activated proteins

The maleimide-activated microparticles and the sulfhydryl-modified Streptavidin are conjugated. The Maleimide groups react with sulfhydryl groups at pH 6.5-7.5 to form stable non-cleavable thioether bonds.
Quenching is performed by adding 2-mercaptoethanol (Sigma) to a final concentration of 2mM and incubation at RT for 30min at 1000 rpm in a shaker incubator. A second quenching step is conducted with N-(2-hydroxyethyl)maleimide (Sigma) to give final concentration of 6mM and Incubation at RT while mixing. Binding capacity was determined using a biotinylated dye-conjugate.

## Example 2

### Library preparation

*Reversible attachment of target-specific primers as analyte-specific reagent(s) to precursor microparticles*

Each fluorescently colour-labelled hybrid hydrogel microparticle is capable of specifically detecting a different target of interest. To equip each class of microparticle with a primer set that is compatible with its target, functionalized gelatin/agarose hybrid microparticle aliquots are incubated with desthio-biotinylated primers in individual 2.0 mL microcentrifuge tubes. The desthiobiotin moiety of the oligonucleotide facilitates release of the oligonucleotides from the microparticle when temperature is raised, and an emulsion formed in the subsequent signal amplification step. This makes the oligonucleotides immediately available for the detection reaction.
Thus, precursor microparticles are washed once with nuclease-free water followed by resuspension of the microparticle pellet in equal amounts of nuclease-free water to make a 50% microparticle slurry. Differently labelled slurry aliquots are prepared in that aliquots of the 50% microparticle slurry are pelleted. An equal volume of each target-specific desthiobiotin-labelled primer pair (component 5-8) is added to one microsphere pellet to obtain a 50% bead slurry at a final concentration of 200nM of each primer thereby assigning a specific analyte-specific reagent. To ensure efficient binding of primers to the hybrid hydrogel matrix, both components are incubated at 20°C for 15min while shaking at 1000rpm. Microspheres are washed three times with a five times higher volume of nuclease-free water to remove unattached primers.

*Component 5: rpoB primer pair (analyte-specific reagent 1)*

- 0.2$\mu$M desthiobiotin-labeled *rpoB* sense primer (5'-ATCAACATCCGGCCGGTGGTCGCC -3') SEQ ID NO:1 (Metabion International AG)
- 0.2$\mu$M desthiobiotin-labeled *rpoB* antisense primer (5'-TCACGTGACAGACCGCCGGGC -3') SEQ ID NO:2 (Metabion International AG)

*Component 6: IS6110 primer pair (analyte-specific reagent 2)*

- 0.2$\mu$M desthiobiotin-labeled *IS6110* sense primer (5'-CGCCGCTTCGGACCACCAGCAC-3') SEQ ID NO:3 (Metabion International AG)
- 0.2$\mu$M desthiobiotin-labeled *IS6110* antisense primer (5'-GTGACAAAGGCCACGTAGGCGAACC-3') SEQ ID NO:4 (Metabion International AG)

*Component 7: IS1081 primer pair (analyte-specific reagent 3)*

- 0.2$\mu$M desthiobiotin-labeled IS1081 sense primer (5'-GCGCGGCAAGATCATCAATGTGGAG-3') SEQ ID NO:5 (Metabion International AG)
- 0.2$\mu$M desthiobiotin-labeled IS1081 antisense primer (5'-GCCACCGCGGGGAGTTTGTCG-3') SEQ ID NO:6 (Metabion International AG)

*Component 8: atpD (internal control) primer pair (analyte-specific reagent 4)*

- 0.2$\mu$M desthiobiotin-labeled internal control (Bac. globigii) sense primer (5'-GCGCGGCAAGATCATCAATGTGGAG-3') SEQ ID NO:7 (Metabion International AG)
- 0.2$\mu$M desthiobiotin-labeled internal control (Bac. globigii) antisense primer (5'-GCCACCGCGGGGAGTTTGTCG-3') SEQ ID NO:8 (Metabion International AG)

*Lyophilisation of microparticle library*

Optionally, the microsphere library is lyophilized to give dry analyte/target-specific microparticle pellets for long-term storage. Therefore, equal amounts of desired target-specific microparticle are pipetted together and sufficiently mixed using a vortexer. The microparticle library is supplemented with an equal volume of a 600mg/mL trehalose solution to generate a 30% (w/v) trehalose containing microparticle library slurry. Subsequently, library aliquots of 100$\mu$l are prepared in RNase/DNase-free PCR strip tubes ready for lyophilisation. The type of excipient (e.g. trehalose) and its concentration in the lyophilisation formulation affects the degree of swelling of the freeze-dried microparticle when exposed to an eluate later in the process.

The library aliquots were freeze-dried under vacuum (-25°C and 0.1mbar) using the Alpha 2-4 LSCplus freeze-drier (Christ) after freezing on dry ice for 2h. The samples were left in the freeze dryer for a total time of 200min. The main drying stage was held at 0.01mbar for 3h with a stepwise increase in temperature, from -25°C to 25°C. The final drying step was conducted at 25°C and 0.05mbar for 20min. The final product is:

*Component 9: lyophilized nanoreactor library*

- comprising target-specific reagents required for parallel detection of *rpoB, IS6110, IS1081* and internal control in one sample

**Example 3**

**Analyte Multiplexing using nanoreactors**

In the following embodiment, a sample (eluate) is encapsulated in a monodispersed suspension using the target-specific microparticles of the nano-reactor library.

*Loading of the analyte and the generic detection reagents to the nanoreactor library*

The generic reagents for analyte detection within the nano-reactors are supplied as a freeze-dried pellet that is resuspended with an analyte-containing eluate derived from an upstream sample preparation process. In this example a real sample mimicked by using a defined concentration of purified PCR products for *rpoB, IS6110, IS1081* and *atpD* with target sequences to be amplified (originally derived from H37Rv DNA) in nuclease-free water.

The entire volume of the sample (100μl) containing either no template molecules or defined amounts of target molecules are diluted in a MTB (=*Mycobacterium tuberculosis*) negative patient sputum eluate and added to a lyophilised generic reagent pellet (component 10). The reagents are carefully resuspended by a short vortexing step. Likewise, the entire volume of the generic reagent mix including the template molecules is added to the nano-reactor library pellet (component 9). The porous microparticles are allowed to absorb the entire liquid including all detection reagents and template molecules for 15 min at ambient temperatures while shaking at 1000rpm. In this process, the four templates are distributed randomly among the four types of reagent specific nanoreactors. In the subsequent digital amplification step only templates matching the specific reagent set of the nanoreactors can be amplified and detected. Consequently, the number of positive events in the digital amplification is reduced by a factor n, where n corresponds to the number of nanoreactor types in the nanoreactor library.

*Component 10 consists of the following final concentrations*

- PCR Buffer: 20mM Tris HCl, 22mM KCl, 22mM $NH_4Cl$, 3mM $MgCl_2$
- 0.2 U/μl Hot Start Taq DNA Polymerase (biotechrabbit GmbH)
- 0.4 mM dNTPs (biotechrabbit GmbH)
- 1μM EvaGreen® Fluorescent DNA Stain (JenaBioscience GmbH) or 0.4μM TaqMan probes
- 0.1% (w/v) low bioburden, protease free, for molecular biology BSA (Sigma)

*Phase transfer of loaded microparticles*

The nano-reactor library is transferred into a non-aqueous phase by dispersing microparticles in component 11 to prevent crosstalk between target-specific reactions. The complete aqueous phase (100μL) is brought in contact with an excess of component 11 (500μL) using a 1.5mL microcentrifuge tube. High shear forces are applied to deagglomerate and emulsify aqueous microparticles in the fluorocarbon oil into single nano-reactors. The mixture is agitated by either application of ultrasound using the Sonifier™ S-450 and the Ultrasonics Sonifier™ Cup Horn (Branson) or by simply sliding the tube over the holes of a microcentrifuge tube rack 20 times at a frequency of approximately 20/s while pressing the tube against the rack surface. This applies mechanical stress and breaks attracting forces between the aqueous microparticles and creates surface tension forming a suspension/suspension. Both the hydrogel microparticles and excess aqueous phase are emulsified in the oil phase. The submicron-scaled droplets that are produced as a byproduct are eliminated by washing the suspension three times the mild centrifugation (400rcf). Repeated washing with the same oil (component 11) removes essentially all undesirable liquid droplets. Component 11 also yields efficient thermostability of the suspension for subsequent digital emulsion/suspension PCR.

*Component $_{11}$: phase transfer* & *signal amplification oil*

- HFE-7500 fluorocarbon oil (3M Deutschland GmbH) supplemented with
- 2-5% (v/v) PicoSurf (Dolomite Microfluidics) OR 2-5% (v/v) FluoSurf (Emulseo)

*Parallel digital PCR amplification reactions in microparticle-templated reaction spaces*

The monodispersed suspension with encapsulated sample is transferred into a detection chamber with an area of approximately 2.5 cm$^2$ and a layer thickness of 100 μm. The chamber detection window is made of a 0.8mm Polycarbonate (Makrolon 6555; Covestro AG) while the opposite side of the chamber is composed of polished and unmodified transparent 125-micron Polycarbonate (Lexan 8010) film (Koenig Kunststoffe GmbH) facilitating efficient heat transfer necessary for individual nanoliter reactions. Nanoreactors suspended in the fluorocarbon oil are forced to form a monolayer owing to the dimension of the reaction chamber. Thus, microcapsules provide an evenly spaced array of approximately 20000-30000 nano-reactors (5000-7500/target) for subsequent parallel signal amplification reactions.

Microparticles are subjected to ultra-rapid temperature cycling using a modified PELTIER element 30x30x4.7mm, 19.3W (Quick-Ohm, Küpper & Co. GmbH, #QC-71-1.4-3.7M) and an established chamber-specific PCR control mode. The thermal conditions applied are: Initial denaturation for 30s at 95°C followed by 30-45 cycles of a two-step PCR consisting of Denaturation at 95°C for is and Annealing/Elongation at 64°C for 4s. Due to their sol-gel switching capability, the suspension becomes an emulsion with individual liquid nanoliter droplets. Furthermore, microparticles release desthiobiotin bound target-specific oligonucleotides from the gelatin matrix when initially heated to 95°C making it available for the PCR. The multiplexed amplification of individual targets takes place in the resulting nano reaction compartments.

Automated Image acquisition is triggered by the BLINK toolbox software and is done with a Fluorescence microscope (Zeiss AxioObserver) equipped with a 5x objective (field of view 4.416mm x 2.774mm) and a pE-4000 (CoolLED Ltd.) light source. The microscope was further equipped with three fluorescence filter sets (Cy5 ET, Cy3 ET, FITC/FAM HC, AHF Analysentechnik) and an automated x-y stage to which the thermocycler with the reaction chamber was mounted.

Image acquisition settings are as following: 100-1000ms and gain of 1-10x. Two images are required for label identification ($\lambda$exc 1 = 550nm, $\lambda$exc 2 = 650nm) and one image for specific PCR signals ($\lambda$exc 3 = 470nm). For optional nanoreactor specific real time analysis, three images corresponding to three fluorescence channels can be taken at the end of each annealing step of the thermal protocol at one position of the chamber.

Upon completion of the thermal protocol, the whole detection chamber is scanned using the same equipment at the settings mentioned above. With the set up outlined before in total 30 images are required to cover the dimension of the amplification/detection chamber. Figure 7 A shows images of the same area for three fluorescence channels, whereby channel 1 and 2 represent the label ratio encoding the respective analyte specific reagent provided with the microparticle and channel 3 showing microparticles with negative (dark) and positive (bright) amplification signals. The graph on the right shows a scatter plot of the fluorescence signal obtained for each microparticle in channels 1 and 2. Four different microparticles species are clearly recognizable.. Optionally, the nanoreactors are subjected to a stepwise elevation (2°C/step) from 50°C - 90°C to analyse DNA melting behaviour of the amplified product in order to determine the degree of specificity or to identify e.g. SNPs. DNA strain denaturation and the associated fluorescence signal decay is monitored by acquiring a fluorescence image at each temperature step.

*Decoding of microparticles & multiplexed analysis*

All images acquired are subjected to an automated multifaceted image processing algorithm. The method employs image segmentation exploiting the Maximally Stable Extremal Regions (MSER) to detect neighboring droplets from the result of MSER-based image segmentation. In detail, images are first subjected to preprocessing involving a median filter. Secondly, the MSER algorithm is applied to the image background to determined convex turning points of background outlines and their Delaunay triangulation to identify appropriate cuts between the droplets/microparticles. Furthermore, droplets/microparticles are segmented using the MSER algorithm. Finally, a plausibility check for droplet/microparticle outlines is performed (contrast, form, convexity). Features including fluorescence signals in each channel, position, diameter/volume, etc. of all segmented droplets/microparticles are subsequently collected. Experiment data is applied to a Jupyter script identifying individual labels (combination of fluorescent dyes Cy3 & Cy5) and their respective specific amplification and melting curve signals.

The following data readouts are possible:

a) Endpoint analysis (digital readout)

The PCR is amplified to an endpoint and thus the total number of fluorescent positive and negative droplets is determined for each individual label. Positive droplets contain at least 1 copy of the specific target and thus show an increase fluorescence signal above a defined intensity threshold. The threshold value is derived from previously performed amplification reactions without template. Droplet digital PCR data for each labelled target is viewed in a 1-D or 2-D plot. The software first clusters the negative and positive fractions for each nanoreactor volume and then fits the fraction of positive droplets to a Poisson algorithm to determine the initial concentration of the target DNA molecules in units of copies/mL input. Since the assay described above is a 4-plex reaction the droplets cluster into various groups depending on the concentration of templates added:

    a) Label component 1 (1:0 ratio Cy3 & Cy5), detection signal (EvaGreen/ probe) negative
    b) Label component 1 (1:0 ratio Cy3 & Cy5), detection signal (EvaGreen/ probe) positive
    c) Label component 2 (3:1 ratio Cy3 & Cy5), detection signal (EvaGreen/ probe) negative
    d) Label component 2 (3:1 ratio Cy3 & Cy5), detection signal (EvaGreen/ probe) positive
    e) Label component 3 (1:3 ratio Cy3 & Cy5), detection signal (EvaGreen/ probe) negative
    f) Label component 3 (1:3 ratio Cy3 & Cy5), detection signal (EvaGreen/ probe) positive
    g) Label component 4 (0:1 ratio Cy3 & Cy5), detection signal (EvaGreen/ probe) negative
    h) Label component 4 (0:1 ratio Cy3 & Cy5), detection signal (EvaGreen/ probe) positive

Such 1-D plots are shown for each microparticle with its respective label component in figure 7 B. Positive microparticles are clearly discriminated from negative microparticles for types 0 and 3, whereas types 2 and 3 only show negative microparticles. The calculated target concentration in the sample is shown in the table in figure 7B.

b) Real-time analysis

Average pixel intensities for each target-specific nanoreactor were tracked through all cycles of the PCR generating real-time fluorescence curves for single nanoreactors. These exhibit the typical exponential, linear, and plateau phases of the PCR, comparable to those observed in microliter-scale reactions. Sigmoid and linear fitting is performed on all nanoreactors using the Levenberg-Marquardt algorithm. Lift (change in fluorescence post PCR vs. prePCR) criteria are applied to eliminate implausible curves. Negative and positive real-time curves are generated, and a cycle threshold value is determined if positive. False positives (evaporating nanoreactors, artefacts) are identified and excluded from the analysis.

Literature:

Adler, M., R. Wacker and C. M. Niemeyer (2003). "A real-time immuno-PCR assay for routine ultrasensitive quantification of proteins." Biochem Biophys Res Commun 308(2): 240-250.

Birtwell, S. and H. Morgan (2009). "Microparticle encoding technologies for high-throughput multiplexed suspension assays." Integr Biol (Camb) 1(5-6): 345-362.

Fulton, R. J., R. L. McDade, P. L. Smith, L. J. Kienker and J. R. Kettman, Jr. (1997). "Advanced multiplexed analysis with the FlowMetrix system." Clin Chem 43(9): 1749-1756.

Leng, X., W. Zhang, C. Wang, L. Cui and C. J. Yang (2010). "Agarose droplet microfluidics for highly parallel and efficient single molecule emulsion PCR." Lab Chip 10(21): 2841-2843.

Mandecki, W., B. Ardelt, T. Coradetti, H. Davidowitz, J. A. Flint, Z. Huang, W. M. Kopacka, X.

Lin, Z. Wang and Z. Darzynkiewicz (2006). "Microtransponders, the miniature RFID electronic chips, as platforms for cell growth in cytotoxicity assays." Cytometry Part A 69A(11): 1097-1105.

Rissin, D. M., C. W. Kan, T. G. Campbell, S. C. Howes, D. R. Fournier, L. Song, T. Piech, P. P.

Patel, L. Chang, A. J. Rivnak, E. P. Ferrell, J. D. Randall, G. K. Provuncher, D. R. Walt and D. C.

Duffy (2010). "Single-molecule enzyme-linked immunosorbent assay detects serum proteins at subfemtomolar concentrations." Nature Biotechnology 28: 595-599.

Tekin, H. C. and M. A. Gijs (2013). "Ultrasensitive protein detection: a case for microfluidic magnetic bead-based assays." Lab Chip 13(24): 4711-4739.

Todd, J., B. Freese, A. Lu, D. Held, J. Morey, R. Livingston and P. Goix (2007). "Ultrasensitive Flow-based Immunoassays Using Single-Molecule Counting."

Wilson, R., A. R. Cossins and D. G. Spiller (2006). "Encoded microcarriers for high-throughput multiplexed detection." Angew Chem Int Ed Engl 45(37): 6104-6117.

**Claims**

1. A library of prefabricated precursor-microparticles for making a library of prefabricated microparticles, said prefabricated microparticles being configured for performing specific detection of one or several analytes of interest in a sample, such specific detection occurring within such microparticles by a suitable chemical or biochemical reaction, each of said prefabricated precursor-microparticles in said library comprising:

   - a porous matrix, preferably a porous polymeric matrix, having a void volume for receiving an aqueous sample and for providing a reaction space for the specific detection of an analyte;
   - a reagent binding component allowing the attachment, preferably the reversible attachment, of an analyte-specific reagent to the precursor-microparticle; said reagent binding component being one of:

      (i) a polymer or polymer mixture that forms said porous matrix or is said porous matrix;
      (ii) a reagent binding molecule attached to said porous matrix;
      (iii) at least one ionisable group, or a plurality of ionisable groups, immobilized on said porous matrix, said ionisable group(s) being capable of changing its(their) charge(s) according to ambient conditions surrounding said precursor-microparticle;
      (iv) at least one charged group, or a plurality of charged groups immobilized on said porous matrix;
      (v) a combination of any of (i) - (iv);

   - a label component attached to, contained within or otherwise associated with said precursor-microparticle for identifying said analyte-specific reagent, when attached to the precursor-microparticle.

2. The library according to claim 1, wherein, in said library, there are at least two separate subsets of prefabricated precursor-microparticles, preferably three or more separate subsets of prefabricated precursor-microparticles, with each subset having its distinct label component attached to, contained within or otherwise associated with said precursor-microparticles within said subset, such that said at least two or more separate subsets of prefabricated precursor-microparticles differ by the respective label component attached to, contained within or otherwise associated with each subset.

3. A library of prefabricated microparticles for performing specific detection of one or several analytes of interest in a sample, such specific detection occurring within such microparticles by a suitable chemical or biochemical reaction, each of said prefabricated microparticles comprising a prefabricated precursor-microparticle according to any of claims 1 - 2 and further comprising an analyte-specific reagent attached, preferably reversibly attached, to said precursor-microparticle.

4. The library of prefabricated microparticles according to claim 3, wherein said analyte-specific reagent that is attached to each of said precursor-microparticles, is attached, preferably reversibly attached, through said reagent binding component by

   a) direct binding of said analyte-specific reagent to said polymer or polymer mixture that forms said porous polymeric matrix or is part of said porous polymeric matrix (i);
   b) said analyte-specific reagent being conjugated to a binding entity which, in turn, binds to said reagent binding molecule (ii);
   c) direct binding of said analyte-specific reagent to said ionisable group(s) (iii) under conditions in which said ionisable group(s) has(have) a suitable net charge;
   d) direct binding of said analyte-specific reagent to said charged group(s) (iv) on said polymer, wherein said analyte-specific reagent has at least one ionisable group, or a plurality of ionisable groups, said ionisable group(s) being capable of changing its(their) charge(s) according to ambient conditions surrounding said analyte-specific reagent; or
   e) a combination of any of (a) - (d).

5. The library of prefabricated precursor-microparticles according to any of claims 1 - 2 or the library of prefabricated microparticles according to any of claims 3 - 4, wherein

   - said polymer (i) is a hydrogel-forming agent selected from the group comprising ia) synthetic polymers, such as poly(methyl)(meth)acrylate, polyamide; ib) silicone-based polymers, e.g. polydimethylsiloxanes; ic) naturally occurring polymers selected from polysaccharides, e.g. agarose, chitin, chitosan, dextran, alginate, carrageenan, cellulose, fucoidan, laminaran; gums selected from xanthan gum, arabic gum, ghatti gum, guar gum, locust bean gum, tragacanth gum, karaya gum, and inulin; polypeptides, e.g. collagens, gelatins; poly-amino acids, such as poly-lysine; polynucleotides; and said polymer mixture is a combination of any of the foregoing;

- said reagent binding molecule (ii) is selected from avidin, in particular tetrameric avidin; streptavidin; monomeric avidin; avidin having a nitrated tyrosine in its biotin binding site; other proteins, derived from or related to, avidin and retaining binding functionality of avidin; biotin; desthiobiotin; iminobiotin; biotin having a cleavable spacer arm; selenobiotin; oxybiotin; homobiotin; norbiotin; iminobiotin; diaminobiotin; biotin sulfoxide; biotin sulfone; epibiotin; 5-hydroxybiotin; 2-thiobiotin; azabiotin; carbobiotin; methylated derivatives of biotin; ketone biotin; other molecules, derived from or related to, biotin and retaining binding functionality of biotin;

- said at least one ionisable group (iii) or said ionisable group of claim 4 d) is selected from

- N-2-acetamido-2-aminoethanesulfonic acid (ACES);
- N-2-acetamido-2-iminodiacetic acid (ADA);
- amino methyl propanediol (AMP);
- 3-1,1-dimethyl-2-hydroxyethylamino-2-hydroxy propanesulfonic acid (AMPSO);
- N,N-bis2-hydroxyethyl-2-aminoethanesulfonic acid (BES);
- N,N-bis-2-hydroxyethylglycine (BICINE);
- bis-2-hydroxyethyliminotrishydroxymethylmethane (Bis-Tris);
- 1,3-bistrishydroxymethylmethylaminopropane (Bis-Tris Propane);
- 4-cyclohexylamino-1-butane sulfonic acid (CABS);
- 3-cyclohexylamino-1-propane sulfonic acid (CAPS);
- 3-cyclohexylamino-2-hydroxy-1-propane sulfonic acid (CAPSO);
- 2-N-cyclohexylaminoethanesulfonic acid (CHES);
- 3-N,N-bis-2-hydroxyethylamino-2-hydroxypropanesulfonic acid (DIPSO);
- N-2-hydroxyethylpiperazine-N-3-propanesulfonic acid (EPPS);
- N-2-hydroxyethylpiparazine-N-4-butanesulfonic acid (HEPBS);
- N-2-hydroxyethylpiperazine-N-2-ethanesulfonic acid (HEPES);
- N-2-hydroxyethylpiperazine-N-2-propanesulfonic acid (HEPPSO);
- 2-N-morpholinoethanesulfonic acid (MES);
- 4-N-morpholinobutanesulfonic acid (MOBS);
- 3-N-morpholinopropanesulfonic acid (MOPS);
- 3-N-morpholino-2-hydroxypropanesulfonic acid (MOPSO);
- piperazine-N-N-bis-2-ethanesulfonic acid (PIPES);
- piperazine-N-N-bis-2-hydroxypropanesulfonic acid (POPSO);
- N-trishydroxymethyl-methyl-4-aminobutanesulfonic acid (TABS);
- N-trishydroxymethyl-methyl-3-aminopropanesulfonic acid (TAPS);
- 3-N-trishydroxymethyl-methylamino-2-hydroxypropanesulfonic acid (TAPSO);
- N-trishydroxymethyl-methyl-2-aminoethanesulfonic acid (TES);
- N-trishydroxymethylmethylglycine (TRICINE);
- trishydroxymethylaminomethane (Tris);
- polyhydroxylated amines;
- imidazole, and derivatives thereof (i.e. imidazoles), especially derivatives containing hydroxyl groups;
- triethanolamine dimers and polymers; and di/tri/oligo/poly amino acids, such as for example Ala-Ala; Gly-Gly; Ser-Ser; Gly-Gly-Gly; or Ser-Gly. Oligo-His, Poly-His, Oligo-Lys, Poly-Lys.

6. The library of prefabricated precursor-microparticles according to any of claims 1 - 2, 5 or the library of prefabricated microparticles according to any of claims 3 - 5, wherein said porous polymeric matrix, or said polymer or polymer mixture that forms or is part of said porous polymeric matrix, is composed of a polymer that is not crosslinked, wherein, preferably, said polymer or polymer mixture that forms or is part of said porous polymeric matrix, is composed of agarose or a combination of agarose and gelatin, wherein, more preferably, in said combination of agarose and gelatin, said agarose is present in a range of from 0.1 %(w/v) to 4%(w/v), and said gelatin is present in a range of from 0.1%(w/v) to 20%(w/v), preferably from 0.5%(w/v) to 20%(w/v).

7. The library of prefabricated microparticles according to any of claims 3 - 6, wherein said analyte-specific reagent is selected from nucleic acids, including aptamers, Spiegelmers; antibodies or antibody fragments; non-antibody proteins capable of specifically binding an analyte or analyte complex, such as receptors, receptor fragments, and affinity proteins; wherein preferably said analyte-specific reagent is selected from nucleic acids, in particular nucleic acid oligomers and nucleic acid primers.

8. The library of prefabricated microparticles according to any of claims 3 - 7, wherein, for each of said microparticles, said analyte-specific reagent is reversibly attached to said microparticle through said reagent binding component by b) said analyte-specific reagent being conjugated to a binding entity which, in turn, binds to said reagent binding molecule (ii), wherein

- said binding entity is independently selected from biotin, desthiobiotin, iminobiotin, biotin having a cleavable spacer arm, selenobiotin, oxybiotin, homobiotin, norbiotin, iminobiotin, diaminobiotin, biotin sulfoxide, biotin sulfone, epibiotin, 5-hydroxybiotin, 2-thiobiotin, azabiotin, carbobiotin, methylated derivatives of biotin, ketone biotin, other molecules, derived from or related to, biotin and retaining binding functionality of biotin; and said reagent binding molecule is independently selected from avidin and streptavidin; or vice versa; or
- said binding entity is biotin, and said reagent binding molecule is selected from monomeric avidin, avidin having a nitrated tyrosine in its biotin binding site, and other proteins, derived from or related to, avidin and retaining binding functionality of avidin; or vice versa.

9. The library of prefabricated microparticles according to any of claims 3 - 8, wherein said library is for performing a specific detection of a single analyte of interest in several samples, wherein, in said library, there are at least two separate subsets of prefabricated microparticles, preferably three or more separate subsets of prefabricated microparticles, with each subset

- having its distinct label component attached to, contained within or otherwise associated with said microparticles of said subset; and

all of said at least two, three or more separate subsets having

- the same analyte-specific reagent attached to the porous matrix of said microparticles of said subsets, said analyte-specific reagent being specific for one analyte of interest;

such that said at least two or more separate subsets of prefabricated microparticles are identical in terms of the analyte-specific reagent attached, but differ by

- the respective label component attached to, contained within or otherwise associated with said microparticles of each subset;

with each subset being unambiguously defined and identifiable by said respective label component.

10. The library of prefabricated microparticles according to any of claims 3 - 8, wherein said library is for performing a specific detection of multiple analytes of interest in a single sample, wherein, in said library, there are at least two separate subsets of prefabricated microparticles, preferably three or more separate subsets of prefabricated microparticles, with each subset

- having its distinct label component attached to, contained within or otherwise associated with said microparticles of said subset; and
- having a different analyte-specific reagent attached to the porous matrix of said microparticles of said subset; each analyte-specific reagent being specific for one analyte of interest;

such that said at least two or more separate subsets of prefabricated microparticles differ by

- the respective label component attached to, contained within or otherwise associated with said microparticles of each subset; and
- the respective analyte-specific reagent attached to each subset;

with each subset being unambiguously defined and identifiable by said respective label component and said respective analyte-specific reagent.

11. The library of prefabricated microparticles according to any of claims 3 - 8, wherein said library is for performing a specific detection of multiple analytes of interest in several samples, wherein, in said library, there are a plurality of different separate subsets of prefabricated microparticles,
with each subset

- having its distinct label component attached to, contained within or otherwise associated with said microparticles of said subset; and

wherein, in said plurality of separate subsets of prefabricated microparticles, there are different classes of separate subsets of prefabricated microparticles, with each of said classes comprising several subsets of microparticles and each class having a different analyte-specific reagent attached to the porous matrix of said microparticles, and all subsets of microparticles within one class having the same analyte-specific reagent attached; each analyte-specific reagent being specific for one analyte of interest;
such that, in said library, said plurality of different separate subsets of prefabricated microparticles differ by

- the respective label component attached to, contained within or otherwise associated with said microparticles of each subset; and

each separate subset of microparticles forms part of one class of subsets of microparticles;
with each subset being unambiguously defined and identifiable by said respective label component and said respective analyte-specific reagent; and such that said different classes of subsets of microparticles differ by the respective analyte-specific reagent attached to the porous matrix of said microparticles; and each of said different classes comprises several subsets of microparticles, all of which subsets within one class having the same analyte-specific reagent attached.

12. A kit for making a library of prefabricated microparticles according to any of claims 3 - 11, said kit comprising:

- at least two containers, namely a first and a second container, and, optionally, further containers, each containing:

• a subset of prefabricated precursor-microparticles as defined in any of claims 1 - 2, 5 and 6, with each subset having its distinct label component attached to, contained within or otherwise associated with said precursor-microparticles within said subset, such that said two subsets of prefabricated precursor-microparticles differ by the respective label component attached to, contained within or otherwise associated with each subset;

- a further container containing:

• a conditioning solution to enable the attachment, preferably the reversible attachment, of an analyte-specific reagent to said subset of said precursor-microparticles through said reagent binding component by

a) direct binding of said analyte-specific reagent to said polymer or polymer mixture that forms said porous polymeric matrix or is part of said porous polymeric matrix (i);
b) said analyte-specific reagent being conjugated to a binding entity which, in turn, binds to said reagent binding molecule (ii);
c) direct binding of said analyte-specific reagent to said ionisable group(s) (iii) under conditions in which said ionisable group(s) has(have) a suitable net charge;
d) direct binding of said analyte-specific reagent to said charged group(s) (iv) on said polymer, wherein said analyte-specific reagent has at least one ionisable group, or a plurality of ionisable groups, said ionisable group(s) being capable of changing its(their) charge(s) according to ambient conditions surrounding said analyte-specific reagent; or
e) a combination of any of (a) - (d);

said reagent binding component being as defined in claim 1, said reagent binding molecule being as defined in any of claims 1, 4 and 5, and said binding entity being as defined in any of claims 4 - 5; said polymer or polymer mixture being as defined in any of claims 1, 4 - 6; and said ionisable groups being as defined in any of claims 1, 4, and 5, and said analyte-specific reagent being as defined in any of claims 3 - 4, 7, and 8.

**13.** A method of making a library of prefabricated microparticles according to any of claims 3 - 11, said method comprising:

- Providing, in any order:

    • a library of prefabricated precursor-microparticles as defined in any of claims 1 - 2, 5 and 6;
    • at least one analyte-specific reagent, as defined in any of claims 3 - 4, 7, and 8;

- mixing said library of prefabricated precursor-microparticles, or selected subsets thereof, and the at least one analyte-specific reagent under conditions allowing the attachment, preferably the reversible attachment, of said at least one analyte-specific reagent to some or all of said prefabricated precursor-microparticles, thus generating a library of prefabricated microparticles according to any of claims 5 - 13;
- optionally, washing said prefabricated microparticles to remove any unattached analyte-specific reagent therefrom.

**14.** A kit for detecting an analyte in a sample, said kit comprising:

a)

    - a container containing a generic detection composition comprising reagents for performing a chemical or biochemical detection reaction of an analyte, wherein said chemical or biochemical detection reaction of an analyte is a nucleic acid amplification, said generic detection composition comprises a buffer, mononucleoside-triphosphates, an amplification enzyme, such as a suitable nucleic acid polymerase, e.g. Taq polymerase, and a nucleic acid dye for the detection of an amplification product, such as an amplified nucleic acid, OR
    - a container containing a first detection composition comprising reagents for performing a chemical or biochemical detection reaction of an analyte, and yet a further container containing a second detection composition comprising a detection reagent, wherein said chemical or biochemical detection reaction of an analyte is an immunochemistry detection reaction, said first detection composition comprises reagents for performing an immunochemistry detection reaction, such as a buffer, and a secondary antibody or secondary antibody fragment coupled to a suitable reporter enzyme and being specific for the same analyte as a primary antibody, antibody fragment, or non-antibody protein, used as analyte-specific reagent (ASR) in said immunochemistry detection reaction; and said second detection composition comprises, as a detection reagent, a suitable substrate for said reporter enzyme which substrate upon having been reacted by said reporter enzyme, becomes detectable, preferably optically detectable, more preferably detectable by fluorescence, OR
    - a container containing a first detection composition comprising reagents for performing a chemical or biochemical detection reaction of an analyte, and yet a further container containing a second detection composition comprising a detection reagent, wherein the chemical or biochemical reaction is an immunochemistry detection reaction, said first detection composition comprises reagents for performing an immunochemistry detection reaction, such as a buffer, and a secondary antibody or secondary antibody fragment coupled to a suitable oligonucleotide tag and being specific for the same analyte as a primary antibody, used as analyte-specific reagent (ASR) in said immunochemistry detection reaction; and said second detection composition comprises, as detection reagent(s), a buffer, mono-nucleoside-triphosphates, an amplification enzyme, such as a suitable nucleic acid polymerase, e. g. Taq polymerase, and a nucleic acid dye for detection of an amplification product, such as an amplified nucleic acid, and primers suitable for amplifying the oligonucleotide tag attached to the secondary antibody; and

b)

    - a container containing a non-aqueous phase, such as an oil, e.g. fluorocarbon oil, optionally supplemented with an emulsifier; and

c)

    - a mixing container for mixing components, wherein, preferably, said kit further comprises

d)

- a container for performing a detection reaction; and wherein, more preferably, said kit further comprises

e)

- the kit according to claim 12, or
- a library of prefabricated microparticles according to any of claims 3 - 11.

15. A method of detecting and/or quantitating an analyte of interest in an aqueous sample, said method comprising the steps:

- providing, in any order:

• an aqueous sample known or suspected to contain an analyte of interest;
• a generic detection composition comprising reagents for performing a chemical or biochemical detection reaction of the analyte;
• a library of prefabricated microparticles according to any of claims 3 - 11, wherein said analyte-specific reagent(s) attached to said microparticles, is(are) chosen such that it is (they are) capable of specifically binding to or reacting with said analyte of interest;

- optionally, mixing said aqueous sample and said generic detection composition;
- incubating said aqueous sample with said library of prefabricated microparticles, thereby allowing said library of microparticles to absorb aqueous sample in the void volumes of said microparticles and, optionally, to bind said analyte of interest, if present in said sample;
- optionally, washing said microparticles;
- adding said generic detection composition to the microparticles, if said aqueous sample has not been previously mixed with said generic detection composition;
- transferring said library of prefabricated microparticles into a non-aqueous phase and removing any aqueous phase surrounding the individual prefabricated microparticle(s), thereby creating a plurality of insulated reaction spaces for detecting said analyte, which reaction spaces comprise an aqueous phase and are confined to said void volume(s) of said microparticles;
- optionally, releasing the analyte-specific reagent(s) attached to said microparticles by applying an external trigger;
- performing a detection reaction of said analyte of interest;
- detecting and/or quantitating said analyte of interest.

16. The method according to claim 15, wherein said analyte of interest is a nucleic acid, and

- said analyte-specific reagent is a nucleic acid or a pair of nucleic acids, sufficiently complementary to said analyte of interest to be able under hybridizing conditions to hybridize to said analyte of interest, wherein, preferably, said analyte-specific reagent is a suitable primer or primer pair for amplification of said analyte of interest;
- said generic detection composition comprises reagents for performing an amplification reaction of said nucleic acid analyte of interest, except for primers, wherein, in particular, said generic detection composition comprises a buffer, mono-nucleoside-triphosphates, an amplification enzyme, such as a suitable nucleic acid polymerase, e.g. Taq polymerase, and a nucleic acid dye for the detection of an amplification product, such as an amplified nucleic acid;
- said step of transferring is a step wherein said prefabricated microparticles are transferred into a non-aqueous phase and suspended and, optionally, repeatedly washed with said non-aqueous phase, said step more optionally involving also a filtration or mechanical agitation to ensure the formation of a monodisperse suspension, of microparticles with no aqueous phase or no substantial aqueous phase remaining outside of any of the microparticles;
- said optional step of releasing the analyte-specific reagent(s) attached to said microparticles by applying an external trigger is a step of temporarily increasing the temperature, changing the pH, or changing the salt conditions, preferably of increasing the temperature, more preferably from ambient temperature to a temperature >90°C.
- said step of performing a detection reaction is a step of performing an amplification reaction of said analyte if present in said aqueous sample; and
- said step of detecting and/or quantitating said analyte of interest is a step of detecting and/or quantitating said amplified analyte.

17. A method of detecting and/or quantitating an analyte of interest in an aqueous sample, said method comprising the steps:

- providing, in any order:

• an aqueous sample known or suspected to contain an analyte of interest;
• a first detection composition comprising necessary reagents for performing a chemical or biochemical detection reaction of the analyte;
• a second detection composition comprising a detection reagent;
• a library of prefabricated microparticles according to any of claims 3 - 11, wherein said analyte-specific reagent(s) attached to said microparticles, is(are) chosen such that it is (they are) capable of specifically binding to or reacting with said analyte of interest;

- optionally, mixing said aqueous sample and said first detection composition;
- incubating said aqueous sample with said library of prefabricated microparticles and, if said aqueous sample has not already been mixed with said first detection composition, also with said first detection composition, thereby allowing said library of microparticles to absorb aqueous sample and said first detection composition in the void volumes of said microparticles and, optionally, to bind said analyte of interest, if present in said sample;
- optionally, washing said library of prefabricated microparticles to remove any non-absorbed or non-reacted first detection composition;
- incubating said library of prefabricated microparticles including absorbed aqueous sample with said second detection composition, thereby allowing said library of microparticles to absorb said second detection composition;
- optionally, further washing said library of prefabricated microparticles to remove any non-absorbed or non-reacted second detection composition;
- transferring said library of prefabricated microparticles into a non-aqueous phase and removing any aqueous phase surrounding the individual prefabricated microparticle(s), thereby creating a plurality of insulated reaction spaces for detecting said analyte which reaction spaces comprise an aqueous phase and are confined to said void volume(s) of said microparticles; wherein, preferably, said step of transferring into a non-aqueous phase is performed immediately after said library of prefabricated microparticles have been incubated with said second detection composition;
- optionally, releasing the analyte-specific reagent(s) attached to said microparticles by applying an external trigger;
- detecting and/or quantitating said analyte of interest by detecting and/or quantitating said detection reagent.

18. The method according to claim 17, wherein said analyte of interest is a protein or other non-nucleic acid molecule, and

- said analyte-specific reagent is a primary antibody, antibody fragment, or a non-antibody protein capable of specifically binding said protein analyte or other non-nucleic acid analyte;
- said first detection composition comprises necessary reagents for performing an immunochemistry detection reaction, such as a buffer, and a secondary antibody or secondary antibody fragment coupled to a suitable reporter enzyme and being specific for said analyte;
- said second detection composition comprises, as a detection reagent, a suitable substrate for said suitable reporter enzyme which substrate upon having been reacted by said reporter enzyme, becomes detectable, preferably optically detectable, more preferably detectable by fluorescence;
- said step of incubating said aqueous sample with said library of prefabricated microparticles and with said first detection composition is a step of performing an immunochemistry reaction involving the binding of said analyte, if present, in said aqueous sample, to said primary antibody, primary antibody fragment, or non-antibody protein, thus forming a complex of analyte and said primary antibody, primary antibody fragment, or non-antibody protein; said immunochemistry reaction further involving a binding of said secondary antibody to said complex, thus forming a sandwich between primary antibody, antibody fragment or non-antibody protein, analyte, and secondary antibody;
- said first optional step of washing said library is a step of removing any non-bound secondary antibody from said library;

- said step of incubating said library of prefabricated microparticles including absorbed aqueous sample with said second detection composition is a step of allowing said substrate to be reacted by said reporter enzyme;
- said further optional step of washing said library is a step of removing any non-reacted substrate from said library;
- said step of transferring is a step wherein said prefabricated microparticles are transferred into a non-aqueous phase and suspended and, optionally, repeatedly washed with said non-aqueous phase, more optionally involving also a filtration or mechanical agitation to ensure the formation of a monodisperse suspension of microparticles with no aqueous phase or no substantial aqueous phase remaining outside of any of the microparticles;
- said optional step of releasing the analyte-specific reagent(s) attached to said microparticles by applying an external trigger is a step of temporarily increasing the temperature, changing the pH, or changing the salt conditions, preferably a step of increasing the temperature, more preferably from ambient temperature to a temperature >90°C;
- said step of detecting and/or quantitating said analyte of interest is a step of detecting and/or quantitating said reacted substrate.

19. A method of detecting and/or quantitating an analyte of interest in an aqueous sample, said method comprising the steps:

- providing, in any order:

  • an aqueous sample known or suspected to contain an analyte of interest;
  • a first detection composition comprising necessary reagents for performing a chemical or biochemical detection reaction of the analyte;
  • a second detection composition comprising a detection reagent;
  • a library of prefabricated microparticles according to any of claims 3 - 11, wherein said analyte-specific reagent(s) attached to said microparticles, is(are) chosen such that it is (they are) capable of specifically binding to or reacting with said analyte of interest;

- optionally, mixing said aqueous sample and said first detection composition;
- incubating said aqueous sample with said library of prefabricated microparticles and, if said aqueous sample has not already been mixed with said first detection composition, also with said first detection composition, thereby allowing said library of microparticles to absorb aqueous sample and said first detection composition in the void volumes of said microparticles and, optionally, to bind said analyte of interest, if present in said sample;
- optionally, washing said library of prefabricated microparticles to remove any non-absorbed or non-reacted first detection composition;
- incubating said library of prefabricated microparticles including absorbed aqueous sample with said second detection composition, thereby allowing said library of microparticles to absorb said second detection composition;
- optionally, further washing said library of prefabricated microparticles to remove any non-absorbed or non-reacted second detection composition;
- transferring said library of prefabricated microparticles into a non-aqueous phase and removing any aqueous phase surrounding the individual prefabricated microparticle(s), thereby creating a plurality of insulated reaction spaces for detecting said analyte which reaction spaces comprise an aqueous phase and are confined to said void volume(s) of said microparticles;
- optionally, releasing the analyte-specific reagent(s) attached to said microparticles by applying an external trigger;
- performing a detection reaction of said analyte of interest; and
- detecting and/or quantitating said analyte of interest.

20. The method according to claim 19, wherein said analyte of interest is a protein or other non-nucleic acid molecule, and

- said analyte-specific reagent is a primary antibody, antibody fragment, or a non-antibody protein capable of specifically binding said protein analyte or other non-nucleic acid analyte;
- said first detection composition comprises necessary reagents for performing an immunochemistry detection reaction, such as a buffer, and a secondary antibody or secondary antibody fragment coupled to a suitable oligonucleotide tag and being specific for the same analyte as said primary antibody;
- said second detection composition comprises, as detection reagent(s), a buffer, mono-nucleoside-triphosphates, an amplification enzyme, such as a suitable nucleic acid polymerase, e. g. Taq polymerase, and a nucleic acid dye for detection of an amplification product, such as an amplified nucleic acid, and primers suitable for amplifying the oligonucleotide tag attached to the secondary antibody;

- said step of incubating said aqueous sample with said library of prefabricated microparticles and with said first detection composition is a step of performing an immunochemistry reaction involving the binding of said analyte, if present, in said aqueous sample, to said primary antibody, primary antibody fragment, or non-antibody protein, thus forming a complex of analyte and said primary antibody, primary antibody fragment, or non-antibody protein; said immunochemistry reaction further involving a binding of said secondary antibody to said complex, thus forming a sandwich between primary antibody, antibody fragment or non-antibody protein, analyte, and secondary antibody;

- said first optional step of washing said library is a step of removing any non-bound secondary antibody from said library;

- said step of incubating said library of prefabricated microparticles including absorbed aqueous sample with said second detection composition is a step of allowing said primers in said second detection composition hybridize to the oligonucleotide tag attached to the secondary antibody;

- said further optional step of washing said library is a step of removing any non-hybridized primers from said library;

- said step of transferring is a step wherein said prefabricated microparticles are transferred into a non-aqueous phase and suspended and, optionally, repeatedly washed with said non-aqueous phase, more optionally involving also a filtration or mechanical agitation to ensure the formation of a monodisperse suspension, of microparticles with no aqueous phase or no substantial aqueous phase remaining outside of any of the microparticles;

- said optional step of releasing the analyte-specific reagent(s) attached to said microparticles by applying an external trigger is a step of temporarily increasing the temperature, changing the pH, or changing the salt conditions, preferably a step of increasing the temperature, more preferably from ambient temperature to a temperature >90°C;

- said step of performing a detection reaction is a step of performing an amplification reaction of said oligonucleotide tag; and

said step of detecting and/or quantitating said analyte(s) of interest is a step of detecting and/or quantitating said amplified oligonucleotide tag(s).

21. The method according to any of claims 15 - 20, wherein said step of releasing the analyte-specific reagent(s) attached to said microparticles by applying an external trigger is performed by raising the temperature to which said microparticles are exposed.

22. The method according to any of claims 15 - 21, wherein,

- the method is a method of detecting and/or quantitating a single analyte in a plurality of aqueous samples, e.g. from different patients,

- in said method, there are provided a plurality of different aqueous samples known or suspected to contain an analyte of interest, e.g. samples from different patients,

- the library of prefabricated microparticles that is provided in said method, is a library according to claim 9, wherein, in such library, there are as many or at least as many separate subsets of microparticles provided, as there are different aqueous samples provided and to be tested, wherein all of said separate substeps have the same analyte-specific reagent attached to the porous matrix of said microparticles of said subsets, said analyte-specific reagent being specific for one analyte of interest;

- in said step of incubating said aqueous samples with said library of prefabricated microparticles, each of said different aqueous samples is incubated separately with a separate subset of microparticles of said library;

- in said step of incubating said library of prefabricated microparticles including absorbed aqueous sample with a second detection composition, each of said separate subsets of microparticles of said library is incubated separately with said second detection composition;

- said step of transferring said library into a non-aqueous phase is performed for each subset of microparticles separately, i.e. each separate subset of microparticles is separately transferred into a non-aqueous phase, and the aqueous phase surrounding the individual microparticles is removed for each subset separately, thereby creating, for each subset separately, a plurality of insulated reaction spaces for detecting said analyte which reaction spaces comprise an aqueous phase and are confined to said void volume(s) of said microparticles,

wherein said method further comprises, after said step of transferring said library into a non-aqueous phase, a step of mixing said plurality of insulated reaction spaces of all the separate subsets in said non-aqueous phase together, before subsequently a detection reaction of said analyte of interest is performed, and before detecting and/or quantitating said analyte of interest.

23. The method according to any of claims 15 - 21, wherein,

- the method is a method of detecting and/or quantitating multiple analytes in a single aqueous sample,
- in said method, there is provided a single aqueous sample known or suspected to contain multiple analytes of interest,
- the library of prefabricated microparticles that is provided in said method, is a library according to claim 10, wherein, in such library, there are as many or at least as many separate subsets of microparticles provided, as there are different analytes of interest to be detected, with each of said separate subsets having a different analyte-specific reagent attached to the porous matrix of said microparticles of said subset; each analyte-specific reagent being specific for one analyte of interest;
- in said step of incubating said aqueous sample with said library of prefabricated microparticles, said aqueous sample is incubated with all of said separate subsets of microparticles of said library together;
- in said step of incubating said library of prefabricated microparticles including absorbed aqueous sample with a second detection composition, all of said separate subsets of microparticles of said library are incubated together with said second detection composition;
- said step of transferring said library into a non-aqueous phase is performed for all subsets of microparticles together, i.e. all subsets of microparticles are transferred together into a non-aqueous phase, and the aqueous phase surrounding the individual microparticles is removed, thereby creating a plurality of insulated reaction spaces for detecting and/or quantitating said multiple analytes which reaction spaces comprise an aqueous phase and are confined to said void volume(s) of said microparticles.

24. The method according to any of claims 15 - 21, wherein,

- the method is a method of detecting and/or quantitating multiple analytes in a plurality of aqueous samples,
- in said method, there are provided a plurality of different aqueous samples known or suspected to contain multiple analytes of interest, e.g. samples from different patients,
- the library of prefabricated microparticles that is provided in said method, is a library according to claim 11, wherein, in such library, there are different classes of separate subsets of prefabricated microparticles, with each of said classes comprising several subsets of microparticles and each of said classes having a different analyte-specific reagent attached to the porous matrix of said microparticles, and all subsets of microparticles within one class having the same analyte-specific reagent attached; each analyte-specific reagent being specific for one analyte of interest; wherein, in said method, in said step of providing said library, there are

  • as many or at least as many different classes of separate subsets of microparticles provided, as there are different analytes to be detected,
  • as many or at least as many different subsets of microparticles provided within each class , as there are aqueous samples provided and to be tested,
  • as many or at least as many different subsets of microparticles provided within the library as there are different aqueous samples to be tested multiplied by the number of different analytes of interest to be detected,

- in said step of incubating said aqueous sample with said library of prefabricated microparticles, , exactly one aqueous sample is incubated with exactly one subset of microparticles from each class, with each aqueous sample being incubated with as many different subsets of microparticles from different classes together as there are classes of microparticles in said library,
- in said step of incubating said library of prefabricated microparticles including absorbed aqueous sample with a second detection composition, the combined subsets from different classes of microparticles with which subsets one respective aqueous sample had been previously incubated, are incubated together with said second detection composition,
- said step of transferring said library into a non-aqueous phase is performed for each aqueous sample separately, namely the combined subsets of microparticles with which one respective aqueous sample had been previously incubated, are transferred together into a non-aqueous phase, and the aqueous phase surrounding the individual microparticles is removed, thereby creating, for each aqueous sample separately, a plurality of insulated reaction spaces for detecting said multiple analytes which reaction spaces comprise an aqueous phase and are confined to said void volume(s) of said microparticles,

wherein said method further preferably comprises, after said step of transferring said library into a non-aqueous phase, a step of mixing said pluralities of insulated reaction spaces of all the separate samples in said non-aqueous phase together, before subsequently a detection reaction of said analytes of interest is performed, and before detecting and/or quantitating said analytes of interest.

25. The method according to any of claims 15 - 24, wherein, in said step of detecting and quantitating said analyte of interest, quantitation of said analyte is performed by a method selected from:

a) digital nucleic acid amplification, in particular digital polymerase chain reaction (PCR);
b) real-time quantitative nucleic acid amplification, in particular real-time polymerase chain reaction (PCR);
c) immunochemistry detection methods, in particular digital immunochemistry detection methods, such as digital immunoassays, e.g. digital enzyme-linked immunosorbent assays (ELISAs);
d) immunochemistry detection methods combined with nucleic acid amplification, such as immuno-polymerase chain reaction; in particular digital immune-PCR;

wherein quantitation is performed using any of methods a) or b), or a combination of a) and b), if the analyte of interest is a nucleic acid; and wherein quantitation is performed using any of methods c) or d), if the analyte is a protein, peptide or other non-nucleic acid analyte.

**Synthesis of encoded precursor-microparticles**        **Library preparation**

Figure 1

Analyte-specific reagents (ASRs)

Labelled prefabricated precursor-microparticle with porous matrix

Labelled Prefabricated microparticle (including ASR)

+

Binding of ASR
by reagent binding,
washing

reagent binding component

label component

Figure 2

```
                  target specific              non-aqueous
                  microparticle -                solvent
                     library

                        │                           │
                        ▼                           ▼
  ┌─────────────┐  ┌──────────────┐          ┌──────────────┐        ┌────────────────────────┐
  │             │  │              │          │              │        │ amplification/ detection│
  │   sample    │─▶│  incubation  │─────────▶│phase transfer│───────▶│assignment of analyte   │
  │             │  │              │          │              │        │specific signal to      │
  └─────────────┘  └──────────────┘          └──────────────┘        │respective label        │
                        ▲                                            │component                │
                        │                                            └────────────────────────┘

                  amplification /
                    detection
                    reagents
```

Figure 3A

| target specific bead - library | | wash buffer(s) | | amplification / detection reagents | | non-aqueous solvent | |
|---|---|---|---|---|---|---|---|

| sample | → | Analyte Binding | → | Washing | → | Reagent Loading | → | phase transfer | → | amplification/ detection assignment of analyte specific signal to respective label component |
|---|---|---|---|---|---|---|---|---|---|---|

Figure 3B

Figure 4

Figure 5

Figure 6

Figure 7

Figure 8

Figure 9

A)Example Library for detection of single analyte in several samples

ASR:

Label component:  1  2

C) Example Library for detection of several analytes in single sample

ASR:

Label component:  1  2  3  4

B) Example Library for detection of several analytes in single sample

ASR:

Label component:  1  2

D) Example Library for detection of several analytes in several samples

ASR:

Label component:  1  2  3  4
5  6  7  8

= container or other means for physically separating subset of microparticles

Figure 10A) – D)

Example Library for detection of several analytes in single sample
from figure 10C)
put into use in a method of detecting/quantitating severaly analytes
in a single sample

ASR:

| | | | | |
|---|---|---|---|---|
| Label component: | 1 | 2 | 3 | 4 |

Figure 10 E)

Figure 11A

Figure 11B

Figure 12

Figure 13

Europäisches
Patentamt

European
Patent Office

Office européen
des brevets

# EUROPEAN SEARCH REPORT

Application Number

EP 19 21 6596

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 343 223 A1 (BLINK AG [DE])<br>4 July 2018 (2018-07-04)<br>* paragraph [0001] - paragraph [0003]; claims 1-14; examples 1-3 *<br>* paragraph [0009] - paragraph [0018] *<br>* paragraph [0027] - paragraph [0033] *<br>----- | 1-24 | INV.<br>G01N33/543<br>C12N15/10<br>C12Q1/6806 |
| X | ROH YOON HO ET AL: "Microfluidic Fabrication of Encoded Hydrogel Microparticles for Application in Multiplex Immunoassay",<br>BIOCHIP JOURNAL, KOREAN BIOCHIP SOC, SEOUL, SOUTH KOREA,<br>vol. 13, no. 1, 12 March 2019 (2019-03-12), pages 64-81, XP036740168,<br>ISSN: 1976-0280, DOI:<br>10.1007/S13206-019-3104-Z<br>[retrieved on 2019-03-12]<br>* the whole document *<br>----- | 1-25 | |
| X | NAK WON CHOI ET AL: "Multiplexed Detection of mRNA Using Porosity-Tuned Hydrogel Microparticles",<br>ANALYTICAL CHEMISTRY,<br>vol. 84, no. 21,<br>28 September 2012 (2012-09-28), pages 9370-9378, XP055373601,<br>US<br>ISSN: 0003-2700, DOI: 10.1021/ac302128u<br>* the whole document *<br>-----<br>-/-- | 1-25 | TECHNICAL FIELDS SEARCHED (IPC)<br><br>G01N<br>C40B<br>C12N<br>C12Q |

The present search report has been drawn up for all claims

| Place of search | Date of completion of the search | Examiner |
|---|---|---|
| Munich | 29 May 2020 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 19 21 6596

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAELLE C. LE GOFF ET AL: "Hydrogel microparticles for biosensing", EUROPEAN POLYMER JOURNAL., vol. 72, 1 November 2015 (2015-11-01), pages 386-412, XP055538456, GB ISSN: 0014-3057, DOI: 10.1016/j.eurpolymj.2015.02.022 * the whole document * | 1-25 | |
| A | XIANGLING XIONG ET AL: "Responsive DNA-Based Hydrogels and Their Applications", MACROMOLECULAR RAPID COMMUNICATIONS, vol. 34, no. 16, 16 July 2013 (2013-07-16), pages 1271-1283, XP055303526, DE ISSN: 1022-1336, DOI: 10.1002/marc.201300411 * the whole document * | 1-25 | |
| A | JIE CHOU ET AL: "Porous Bead-Based Diagnostic Platforms: Bridging the Gaps in Healthcare", SENSORS, vol. 12, no. 12, 9 November 2012 (2012-11-09), pages 15467-15499, XP055112077, DOI: 10.3390/s121115467 * the whole document * | 1-25 | **TECHNICAL FIELDS SEARCHED (IPC)** |

The present search report has been drawn up for all claims

| Place of search | Date of completion of the search | Examiner |
|---|---|---|
| Munich | 29 May 2020 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 19 21 6596

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2020

| Patent document cited in search report | | Publication date | Patent family member(s) | | Publication date |
|---|---|---|---|---|---|
| EP 3343223 | A1 | 04-07-2018 | AU | 2017385533 A1 | 11-07-2019 |
| | | | CA | 3048761 A1 | 05-07-2018 |
| | | | EP | 3343223 A1 | 04-07-2018 |
| | | | EP | 3563155 A1 | 06-11-2019 |
| | | | JP | 2020503893 A | 06-02-2020 |
| | | | US | 2019331677 A1 | 31-10-2019 |
| | | | WO | 2018122162 A1 | 05-07-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ADLER ; WACKER et al.** *Immuno-PCR,* 2003
- **SCHMITTGEN et al.** *Nature Protocols,* 2008, vol. 3, 1101-1108
- **ADLER, M. ; R. WACKER ; C. M. NIEMEYER.** A real-time immuno-PCR assay for routine ultrasensitive quantification of proteins. *Biochem Biophys Res Commun,* 2003, vol. 308 (2), 240-250
- **BIRTWELL, S. ; H. MORGAN.** Microparticle encoding technologies for high-throughput multiplexed suspension assays. *Integr Biol (Camb),* 2009, vol. 1 (5-6), 345-362
- **FULTON, R. J. ; R. L. MCDADE ; P. L. SMITH ; L. J. KIENKER ; J. R. KETTMAN, JR.** Advanced multiplexed analysis with the FlowMetrix system. *Clin Chem,* 1997, vol. 43 (9), 1749-1756
- **LENG, X. ; W. ZHANG ; C. WANG ; L. CUI ; C. J. YANG.** Agarose droplet microfluidics for highly parallel and efficient single molecule emulsion PCR. *Lab Chip,* 2010, vol. 10 (21), 2841-2843
- **MANDECKI, W., B. ARDELT, T. CORADETTI, H. DAVIDOWITZ, J. A. FLINT, Z. HUANG, W. M. KOPACKA, X. LIN, Z. WANG AND Z. DARZYNKIEWICZ.** Microtransponders, the miniature RFID electronic chips, as platforms for cell growth in cytotoxicity assays. *Cytometry Part A,* 2006, vol. 69A (11), 1097-1105
- **RISSIN, D. M., C. W. KAN, T. G. CAMPBELL, S. C. HOWES, D. R. FOURNIER, L. SONG, T. PIECH, P. P. PATEL, L. CHANG, A. J. RIVNAK, E. P. FERRELL, J. D. RANDALL, G. K. PROVUNCHER, D. R. WALT AND D. C. DUFFY.** Single-molecule enzyme-linked immunosorbent assay detects serum proteins at subfemtomolar concentrations. *Nature Biotechnology,* 2010, vol. 28, 595-599
- **TEKIN, H. C. ; M. A. GIJS.** Ultrasensitive protein detection: a case for microfluidic magnetic bead-based assays. *Lab Chip,* 2013, vol. 13 (24), 4711-4739
- **TODD, J. ; B. FREESE ; A. LU ; D. HELD ; J. MOREY ; R. LIVINGSTON ; P. GOIX.** *Ultrasensitive Flow-based Immunoassays Using Single-Molecule Counting,* 2007
- **WILSON, R. ; A. R. COSSINS ; D. G. SPILLER.** Encoded microcarriers for high-throughput multiplexed detection. *Angew Chem Int Ed Engl,* 2006, vol. 45 (37), 6104-6117